(19) **Europäisches Patentamt / European Patent Office / Office européen des brevets**

(11) **EP 1 405 122 B1**

(12) **EUROPÄISCHE PATENTSCHRIFT**

(45) Veröffentlichungstag und Bekanntmachung des Hinweises auf die Patenterteilung:
**18.07.2007   Patentblatt 2007/29**

(51) Int Cl.:
***G02B 27/01*** *(2006.01)*    ***G06F 3/00*** *(2006.01)*

(21) Anmeldenummer: **01986772.0**

(22) Anmeldetag: **08.10.2001**

(86) Internationale Anmeldenummer:
**PCT/EP2001/011634**

(87) Internationale Veröffentlichungsnummer:
**WO 2002/031581 (18.04.2002 Gazette 2002/16)**

(54) **VORRICHTUNG  ZUR BESTIMMUNG DER ORIENTIERUNG EINES AUGES**

DEVICE FOR DETERMINING THE ORIENTATION OF AN EYE

SYSTEME  PERMETTANT DE DETERMINER L'ORIENTATION D'UN OEIL

(84) Benannte Vertragsstaaten:
**AT BE CH CY DE DK ES FI FR GB GR IE IT LI LU MC NL PT SE TR**
Benannte Erstreckungsstaaten:
**LT LV**

(30) Priorität: **07.10.2000   WOPCT/EP00/09840**
**07.10.2000   WOPCT/EP00/09843**
**07.10.2000   WOPCT/EP00/09841**
**07.10.2000   WOPCT/EP00/09842**
**22.05.2001   WOPCT/EP01/05886**
**08.06.2001   DE 10127826**

(43) Veröffentlichungstag der Anmeldung:
**07.04.2004   Patentblatt 2004/15**

(73) Patentinhaber: **Dickerson, David**
**85354 Freising (DE)**

(72) Erfinder:
• **EBERL, Roland H. C.**
  **82061 München-Neuried (DE)**
• **EBERL, Heinrich A.**
  **87463 Probstried (DE)**
• **DICKERSON, David**
  **85354 Freising (DE)**

(56) Entgegenhaltungen:
**WO-A-99/42315          DE-A- 19 731 303**
**US-A- 6 120 461**

**Beschreibung**

*Gebiet der Erfindung*

Die vorliegende Erfindung betrifft eine Vorrichtung und ein Verfahren zur Bestimmung der Lage und/oder Orientierung, insbesondere der Blickrichtung, eines Auges durch serielles Auffangen eines von einem Teil des Auges abgestrahlten Lichtstrahls.

*Stand der Technik*

Die Kenntnis der momentanen Orientierung eines Auges ist eine notwendige Voraussetzung für eine Vielzahl unterschiedlichster Anwendungen:

In der Medizin ist es bei der Behandlung von Fehlsichtigkeiten, Netzhautablösungen, Makuladegenerationen etc. notwendig, willkürliche und vor allem unwillkürliche Orientierungsänderungen des Auges zu erfassen, um beispielsweise einen Laserstrahl geeignet so nachführen zu können, daß er jeweils den selben Punkt auf der Netzhaut trifft bzw. auf der Netzhaut eine bestimmte Trajektorie abfährt.

In der Psychologie läßt die Orientierung des Auges diverse Rückschlüsse zu - so zeigt beispielsweise eine plötzliche starke Verdrehung einen beginnenden Epilepsie-Anfall oder einen Black-out an. Wesentlich weitergehende Information läßt sich darüber hinaus aus der Blickrichtung des Auges gewinnen, die beispielsweise durch die Mittelsenkrechte der Pupille bestimmt werden kann. Damit läßt sich beispielsweise das Erkennungsmuster von Versuchspersonen beim Betrachten bestimmter Bilder analysieren.

Die Kenntnis der Blickrichtung bietet darüber hinaus die Möglichkeit, die Information, wohin der Betrachter gerade blickt, zu verwenden, um den fixierten Gegenstand zu identifizieren, sei es ein bestimmter Menüpunkt auf einem (virtuellen) Bildschirm, ein zu aktivierendes Gerät (Lichtschalter etc.), ein ausgewähltes Ziel eines Raketenschützen oder ähnliches.

Desweiteren ermöglicht die Kenntnis der Orientierung und insbesondere der Blickrichtung des Auges es beispielsweise, visuelle Informationen in das Auge hineinzuprojizieren, die mit dem wahrgenommenen Umgebungsbild bzw. der virtuellen Orientierung des Betrachters (etwa bei Verwendung von virtual-reality-Brillen o.ä.) korreliert sind, so daß die hineinprojizierten Bilder beispielsweise scheinbar im Raum oder relativ zu einem Objekt ruhen bzw. sich das eingespielte Bild entsprechend der Blickrichtung des virtual-reality-Brillen-Benutzers ändert.

Daher wurden zur Erfassung der Orientierung des Auges bereits diverse Vorrichtungen und Verfahren entwikkelt:

Die DE 196 31 414 A1 erwähnt Sensoren, die direkt am Augapfel befestigt werden. Diese stellen zum einen natürlich eine beträchtliche Gefährdung und Beeinträchtigung der Versuchsperson dar, können zum anderen auch die Blickrichtung des Auges nicht erfassen (beispielsweise bei schielenden Personen) und sind darüber hinaus relativ zu den oft minimalen unwillkürlichen Augenbewegungen recht ungenau.

Darüber hinaus ist die integrale Aufnahme des Retina-Reflexbildes durch CCD-Kameras erwähnt, was einerseits aufgrund der hohen Belichtung für das lichtschwache Reflexbild geeignet ist, andererseits aber eine sehr hohe Datenmenge und damit geringe Verarbeitungsgeschwindigkeiten bedingt sowie Verzerrungsprobleme beinhaltet.

Die o.g. Druckschrift schlägt daher eine Vorrichtung vor, bei der ein von der Retina reflektiertes Bild der Umgebung mittels Scanvorrichtung seriell abgetastet, elektronisch modifiziert und nachfolgend in das Auge zurückprojiziert wird.

Hierbei wird die Orientierung des Auges, insbesondere die Blickrichtung, jedoch nicht bestimmt, sondern lediglich ein reflektiertes und anschließend bearbeitetes Bild der Umgebung auf dem selben Strahlengang in ein Auge zurückprojiziert, um eine Überdeckung mit dem tatsächlich wahrgenommenen Bild zu gewährleisten.

In der Anmeldung WO 02097511 sind ebenfalls mannigfaltige Verfahren und Vorrichtungen zur Anpassung eines optischen Systems an die Blickrichtung des menschlichen Auges beschrieben.

Näheres zum Stand der Technik enthält WO 99/42315, die ein Verfahren zum Steuern oder Bedienen von Systemen durch Bildinformationen vorschlägt, bei dem ein Objekt oder eine Szene mit dem menschlichen Auge durch eine opto-elektronische Brille erfaßt wird und das Netzhautreflexbild zur Gewinnung von Bildinformationen aufgenommen wird. Die Bildinformationen werden ausgewertet und mit gespeicherten Daten verglichen. Bildinformationen werden in einem Scan-Vorgang vom Reflexionsbild der Netzhaut aufgenommen und einem Bildverarbeitungs- und Speichersystem zugeführt. Vorgeschlagen wird die Auslösung einer gewünschten Funktion durch optische Erfassung der Bedienfunktion eines Geräts mit den Augen an einer geeigneten Stelle und Aktivieren eines einzigen Bedienelements. Dabei wird das von der Netzhaut reflektierte Muster im Bereich der Fovea Centralis beider Augen ausgewertet. Es kann ein Teil des Bildaufnahmezyklus dazu verwendet werden, die Pupillenöffnung zu lokalisieren und zu verfolgen und die Symmetrieachse der Bildabtastung auf sie zu zentrieren. Vorgeschlagen wird auch die Einstellung einer hohen Auflösung im Bereich des schärfsten Sehens in der Fovea Centralis, die aber zu den Rändern des Gesichtsfeldes auf der Netzhaut stark abnimmt.

Aufgabe der vorliegenden Erfindung ist es daher, eine Vorrichtung und ein Verfahren zur Verfügung zu stellen, mit der die Lage und/oder die Orientierung, insbesondere die Blickrichtung, eines Auges schnell und präzise bestimmt werden kann.

### *Zusammenfassung der Erfindung*

Erfindungsgemäß wird diese Aufgabe durch die Vorrichtung zur Bestimmung der Orientierung eines Auges gemäß Anspruch 1 oder Anspruch 8 gelöst. Bevorzugte Ausführungsformen der Erfindung sind in den Unteransprüchen beschrieben.

Die beanspruchte Erfindung läßt sich durch die im Folgenden beschriebenen Ausführungsbeispiele einer Vorrichtung bzw. eines Verfahrens zur Bestimmung der Orientierung eines Auges besser verstehen. Im allgemeinen beschreiben die beschriebenen Ausführungsbeispiele bevorzugte Ausführungsbeispiele der Erfindung. Dem aufmerksamen Leser wird jedoch auffallen, daß einige Aspekte der beschriebenen Ausführungsbeispiele über den Schutzumfang der Ansprüche hinausragen. Sofern die beschriebenen Ausführungsbeispiele tatsächlich über den Schutzumfang der Ansprüche hinausragen, sind die beschriebenen Ausführungsbeispiele als zusätzliches Hintergrundinformation zu betrachten und stellen keine Definition der Erfindung per se dar. Entsprechend ist das Wort "erfindungsgemäß" im Folgenden gegebenenfalls als "vorzugsweise" zu verstehen.

Bei einer erfindungsgemäßen Vorrichtung bzw. einem erfindungsgemäßen Verfahren zur Bestimmung der Lage und/oder der Orientierung, insbesondere der Blickrichtung, eines Auges, beschreibt ein Ausgangs- bzw. Endpunkt eines von einem Teil des Auges abgestrahlten und von einem Detektorsystem erfaßten Lichtstrahls und/oder eines von einem Projektionssystem auf bzw. in das Auge projizierten Lichtstrahls ein Bewegungsmuster einer Scan- und/oder Projektionsbewegung quasi zweidimensionale im Auge, wenn die Richtung des Lichtstrahls gemäß der Scan- bzw. Projektionsbewegung zeitlich verändert wird.

Eine erfindungsgemäßen Vorrichtung bzw. ein erfindungsgemäßes Verfahren zur Bestimmung der Lage und/oder der Orientierung, insbesondere der Blickrichtung, eines Auges nach der vorliegenden Erfindung beruht also darauf, daß ein von einem Teil des Auges abgestrahltes Lichtsignal mit Hilfe eines Detektorsystems erfaßt und ausgewertet wird. Neben dem Detektorsystem kann eine erfindungsgemäße Vorrichtung noch weitere optische Vorrichtungen umfassen, insbesondere ein Projektionssystem.

Der Begriff "Lage" bzw. "Orientierung" kann sich dabei auf die Lage bzw. Orientierung des Auges relativ zur Vorrichtung (Relativposition) oder relativ zur Umgebung (Absolutposition) beziehen. Im folgenden ist Lage und/oder Orientierung der Einfachheit halber manchmal zu Orientierung zusammengefaßt, der Begriff "Orientierung" kann also stets die kinematische Orientierung, die kinematische Lage oder beides bedeuten.

Beide Positionen lassen sich bei Bedarf bei Kenntnis der Lage bzw. Orientierung der Vorrichtung relativ zur Umgebung, die beispielsweise mittels einer geeigneter Orientierungsbestimmungsvorrichtung (Lasertriangulation, Radar, Global Positioning System (GPS) Empfänger o.ä.) festgestellt wird, nach den Regeln der Kinematik eindeutig ineinander umrechnen. Dabei kann die Orientierungsbestimmungsvorrichtung an der erfindungsgemäßen Vorrichtung selbst befestigt sein (beispielsweise ein GPS-Empfänger mit zugehöriger Auswerteeinheit) und/oder die Orientierung der erfindungsgemäßen Vorrichtung von der Umgebung aus bestimmen (beispielsweise mittels Lasertriangulation oder dergleichen). Die Bestimmung der Orientierung eines Auges kann auch die Bestimmung einer Orientierungsänderung des Auges gegenüber einer gewählten Referenzlage umfassen.

Eine erfindungsgemäße Vorrichtung kann in einer bevorzugten Ausführung in Form einer Brille ausgebildet sein. Gleichermaßen kann sie beispielsweise auch an einem am Kopf tragbaren Gestell, einem Helm oder dergleichen angeordnet bzw. integriert sein. Gleichermaßen kann sie beispielsweise auch an einem ortsfesten Gerät angeordnet bzw. integriert sein, das die von der erfindungsgemäßen Vorrichtung gelieferten Informationen bezüglich der Orientierung des Auges verwendet, etwa an einem Gerät zur Laserchirurgie oder dergleichen. Gleichermaßen kann sie an einem relativ zur Umgebung und relativ zum Träger beweglichen Objekt wie beispielsweise einem tragbaren elektronischen Notizbuch, einem Laptop oder dergleichen angeordnet bzw. integriert sein.

Im folgenden werden nun für jeden der Begriffe "Teil des Auges", "Lichtsignal", "optische Vorrichtung", "Detektion und Auswertung" verschiedenen Merkmale vorgeschlagen, die bei einer erfindungsgemäßen Vorrichtung bzw. einem erfindungsgemäßen Verfahren alternativ oder gemeinsam kombiniert sein können.

Es wird expressis verbis darauf hingewiesen, daß die vorliegende Erfindung alle möglichen Kombinationen der beschriebenen Merkmale umfaßt, sofern die Kombination nicht für den Fachmann von vornherein sinnlos ist.

Im folgenden werden, wo es zweckmäßig ist, Merkmale eines erfindungsgemäßen Verfahrens beschrieben. Dabei ist stets auch ausdrücklich eine Vorrichtung offenbart, die geeignet ist, das vorgeschlagenen Verfahren auszuführen, beispielsweise ein geeignet programmierter Computer, Sensoren, die in der Lage sind, die notwendigen Informationssignale zu liefern, Signalverarbeitungsvorrichtungen, die in der Lage sind, diese Signale geeignet aufzubereiten (zu filtern, D/A bzw. A/D zu wandeln, zu speichern o.ä.) etc. Umgekehrt ist mit der Funktion einer erfindungsgemäßen Vorrichtung stets auch ein entsprechendes Verfahren offenbart.

Als Blickrichtung eines Auges wird im folgenden vorzugsweise die Gerade durch den Pupillenmittelpunkt und die Fovea centralis definiert, da ein Mensch im Regelfall sein Auge so auf einen zu betrachtenden Punkt richtet, daß dieser im Bereich des schärfsten Sehens abgebildet wird. In der Literatur werden eine Vielzahl verschiedener Achsen des Auges verwendet, beispielsweise die Fixierungsachse ("fixation axis") durch fixierten, betrachteten Punkt und Fovea centralis, die visuelle Achse ("visual axis") durch Knotenpunkt und Fovea centralis oder die achromatische Achse ("achromatic axis", Achse mit verschwindender transversaler chromatischer Aberation), die jeweils durch geometrische Beziehungen (Geraden durch Fovea centralis, Pupillen-, Linsenmittelpunkt, Brennpunkt etc.) festgelegt sind. Die vorliegende Erfindung ist nicht auf die oben eingeführte Definition der Blickrichtung beschränkt. Alternativ kann jede der dem Fachmann geläufigen Geraden als Blickrichtung verwendet sein.

1. Lichtsignal

1.1 Definition

Kern der vorliegenden Erfindung ist ein von einem Teil des Auges abgestrahlter Lichtstrahl, der zur Bestimmung der Orientierung und insbesondere der Blickrichtung des Auges dient. Als Lichtstrahl wird dabei im Sinne der geometrischen Optik stets ein Strahlenbündel bezeichnet. Dessen Durchmesser kann sehr klein, im Grenzfall unendlich klein (dann geht der Lichtstrahl in den sog. Hauptstrahl über) sein, vorzugsweise kleiner gleich der Eintrittspupille des Detektors. Dabei hat sich überraschenderweise gezeigt, daß entgegen einem im Stand der Technik herrschenden Vorurteil auch mit sehr kleinen Strahldurchmessern gearbeitet werden kann, wenn genügend starke Lichtquellen (beispielsweise Laser oder dergleichen) und/oder genügend empfindliche Detektoren verwendet werden. Weiters wird explizit darauf hingewiesen, daß der Begriff "Lichtstrahl" elektromagentische Strahlung jeglicher Wellenlänge umfassen kann, neben dem sichtbaren also insbesondere auch infrarotes Licht. Der Begriff "abgestrahlter" Lichtstrahl umfaßt sowohl im Auge generierte Lichtstrahlen, beispielsweise die Wärmestrahlung von Blutgefäßen in der Netzhaut, als auch an Teilen des Auges reflektierte oder gestreuten Lichtstrahlen, die von außen auf das Auge fallen.

1.2 Passive Abtastung

Vorzugsweise kann als abgestrahltes Licht Umgebungslicht verwendet sein, das aus der Umgebung in das geöffnete Auge einfällt und dort an wenigstens einem Teil wie beispielsweise der Netzhaut reflektiert wird. Anschließend wird aus diesem reflektierten Licht gezielt durch ein Detektorsystem pixelweise je ein Lichtstrahl ausgewählt und erfaßt bzw. aufgefangen. Dies wird im folgenden als "passive Abtastung" bezeichnet.

Als abgestrahltes Licht kann gleichermaßen Licht verwendet sein, das von einem Teil des Auges emittiert wird, beispielsweise Wärmestrahlung. Anschließend wird aus diesem emittierten Licht gezielt durch geeignete Vorrichtungen pixelweise je ein Lichtstrahl ausgewählt und aufgefangen bzw. erfaßt. Dies wird im folgenden ebenfalls als "passive Abtastung" bezeichnet.

Vorteilhafterweise ist hier kein Projektionssystem nötig. Es wird auch kein störendes zusätzliches Bildsignal in das Auge hineinprojiziert. Aus dem polyspektralen Umgebungslicht können darüber hinaus Lichtstrahlen besonders geeigneter Wellenlängen herausgefiltert werden.

1.3 Aktive Abtastung

Alternativ kann erfindungsgemäß zunächst durch ein Projektionssystem, beispielsweise einen Laser oder einen anderen Strahler, aktiv Licht in das Auge hineinprojiziert und ein am betreffenden Teil des Auges reflektierter Strahl anschließend aufgefangen bzw. erfaßt werden. Dies wird im folgenden als "aktive Abtastung" bezeichnet.

1.3.1 Modulation

Erfindungsgemäß kann das aktiv in das Auge eingestrahlte Licht geeignet moduliert sein, beispielsweise, um es mittels einer entsprechenden Signalverarbeitung einfach vom Umgebungslicht unterscheiden oder den Zeitpunkt seiner Ausstrahlung bestimmen zu können. Dabei werden stets eine oder mehrere charakteristische Größen des aktiv eingestrahlten Lichts wie beispielsweise die Intensität, die Wellenlänge bzw. Frequenz, die Polarisation, die Strahldichte oder dergleichen über der Zeit geändert.

*1.3.1. Amplitudenmodulation*

Als Amplitudenmodulation wird nachfolgend eine Modulation bezeichnet, bei der wenigstens eine der oben angegebenen charakteristischen Größen in verschiedenen Zeitabschnitten bzw. zu verschiedenen Zeitpunkten jeweils bestimmte Werte aufweisen. Ein solcherart moduliertes Licht kann beispielsweise vom Umgebungslicht aufgrund der charakteristischen Größe unterscheidbar und/oder der Zeitpunkt seiner Ausstrahlung kann bestimmbar sein.

*1.3.1.2 Frequenzmodulation*

Alternativ oder zusammen mit der oben beschriebenen Amplitudenmodulation kann das aktiv eingestrahlte Lichtsignal auch frequenzmoduliert sein. Als Frequenzmodulation wird nachfolgend die Änderung einer Periodizität wenigstens einer der oben angegebenen charakteristischen Größen bezeichnet, d.h., eine der Größen ändert sich periodisch und die Periodendauer dieser periodischen Änderung weist in verschiedenen Zeitabschnitten bzw. zu verschiedenen Zeitpunkten bestimmte Werte auf. Anhand der Periodendauer eines reflektierten erfaßten Lichtsignals kann beispielsweise der Zeitpunkt der Aussendung dieses Lichtsignals und damit die Laufzeit des Lichtsignals ermittelt werden.

*1.3.1.3 Andere Modulation*

Es können auch eine oder mehrere charakteristische Größen des aktiv eingestrahlten Lichtstrahls gleichzeitig oder nacheinander amplituden- und/oder frequenzmoduliert sein. Weiters kann in analoger Weise nicht nur der Wert (Amplitudenmodulation) wenigstens einer charakteristischen Größe des Lichtstrahls und/oder die Periodizität (Frequenzmodulation) dieser Größe moduliert sein, also in verschiedenen Zeiträumen jeweils bestimmte Werte aufweisen, sondern beispielsweise auch der Phasenwinkel periodisch sich ändernder Größen (Phasenmodulation).

Das Lichtsignal kann auf andere Weise so moduliert sein, daß es eine Information beinhaltet, beispielsweise, wann es emittiert wurde oder die das Lichtsignal gegenüber Umgebungslicht kennzeichnet.

*1.3.2 Anpassung der aktiven Lichtstärke*

Die aktive Abtastung weist gegenüber der passiven Abtastung den Vorteil auf, daß sichergestellt werden kann, daß stets Licht ausreichender Intensität aus dem Auge abgestrahlt wird, indem aktiv entsprechend viel Licht eingestrahlt und reflektiert wird.

*1.3.3 Strahldurchmesser*

*1.3.3.1 Punktuelle Beleuchtung*

Erfindungsgemäß kann ein Lichtstrahl, der einen sehr kleinen Durchmesser aufweisen kann, von einem Strahler, beispielsweise einem Laser, emittiert und so gelenkt werden, daß nacheinander, i.e. sequentiell, verschiedene Punkte auf dem Teil des Auges beleuchtet werden (punktuelle Beleuchtung).

Die an diese Punkten reflektierten Lichtstrahlen können gezielt von einem Detektorsystem erfaßt werden (punktuelle Abtastung). Beispielsweise wird jeweils ein Lichtstrahl emittiert und der reflektierte Lichtstrahl wird im Detektorsystem erfaßt.

Gleichermaßen kann ein Detektorsystem bei punktueller Beleuchtung auch einen größeren Bereich erfassen, d.h. es kann gleichzeitig Lichtstrahlen, die an verschiedenen Punkten reflektiert werden, erfassen (flächenhafte Abtastung). Da jeweils nur ein Punkt aktiv beleuchtet wird, empfängt das Detektorsystem auch jeweils nur einen aktiv eingestrahlten und reflektierten Lichtstrahl. Dies ist vorteilhaft, da das Detektorsystem nicht nacheinander auf verschiedene Punkte fokussiert bzw. auf Lichtstrahlen von verschiedenen Punkten eingestellt werden muß. Es sei angemerkt, daß eine punktuelle oder eine flächenhafte Abtastung auch bei passiver Abtastung und/oder flächenhafter Beleuchtung möglich sind.

*1.3.3.1.1 Erfassung von Licht aus der ganzen Pupille*

Ein in das Auge eingestrahlter Lichtstrahl, der vorzugsweise einen sehr kleinen Durchmesser aufweisen kann, wird an einem reflektierenden Teil des Auges, beispielsweise der Netzhaut, größtenteils in eine bestimmte Richtung zurückreflektiert bzw. zurückgestreut. Ein Teil dieser in anderer als der bestimmten Richtung reflektierten bzw. gestreuten Lichtstrahlen gelangt durch die Pupille aus dem Auge heraus und verläuft näherungsweise koaxial zum reflektierten Hauptstrahl. Die reflektierten Lichtstrahlen, die gerade noch die Pupille passieren, werden als Randstrahlen bezeichnet. Vorzugsweise ist das Detektorsystem so ausgebildet, daß auch diese Randstrahlen erfaßt werden können, wodurch sich vorteilhaft die erfaßbare Lichtmenge erhöht.

*1.3.3.2 Flächenhafte Beleuchtung*

Statt eines einzelnen Lichtstrahls mit einem möglicherweise sehr kleinen Durchmesser, kann auch ein größerer Bereich des Auges flächenhaft aktiv beleuchtet werden (flächenhafte Beleuchtung). Beispielsweise kann ein Lichtstrahl mit größerem Durchmesser so auf das Auge gerichtet werden, daß jeweils ein ganzer Bereich der Netzhaut oder der Komea beleuchtet wird und Licht reflektiert. Aus diesem reflektierten Licht kann dann ein zur Bestimmung der Orientierung verwendete reflektierte Lichtstrahl analog zur passiven Abtastung vom Detektorsystem ausgewählt werden (punktuelle Abtastung).
Gleichermaßen kann das Detektorsystem wie bei der oben beschriebenen flächenhaften Abtastung auch einen größeren Bereich erfassen.
Das aktiv eingestrahlte Licht kann geeignet so moduliert sein, daß es besonders gut auszuwählen ist, indem es etwa eine bestimmte Wellenlänge, Polarisation etc. aufweist. Der beleuchtete Bereich kann den gesamten Teil des Auges umfassen, an dem Licht reflektiert und zur Bestimmung der Orientierung des Auges erfaßt wird. Alternativ können auch nacheinander Teilbereiche beleuchten werden, so daß jeweils mindestens der Punkt, an dem Licht reflektiert und zur Bestimmung der Orientierung des Auges erfaßt wird, beleuchtet wird.

*1.4 Mischbetrieb*

Aktive Abtastung und passive Abtastung können erfindungsgemäß kombiniert sein. Beispielsweise wird das vom Auge wahrgenommene Bild aus dem reflektierten Umgebungslicht erfaßt und zusätzlich ein Lichtstrahl aktiv eingestrahlt, um anhand dieses reflektierten Strahls bestimmte Strukturen, Kennzeichen bzw. Merkmale des Auges (Fovea centralis, blinder Fleck etc.) zu erfassen. Vorteilhafterweise kann die Kombination variabel sein, so daß beispielsweise in Phasen ausreichender Umgebungslichtintensität keine aktive Abtastung erfolgt und, sobald zu wenig Umgebungslicht reflektiert wird, zusätzlich aktiv Licht eingestrahlt wird.
Vorteilhafterweise können auch verschiedene passive und/oder aktive Abtastarten kombiniert sein. So können die Vorteile beider Verfahren kombiniert sein. Beispielsweise kann aktiv eingestrahltes und an der Netzhaut reflektiertes Licht im Infrarotbereich oder von den Blutgefäßen der Netzhaut ausgestrahltes Infrarotlicht erfaßt werden, d.h. es können viele Lichtstrahlen erfaßt werden, um so beispielsweise einzelne Blutgefäße zur Bestimmung der Orientierung präzise zu erfassen. Gleichzeitig kann ein von der Netzhaut reflektiertes Bild der Umgebung erfaßt werden, um eine Korrelation des wahrgenommenen Bildes zu den Blutgefäßen zu ermitteln. Aus der Korrelation zwischen den bezüglich des Auges festen Blutgefäßen und einem bezüglich der Umgebung festen Bild kann dann die Orientierung des Auges relativ zur Umgebung ermittelt werden.

*1. 5 Wellenlänge*

Bei der passiven Abtastung können aus dem Ungebungslicht, das sowohl den Bereich des sichtbaren Lichts als auch das übrigen Spektrum elektromagnetischer Strahlung, insbesondere Infrarotstrahlung, umfassen kann, wie oben beschrieben bestimmte Wellenlängen herausgefiltert, d.h. absorbiert oder durchgelassen werden, die beispielsweise besonders gut erfaßbar sind. Bei der aktiven Abtastung kann ebenfalls sowohl sichtbares Licht als auch elektromagnetische Strahlung anderer Wellenlängenbereiche, insbesondere Licht aus dem ultravioletten oder infraroten Bereich verwendet werden. Infrarotes Licht ist insbesondere vorteilhaft, da das zusätzliche aktive Signal vom Auge nicht wahrgenommen wird.

*1.6 "Flying spot"*

Sowohl bei der passiven als auch bei der aktiven Abtastung werden abgestrahlte Lichtstrahlen bevorzugt seriell aufgefangen und ausgewertet. D.h. Lichtstrahlen, die von bestimmten Punkten ("Pixeln") entlang einer Kurve auf dem betreffenden Teil des Auges abgestrahlt werden, werden nacheinander von dem Detektorsystem erfaßt. Diese sequentielle Abtastung wird "flying spot"-Verfahren genannt, da hierbei der Blickpunkt ("spot") des Detektorsystems die Kurve quasi abfährt. Allgemein werden Abtast- und Projektionsverfahren, bei dem räumlich begrenzte Gebiete typischerweise entlang einer geraden oder gekrümmten Kurve seriell abgetastet bzw. beleuchtet werden, in der Fachsprache häufig als "Flying-Spot"-Verfahren (zu deutsch: "fliegender Punkt"- Verfahren) bezeichnet.
Als Kurve wird in der vorliegenden Anmeldung im mathematischen Sinne jede beliebige kontinuierliche oder diskrete Folge von zwei- bzw. dreidimensionalen Punkten bezeichnet, wobei jedem Punkt ein oder mehrere Werte zugeordnet sein können. Um Verwechslungen zu vermeiden, wird die Kurve der Punkte des Auges, die beleuchtet bzw. abgetastet werden, im folgenden als Trajektorie oder Bewegungsmuster bezeichnet. D.h., die Ausgangs- bzw. Endpunkte der beleuchteten bzw. erfaßten Lichtstrahlen beschreiben ein zwei- bzw. dreidimensionales Bewegungsmuster im bzw. auf dem Auge. Das Bewegungsmuster, daß sich bei der Abtastung bzw. Erfassung der Lichtstrahlen ergibt, wird als Bewegungsmuster der Scanbewegung bzw. Abtastmuster bezeichnet. Das Bewegungsmuster, daß sich bei der Projektion der Lichtstrahlen ergibt, wird als Bewegungsmuster der Projektionsbewegung bzw. Projektionsmuster bezeichnet.

Bei der flächenhaften Abtastung wird eine dreidimensionale, in der Regel gekrümmte Struktur des Auges auf ein zweidimensionales Bild abgebildet. Dabei ergeben sich unerwünschte Verzerrungen. Darüberhinaus ist die Fokussierung auf eine dreidimensionale Fläche problematisch.

Bei vielen herkömmlichen augenbezogenen Informationssystemen werden optische Signals mittels eines flächigen Detektors flächenartig aus dem Auge erfaßt bzw. mittels eines flächigen Projektors flächenartig in das Auge projiziert. Diese Vorgehensweise birgt den Nachteil, daß eine optisch korrekte Abbildung eines gekrümmten Augenteils auf einen flächigen Detektor bzw. eines flächigen Projektors auf ein gekrümmtes Augenteil nur mit erheblichem Aufwand zu erzielen ist. Dieses Problem tritt beim Flying-Spot-Verfahren nur im erheblich verringertem Maße auf.

Vorteilhafterweise wird bei der sequentiellen bzw. punktuellen Abtastung jeweils ein Punkt einer Struktur des Auges auf einen Bildpunkt abgebildet. Dadurch lassen sich die oben dargestellten Verzerrungs- bzw. Fokussierprobleme reduzieren.

Daher ist auch die Verwendung von Lichtstrahlen mit kleinem Durchmesser vorteilhaft. Vorteilhafterweise weisen daher die verwendeten Lichtstrahlen am Luft-Augapfel-Übergang einen Durchmesser von unter 100 $\mu$m, bevorzugt von unter 50 $\mu$m, besonders bevorzugt von unter 10 $\mu$m bzw. besonders vorteilhaft von unter 5 $\mu$m auf.

Das Flying-Spot-Verfahren wird zudem aufgrund seiner Kompatibilität mit einem holographischen Element vorzugsweise beim erfindungsgemäßen Verfahren angewandt.

1.6.1 Spiral-, Kreis- oder Ellipsenscan

Bei Verwendung von mechanisch gesteuerten Ablenkeinrichtungen wie Spiegel oder dergleichen sind als Kurven beispielsweise Kreise, Ellipsen oder Spiralen vorteilhaft, da hierbei die Ablenkeinrichtungen harmonische Bewegungen (etwa sinusförmige Auf-Ab-Bewegung o.ä., s. den Abschnitt "optische Vorrichtungen") ausführen, was die Anregung unerwünschter Schwingungen dieser Einrichtungen stark vermindert. Dies gewährleistet eine höhere Präzision der Bestimmung der Orientierung des Auges aufgrund der verringerten systematischen Fehler bei der Lichtstrahlablenkung. Gleichermaßen kann beispielsweise auch ein Linien-Zeilen-Raster abgefahren werden.

Auch ohne mechanische Ablenkeinrichtungen sind als Kurven beispielsweise Kreise, Ellipsen oder Spiralen vorteilhaft: Die menschliche monookulare Wahrnehmung ist im wesentlichen rotationssymmetrisch um eine durch die Fovea centralis und dem optischen Mittelpunkt der Linse verlaufende Sehachse. Dementsprechend sind viele Teile des Auges, zum Beispiel die Iris, die Pupille, die Kornea, die Linse und in manchen Hinsichten auch die Retina, bei den meisten Menschen näherungsweise rotationssymmetrisch um die Sehachse ausgebildet.

Das Auge wird deshalb erfindungsgemäß vorzugsweise gemäß dem Flying-Spot-Verfahren mit einem spiral- oder kreisförmigen Abtast- bzw. Projektionsmuster, vorzugsweise um die Sehachse, überstreichen, wobei unter "kreisförmig" eine Vielzahl von konzentrischen Kreisen verstanden werden kann. Stehen die Projektions- bzw. Abtaststrahlen entsprechend schräg zur Sehachse, kann die Verwendung eines ellipsenförmigen Abtast- bzw. Projektionsmusters vorteilhaft sein, wie in der DE 197 28 890 A1 beschrieben ist.

1.6.2 Strahlen senkrecht zum Auge

Fallen Lichtstrahlen senkrecht auf den Luft-Augapfel-Übergang, so wird ein gewisser Anteil des Lichtes in entgegengesetzte Richtung zum einfallenden Lichtstrahl zurückreflektiert, während der restliche Anteil quasi ungehindert durchstrahlt, wonach es von "tieferliegenden" Teilen des Auges absorbiert bzw. gestreut wird. Ersteres gilt analog für über den Kornea-Luft-Übergang aus dem Auge austretende Lichtstrahlen.

Bei einem erfindungsgemäßen Verfahren wird vorzugsweise gemäß dem Flying-Spot-Verfahren projiziert bzw. abgetastet. Dabei wird vorzugsweise ein "schmaler" Lichtstrahl verwendet, der am Luft-Augapfel-Übergang einen im Vergleich zur Augapfelkrümmung, insbesondere zur Korneakrümmung, unwesentlichen Durchmesser aufweist. Der Lichtstrahl wird vorzugsweise derart projiziert bzw. abgetastet, daß alle seiner Einzelstrahlen den Luft-Augapfel-Übergang möglichst senkrecht begegnen.

Die Kornea, das heißt der Luft-Kornea-Übergang, verursacht ungefähr 80% der vom Auge auf einen darauffallenden Lichtstrahl ausgeübten Brechung. Somit hat die oben beschriebene Vorgehensweise nicht nur den Vorteil, daß wenig Licht am Luft-Komea-Übergang in eine unnützliche Richtung gebrochen wird, sondern auch den Vorteil, daß die Strahlen eine geringe Brechung durch das optische System des Auges erfahren. Dies wirkt sich nicht nur auf die räumliche Projektions- bzw. Abtastgenauigkeit positiv aus, sondern ist auch bei Anwendungen vorteilhaft, bei denen die Geometrie der Lichtstrahlen eine wesentliche Rolle spielt.

Die Tatsache, daß senkrecht auf das Auge fallende Strahlen partiell in entgegengesetzte Richtung zurückreflektiert werden, kann dazu verwendet werden, Informationen über die Topologie des Auges zu gewinnen. Dies kann zum Beispiel über eine ein Projektionssystem und ein Detektorsystem umfassende Projektor-Detektor-Anordnung erfolgen, die Licht ungefähr senkrecht auf das Auge projiziert und anschließend die Koaxialität des erfaßten zurückreflektierten Lichtstrahls und des projizierten Lichtstrahls ermittelt. Sind diese Lichtstrahlen nicht im wesentlichen (insbesondere die Korneaoberfläche weist viele mikro- und makroskopische Unregelmäßigkeiten aus und darf deshalb nicht als glatt reflektierende Oberfläche betrachtet werden) koaxial, so kann daraus geschlossen werden, daß der projizierte Lichtstrahl nicht senkrecht auf das Auge traf. Solche Informationen über die Topologie des Auges können unter anderem zur Ermittlung der Lage und/oder Orientierung des Auges verwendet werden.

Für eine derartige Projektor-Detektor-Anordnung bietet sich eine konfokale Anordnung des Projektionssystems und des Detektorsystems, beispielsweise über einen Teilerspiegel an.

Die Verwendung eines holographischen Elements ist insbesondere bei der Projektion bzw. der Abtastung eines senkrecht zum Auge verlaufenden Lichtstrahls vorteilhaft, da eine einfache derartige virtuelle Ausgestaltung einer Lichtleitvorrichtung ermöglicht, daß Lichtstrahlen aus einem einzigen Projektionssystem senkrecht auf verschiedene Gebiete des Auges und/oder von verschiedenen Gebiete des Auges senkrecht austretende bzw. zurückreflektierte Lichtstrahlen in ein einziges Detektorsystem gelenkt werden können.

Vorstehend wurden die verschiedenen Lichtsignale näher beschrieben, die bei einer erfindungsgemäßen Vorrichtung bzw. einem erfindungsgemäßen Verfahren verwendet sein können. Als nächstes wird auf das Teil des Auges eingegangen, von dem diese Lichtstrahlen abgestrahlt sein können.

2. Abstrahlender Teil des Auges

Zur Bestimmung der Orientierung und insbesondere der Blickrichtung des Auges wird ein an wenigstens einem Teil des Auges abgestrahlter Lichtstrahl erfaßt. Dabei bieten sich aufgrund ihres Reflektions- bzw. Emissionsverhaltens und insbesondere aufgrund bestimmter auf ihnen vorhandener charakteristischer Merkmale verschiedene Bereiche des Auges an, auf die im folgenden näher eingegangen wird. Gleichermaßen kann jedoch auch Licht zur Bestimmung der Orientierung des Auges verwendet werden, das von anderen, hier nicht explizit genannten Teilen des Auges abgestrahlt wird.

*2.1 Kornea*

Erfindungsgemäß können Lichtstrahlen zur Bestimmung der Orientierung des Auges verwendet sein, die von der Kornea abgestrahlt werden. Vorzugsweise werden dabei diejenigen Lichtstrahlen verwendet, die an der äußeren, in Blickrichtung vorderen Oberfläche reflektiert werden.

Die Kornea weist auf ihrer äußeren Oberfläche stets eine Vielzahl mikroskopischer (beispielsweise kleine, durch Fremdpartikeln wie Staub etc. verursachte Narben) und/oder makroskopischer (beispielsweise aufgrund eines chirurgischen Eingriffs an der Kornea) Unregelmäßigkeiten auf, die zwar teilweise nach wenigen Tagen aufgrund von Heilungsvorgängen verschwunden sind, jedoch für eine ausreichende Zeitdauer je ein bezüglich der Orientierung des Auges körperfestes optisch erfaßbares Kennzeichen darstellen. Gleichermaßen kann die Kornea auch andere signifikante Merkmale wie beispielsweise Trübungen oder Verfärbungen aufweisen, die ebenfalls zur Bestimmung der Orientierung benutzt werden können.

Beispielsweise können zur Bestimmung der Orientierung des Auges eine oder mehrere dieser Unregelmäßigkeiten erfaßt und diese Unregelmäßigkeit bzw. Unregelmäßigkeiten über der Zeit "verfolgt", d.h. beispielsweise ihre jeweilige Lage in einer zweidimensionalen Abbildung erfaßt werden. Aus der Änderung der Lage der Unregelmäßigkeit bzw. Unregelmäßigkeiten kann dann eine Orientierungsänderung des Auges bestimmt werden.

Gleichermaßen kann beispielsweise zur Bestimmung der Orientierung des Auges eine Referenzkarte der Kornea erstellt werden, auf der die Lage verschiedener signifikanter Merkmale bezüglich eines Referenzkoordinatensystems gespeichert ist. Wird das Auge nun erneut in gleicher Weise abgetastet, kann eine Bild- bzw. Mustererkennung die signifikanten Merkmale in der neu erstellten Karte erkennen und durch Vergleich der Lage und Orientierung der Merkmale auf der Referenz- bzw. der neuen Karte eine Orientierungsänderung des Auges ermitteln (s. auch den Abschnitt "Auswertung").

Alternativ oder zusätzlich kann auch ein von bzw. an der in Blickrichtung hinteren Oberfläche der Komea abgestrahlter oder reflektierter Lichtstrahl erfaßt werden. Anhand dieses Lichtstrahls kann beispielsweise das charakteristisch dunkleReflexbild der Makulagrube auf der hinteren Oberfläche erfaßt werden, dessen Position auf der Kornea direkt auf die Orientierung des Auges hindeutet. Ebenfalls kann die Phasenverschiebung zwischen an der vorderen und an der hinteren Oberfläche reflektierten Lichtstrahlen ermittelt werden (beispielsweise nach Purkinje).

*2.2 Lederhaut, Iris, Pupille*

Erfindungsgemäß können Lichtstrahlen verwendet sein, die von der Iris bzw. der angrenzenden Lederhaut abgestrahlt werden. Infolge der stark unterschiedlichen Reflektionsgrade von weißer Lederhaut, farbiger Iris und quasi lichtschluckender Pupille lassen sich aus den abgestrahlten Strahlen die Übergänge Lederhaut-Iris und/oder Iris-Pupille im Regelfall sehr einfach und genau bestimmten. Damit lassen sich beispielsweise die Lage der Pupille und/oder der Iris bestimmen, indem anhand einer stark wechselnden Helligkeit des reflektierten Lichts auf einen Übergang Iris-Pupille geschlossen und so Punkte auf dem Umfang der Pupille gefunden werden (s. den Abschnitt "Auswertung").

Zusätzlich oder alternativ kann auch die Struktur der Iris bestimmt werden. Diese weist charakteristische Merkmale (Muster, Farbunterschiede, Verletzungen oder dergleichen) auf, deren Lage beispielsweise in einer Referenzkarte gespeichert und mit der aktuell identifizierten Lage der selben Merkmale verglichen werden kann, um so eine Orientierung des Auges zu bestimmen. Wie bei der Kornea kann also anhand der Erfassung bestimmter charakteristischer Merkmale eine Orientierung des Auges ermittelt werden.

*2.3 Netzhaut*

Vorzugsweise können erfindungsgemäß Lichtstrahlen verwendet sein, die von der Netzhaut abgestrahlt werden. Daraus lassen sich charakteristische Merkmale der Netzhaut, etwa einzelne größere Blutgefäße oder die Makula mit der Fovea centralis ermitteln, die nicht nur die Bestimmung der Orientierung sondern beispielsweise zusammen mit der Pupillenmitte insbesondere auch der Blickrichtung des Auges gestatten, die als Gerade durch Pupillenmittelpunkt und Fovea centralis definiert sein kann (s. auch den Abschnitt "Auswertung").

Gleichermaßen ist es möglich, aus dem von der Netzhaut reflektierten Umgebungslicht ein Umgebungsreflexbild zu erfassen, das Rückschlüsse auf die wahrgenommenen Umgebung ermöglicht. Beispielsweise kann dann aus einer zeitlichen Verschiebung bzw. Verzerrung des Umgebungsreflexbildes die Orientierungsänderung des Auges relativ zur Umgebung bestimmen werden.

Zur Unterscheidung wird im folgenden das Bild der Umgebung, das durch von der Umgebung auf die Netzhaut einfallendes Licht erzeugt und von der Netzhaut zumindest teilweise wieder reflektiert wird, als Umgebungsreflexbild (der Netzhaut) bezeichnet (passive Abtastung), während das Bild, das sich aus aktiv auf die Netzhaut eingestrahltem und von dieser reflektiertem Licht ergibt, als Netzhautreflexbild bezeichnet wird (aktive Abtastung). Der Begriff Netzhautreflexbild umfaßt außerdem im Zuge einer kompakteren Darstellung der verschiedenen Varianten auch ein von einem Teil des Auges selbst emittiertes Bild, beispielsweise das Bild der Wärmestrahlung, die die Netzhaut aussendet, obwohl es sich hierbei nicht eigentlich um reflektiertes Licht handelt.

Nach der Erläuterung der verschiedenen Lichtsignale und der verschiedenen Teile des Auges, von denen diese abgestrahlt und zur Bestimmung der Orientierung des Auges verwendet sein können, wird im folgenden auf die optischen Vorrichtungen eingegangen, mit der die Lichtstrahlen aufgefangen und gegebenenfalls vorher ausgestrahlt werden können.

3. Optische Vorrichtungen

Die vorliegende Erfindung umfaßt ein Detektorsystem. Diese umfaßt einen Detektor zum Erfassen von Lichtstrahlen, die wie oben beschrieben von einem bestimmten Teil des Auges abgestrahlt werden. Zusätzlich kann das Detektorsystem eine erste Lichtleitanordnung (Detektor-Lichtleitvorrichtung) beispielsweise zur Ablenkung und/oder Fokussierung von Lichtstrahlen umfassen, mit der beispielsweise nacheinander verschiedene von einem Teil des Auges abgestrahlte Lichtstrahlen in den Detektor geleitet und/oder auf dessen Empfängerbereich fokussiert werden können. Vorzugsweise ist diese Leitvorrichtung so gestaltet, daß Lichtstrahlen, die von verschiedenen Punkten des Auges abgestrahlt wurden, nacheinander, i.e. sequentiell, in den Detektor geleitet werden, so daß dieser eine entsprechende Folge von Bildpunkten erfaßt.

Zum Zwecke einer aktiven Abtastung kann eine erfindungsgemäße Vorrichtung neben dem Detektorsystem wenigstens eine weitere optische Vorrichtung umfassen, nämlich ein Projektionssystem zur Projektion von Licht. Das Projektionssystem umfaßt einen Strahler zur Emission von Licht. Weiters kann das Projektionssystem eine Lichtmodulationsvorrichtung umfassen, um beispielsweise das vom Strahler emittierte Licht geeignet zu modulieren. Weiters kann das Projektionssystem eine Projektions-Lichtleitanordnung beispielsweise zur Ablenkung und/oder Fokussierung der emittierten Lichtstrahlen umfassen, mit der beispielsweise nacheinander Lichtstrahlen, die vom Strahler emittiert werden, auf bestimmte Punkte des Auges gelenkt und/oder fokussiert werden. Vorzugsweise wird die Detektor-Lichtleitvorrichtung zumindest teilweise vom Projektionssystem verwendet. Z.B. kann Licht zunächst aktiv auf das Auge eingestrahlt und das reflektierte Licht auf dem selben Strahlengang in den Detektor geleiten werden. Hierdurch wird für Projektion und Erfassung zumindest teilweise der selbe Strahlengang verwendet. Dies ist vorteilhaft, da systematische Fehler der Lichtleitvorrichtung kompensiert werden können das Projektionssystem, auf eine eigene Lichtleitanordnung zumindest teilweise verzichten kann. Darüberhinaus ist es vorteilhaft, daß der Detektor quasi automatisch das vom Strahler emittierte Lichtsignal erfaßt und nicht eigens auf den reflektierenden Punkt des Auges eingestellt werden muß.

Im Folgenden werden diese einzelnen Elemente erfindungsgemäßer optischer Vorrichtungen im einzelnen näher erläutert.

*3.1 Detektor*

Ein erfindungsgemäßes Detektorsystem umfaßt einen Detektor zur Erfassung von Lichtstrahlen. Der Detektor weist einen Empfängerbereich auf und erfaßt wenigstens eine charakteristische Größe von Lichtstrahlen, die auf diesen Empfängerbereich auftreffen, beispielsweise die Strahlungsenergie bzw. die Strahlungsleistung, die Bestrahlungsstärke, die Wellenlänge bzw. Frequenz, die Polarisation oder dergleichen. Der Detektor gibt ein Signal entsprechend der erfaßten Größe bzw. Größen aus. Durch eine geeignete Vorrichtung, beispielsweise einen Analog/Digitalwandler und/oder einen Filter, kann dieses Signal gegebenenfalls vor der Ausgabe geeignet bearbeitet werden. Beispielsweise kann ein opto-elektronischer Detektor ein optisches Signal erfassen und ein entsprechendes elektrisches Signal ausgeben, ein opto-optischer Detektor kann ein optisches Signal erfassen und ein entsprechendes optisches Signal ausgeben.

Beispielsweise kann der Detektor eine Photodiode umfassen, in der während der Bestrahlung ein Strom fließt, aus dessen Stärke die Beleuchtungsstärke ermittelt werden kann. Der Detektor kann auch einen Photowiderstand aufweisen, dessen ohmscher Widerstand sich in Abhängigkeit von dem absorbierten Licht ändert. Aus der Änderung des Widerstandes kann wiederum die Bestrahlung ermittelt werden. Gleichermaßen kann der Detektor eine Photozelle, einen Phototransistor, einen Photomultiplier, eine Bildverstärkerröhre oder prinzipiell jedes andere Bauelement umfassen, das die Erfassung einer physikalischen Größe eines auf das Bauelement treffenden Lichtstrahls ermöglicht.

*3.2 Detektor-Lichtleitanordnung*

Vorzugsweise erfaßt der oben beschriebene Detektor nacheinander Lichtstrahlen, die von verschiedenen Punkten des Auges abgestrahlt werden. Hierzu kann der Detektor selbst entsprechend so ausgebildet bzw. gesteuert sein, daß er nacheinander gezielt Lichtstrahlen erfassen kann, die aus verschiedenen Richtungen bzw. auf verschiedenen Punkten seines Empfängerbereichs auftreffen. Alternativ oder zusätzlich kann eine Detektor-Lichtleitanordnung vorhanden sein, die jeweils einen Lichtstrahl auf den Empfängerbereich des Detektors leitet und/oder den Lichtstrahl auf den Empfängerbereich fokussiert. Die Detektor-Lichtleitanordnung kann so gesteuert werden, daß nacheinander Lichtstrahlen auf den Detektor gelenkt werden, die aus verschiedenen Richtungen auf die Detektor-Lichtleitanordnung treffen. Durch entsprechende zeitliche Steuerung der Detektor-Lichtleitanordnung können so gezielt nacheinander die Lichtstrahlen auf den Detektor gelenkt werden, die von bestimmten Punkten des Auges abgestrahlt wurden. Der Blickpunkt ("spot") des Detektors beschreibt damit ein Bewegungsmuster auf einem Teil des Auges, scannt diesen Bereich also mit einem bestimmten Bewegungsmuster ab.

Als Detekor-Lichtleitanordnung ist jede Vorrichtung möglich, die diese Funktion erfüllt, also so gesteuert werden kann, daß nacheinander Lichtstrahlen aus verschiedenen, vorgegebenen oder bestimmbaren Richtungen jeweils auf den Empfängerbereich des Detektors gelenkt und/oder fokussiert werden. Nachfolgend werden einige mögliche Merkmale solcher Vorrichtungen näher erläutert.

*3.2.1 Spiegel*

Eine erfindungsgemäße Detektor-Lichtleitanordnung kann einen oder mehrere Spiegel umfassen. Deren Orientierung zueinander, zum Auge, dessen Orientierung bestimmt werden soll, und zum Detektor kann so einstellbar sein, daß Lichtstrahlen, die von bestimmten Punkten des Auges abgestrahlt wurden, auf den Detektor gelenkt werden. Dabei können einige dieser Spiegel bezüglich des Detektors fest angeordnet sein ("feste Spiegel"), während andere Spiegel in ihrer Orientierung verstellbar sein können ("bewegliche Spiegel").

Die Orientierung der beweglichen Spiegel kann so steuerbar sein, daß ein gewünschtes Bewegungsmuster abgetastet werden kann, d.h., daß nacheinander Lichtstrahlen, die von bestimmten Punkten des Auges abgestrahlt wurden, auf den Detektor gelenkt werden. Aufgrund der Umkehrbarkeit des Strahlenganges läßt sich die Detektor-Lichtleitvorrichtung auch so auffassen, als ob ein gedachter Lichtstrahl vom Detektor emittiert und durch die Detektor-Lichtleitanordnung auf den Punkt des Auges gelenkt und/oder fokussiert würde, der gerade vom Detektor abgetastet werden soll.

*3.2.2 Holographie*

Anstelle der oben beschriebenen Spiegel können auch holographische Elemente, verwendet sein, die die gewünschte Ablenkung der Lichtstrahlen bewirken. Solche holographischen Elemente weisen gegenüber herkömmlichen Spiegeln mehrere Vorteile auf. Zum einen weisen sie ein geringeres Gewicht als ein Spiegel auf. Zum anderen können sie so ausgestaltet sein, daß sie vom Auge selbst nicht wahrgenommen werden. Vor allem ist es möglich, holographische Elemente mit nahezu beliebigem Reflektionsverhalten herzustellen. Beispielsweise kann ein holographisches Element nur Licht in einem bestimmten Wellenlängenbereich reflektieren. Liegt dieser Wellenlängebereich beispielsweise im Infrarotbereich, ist das holographische Elemente vom Auge nicht wahrnehmbar.

Ein holographisches Element kann beispielsweise eine holographische Beschichtung auf einem Träger sein.

Wird ein holographisches Element anstelle eines beweglichen Spiegels zur veränderbaren Ablenkung von Lichtstrahlen eingesetzt, kann die hierbei erforderliche Änderung der Orientierung der Reflektion analog zum beweglichen Spiegel durch eine Orientierungsänderung des holographischen Elements erfolgen. Gleichermaßen kann aber auch das Reflektionsverhalten des holographischen Elements beispielsweise elektronisch verändert werden, was eine erheblich raschere und präzisere Abtastung ermöglicht. Hierzu muß das holographische Element elektro-holographisch sein.

### 3.2.3 Andere Elemente

Die Detektor-Lichtleitvorrichtung kann andere optische Elemente umfassen, insbesondere beispielsweise Blenden, die den Durchmesser des erfaßten Lichtstrahls einschränken und damit den abgetasteten Bereich des abgetasteten Teils des Auges räumlich beschränken, oder optische oder elektro-optische Linsen, die den Lichtstrahl geeignet aufweiten bzw. fokussieren, beispielsweise auf den Empfängerbereich des Detektors.

### 3.3 Projektionssystem

Zum Zwecke einer aktiven Abtastung kann neben dem Detektor und eventuell der Detektor-Lichtleitanordnung zusätzlich ein Projektionssystem mit einem Strahler vorhanden sein, der Lichtstrahlen emittiert. Weiters kann das Projektionssystem eine Lichtmodulationsvorrichtung umfassen, um das vom Strahler emittierte Licht geeignet zu modulieren. Weiters kann der Strahler eine Projektions-Lichtleitanordnung beispielsweise zur Ablenkung und/oder Fokussierung der emittierten Lichtstrahlen umfassen, mit der beispielsweise nacheinander Lichtstrahlen, die vom Strahler emittiert werden, auf bestimmte Punkte des Auges gelenkt und/oder fokussiert werden. Diese Elemente werden nachfolgend erläutert.

### 3.3.1 Strahler

Ein Strahler emittiert Lichtstrahlen, die vorzugsweise auf einen Teil des Auges treffen und von diesem reflektiert werden. Als Strahler ist daher prinzipiell jede Vorrichtung denkbar, die Lichtstrahlen emittieren kann, beispielsweise Glühbirnen, Laser, LEDs oder dergleichen.

Vorteilhafterweise ist wenigstens eine der physikalischen Größen des vom Strahler emittierten Lichts einstellbar, so daß das emittierte Licht vom Umgebungslicht unterscheidbar ist. Beispielsweise kann ein Laser nur Licht in einem bestimmten, eng begrenzten Wellenlängenbereich aussenden, oder der Strahler sendet Lichtstrahlen mit einem bestimmten zeitlichen Muster bezüglich Intensität, Wellenlänge oder dergleichen aus.

Der Begriff "Strahler" kann bei der vorliegenden Erfindung auch mehrere einzelne Lichtquellen umfassen, beispielsweise verschiedenfarbige LEDs oder Laser, die Licht mit unterschiedlichen Wellenlängen aussenden können.

### 3.3.2 Lichtmodulationsvorrichtung

Das vom Strahler emittierte Licht kann in einer Lichtmodulationsvorrichtung geeignet moduliert werden, bevor es auf das Auge trifft. Beispielsweise kann eine Lichtmodulationsvorrichtung einen Farbfilter umfassen, der nur Licht einer bestimmten Wellenlänge passieren läßt. Gleichermaßen kann eine Lichtmodulationsvorrichtung einen Polarisationsfilter umfassen, der nur Licht einer bestimmten Polarisation passieren läßt. Solche und andere Filter können so steuerbar sein, daß das Licht über der Zeit moduliert werden kann.

### 3.3.3 Projektions-Lichtleitanordnung

Um vom Strahler emittiertes Licht dahin zu lenken, von wo es reflektiert werden soll, um anschließend der erfindungsgemäßen Vorrichtung erfaßt zu werden, kann eine Projektions-Lichtleitanordnung vorhanden sein, in die vom Strahler emittiertes Licht eintritt und die dieses Licht auf den gewünschten Bereich lenkt.

Prinzipiell kann dazu aufgrund der Umkehrbarkeit des Strahlenganges eine zu irgendeiner der beschriebenen Detektor-Lichtleitanordnungen analoge Anordnung dienen.

Insbesondere kann die Detektor-Lichtleitvorrichtung selbst einen Teil der Projektions-Lichtleitanordnung bilden, indem das vom Strahler emittierte Licht gegensinnig parallel in den Strahlengang des vom Detektorsystem erfaßten Lichts vor, in bzw. nach der Detektor-Lichtleitvorrichtung eintritt. Eine solche Anordnung weist den Vorteil auf, daß möglicherweise vorhandenen systematische Fehler bei Projektion und Abtastung identisch sind und sich gegenseitig kompensieren. Ein weiterer Vorteil besteht darin, daß das Detektorsystem quasi automatisch den Lichtstrahl erfaßt, den der Strahler emittiert hat.

Hierzu kann beispielsweise ein Teilerspiegel zwischen Detektor-Lichtleitvorrichtung und Detektor vorhanden sein, der Licht, das von einem Strahler kommt, in die Detektor-Lichtleitvorrichtung teilweise passieren läßt und Licht, das aus der Detektor-Lichtleitvorrichtung kommt, teilweise zum Detektor reflektiert, wobei Strahlen in Richtung Detektor vozugsweise Vorrang gegeben wird. Beispielsweise kann das Verhältnis der zum Detektor reflektierten Strahlen zu den auf den Teilerspiegel fallenden Strahlen 95%, 90%, 85% oder 80% betragen. Das Verhältnis der den Teilerspiegel passierenden Strahlen zu den auf den Teilerspiegel fallenden Strahlen kann beispielsweise 5%, 10%, 15% oder 20% betragen. D.h., ein auf den Teilerspiegel fallender Lichtstrahl wird beispielsweise zu 95% reflektiert und kann zu 5% den Spiegel passieren.

Daß ein solcher Teilerspiegel den Projektionsstrahl hindert, ist unkritisch, da dies durch eine Erhöhung der Strahlerleistung ausgeglichen werden kann.

*3.4 Außenaufnahme*

Zusätzlich kann eine erfindungsgemäße Vorrichtung eine Vorrichtung zur Aufnahme eines Bildes der Umgebung wie beispielsweise eine Kamera umfassen. Dieses Bild kann verarbeitet werden, um beispielsweise signifikante Muster der Umgebung zu identifizieren.

Ein durch eine solche Vorrichtung aufgenommenes Bild der Umgebung kann beispielsweise geeignet mit einem an der Netzhaut oder der Kornea des Auges reflektierten Bild der Umgebung ("Umgebungsreflexbild") verglichen werden. Beispielsweise kann in beiden Bildern das selbe Objekt identifiziert und aus der räumlichen Zuordnung der beiden Bilder des Objekts die Orientierung der Vorrichtung bzw. des Auges relativ zur Umgebung ermittelt werden.

Bevorzugt kann eine solche Vorrichtung zur Aufnahme eines Bildes der Umgebung näherungsweise konfokal zum Auge angeordnet sein.

*3.5 Lage der Vorrichtung*

Zusätzlich kann eine erfindungsgemäße Vorrichtung eine Orientierungsbestimmungsvorrichtung zur Bestimmung der Orientierung der Vorrichtung umfassen, um beispielsweise anhand einer Orientierung des Auges relativ zur Vorrichtung eine Orientierung des Auges relativ zur Umgebung zu bestimmen.

Eine solche Orientierungsbestimmungsvorrichtung kann fest mit der erfindungsgemäßen Vorrichtung verbunden sein und die Orientierung und/oder Lage der Vorrichtung relativ zur Umgebung bestimmen. Beispielsweise kann eine Orientierungsbestimmungsvorrichtung einen GPS-Empfänger und die zugehörige Auswerteeinrichtung umfassen, die aus den empfangenen GPS-Signalen die Lage der Vorrichtung ermittelt.

Gleichermaßen kann eine Orientierungsbestimmungsvorrichtung auch fest mit der Umgebung verbunden sein und die Orientierung der Vorrichtung beispielsweise mittels Triangulation oder dergleichen ermitteln.

*3.6 Markierungen*

Eine erfindungsgemäße Vorrichtung kann eine oder mehrere Markierungen enthalten. Eine Markierung kann beispielsweise vor dem Auge angeordnet sein, entweder innerhalb oder außerhalb des Blickfeldes. Beispielsweise kann eine solche Markierung auf einem Brillenglas vor dem Auge angeordnet sein.

Eine Markierung kann beispielsweise dazu dienen, optische Referenzwerte zu bestimmen. Beispielsweise kann ein an einer solchen Markierung reflektierter Lichtstrahl vom Detektor erfaßt werden. Eine charakteristische Größe dieses Lichtstrahls kann dann als Referenzwert, beispielsweise für eine 100% Reflektion, bestimmt werden.

Eine solche Markierung kann auch als Fixpunkt bei der Bestimmung der Orientierung des Auges bezüglich der Vorrichtung verwendet werden. Beispielsweise kann eine Markierung so vorhanden sein, daß der Detektor außer Lichtstrahlen, die von einem signifikanten Bereich des Auges abgestrahlt werden (beispielsweise der Fovea centralis oder der Iris), auch Lichtstrahlen erfassen kann, die von der Markierung abgestrahlt, beispielsweise reflektiert, werden. Anhand der Richtungen, aus denen die jeweiligen Lichtstrahlen erfaßt werden, kann eine Position des signifikanten Bereichs des Auges relativ zur Markierung und damit eine Orientierung des Auges relativ zur Vorrichtung bestimmt werden.

Eine solche Markierung kann auch dazu verwendet werden, die optische Vorrichtung zu kalibrieren bzw. nachzukalibrieren. Aufgrund äußerer Einflüsse (beispielsweise eines Stoßes) oder innerer Einflüsse (beispielsweise Temperaturdehnung) kann sich im Verlauf des Betriebs die Lage und Orientierung einzelner Elemente der optischen Vorrichtung verändern. Beispielsweise können die Spiegel aufgrund einer Verformung der Vorrichtung, auf der sie angeordnet sind, ihre Position zueinander verändern. Um eine solche Veränderung zu bestimmen, kann beispielsweise zunächst eine Referenzlage der Markierung bestimmt werden, indem bestimmt wird, wie die Detektor-Lichtleitanordnung gesteuert sein muß, damit der Detektor Licht erfaßt, daß von der Markierung reflektiert wird. Wird dann später analog die Lage der Markierung bestimmt, so kann aus einer Veränderung gegenüber der Referenzlage die Änderung der optischen Vorrichtung bezüglich der Markierung bestimmt werden. Ist dabei die Markierung bezüglich des Detektors fest, so beruht die (scheinbare) Lageänderung auf einer Veränderung der Detektor- Lichtleitanordnung, die somit bestimmt werden kann.

Vorteilhafterweise kann bei der aktiven Abtastung die Markierung gezielt mit einem Lichtstrahl angestrahlt und der reflektierte Lichtstrahl vom Detektor erfaßt werden, beispielsweise um eine der vorstehenden Bestimmungen (Referenzwert, Fixpunkt, Kalibrierung) durchzuführen.

Vorteilhafterweise kann die Markierung für das Auge nicht wahrnehmbar ausgestaltet sein. Beispielsweise kann die Markierung nur im Infrarotbereich sichtbar sein, so daß sie von einem Infrarot-Detektor erfaßbar ist, von einem menschlichen Auge hingegen nicht als störend wahrgenommen wird. Dies kann beispielsweise mittels eines Hologramms bzw. einer holographischen verwirklicht sein.

Eine Markierung kann alternativ oder zusätzlich auch selbst aktiv Licht emittieren.

Weitere erfindungsgemäße Markierungen bzw. deren Verwendung sind im Abschnitt 4.3 offenbart.

*3.7 Fokussierung*

Die oben beschriebenen optischen Vorrichtungen, also insbesondere das Detektorsystem und/oder das Projektionssystem können wenigstens eine geeignete Fokussiervorrichtung umfassen, mit der beispielsweise der Abstand des abgetasteten Bereichs von Detektor und/oder Strahler einstellbar ist und damit beispielsweise bestimmbar ist, ob die Netzhaut oder die Komea abgetastet bzw. beleuchtet wird. Bei Lichtstrahlen mit kleinen Durchmessern kann der Nutzen einer solche Fokussiervorrichtung eingeschränkt sein.

Nachdem nun die Merkmale der optischen Vorrichtung aufgeführt und näher erläutert wurden, die einen von einem Teil eines Auges abgestrahlten Lichtstrahl erfassen bzw. Licht auf das Auge einstrahlen, wird im folgenden auf die Erfassung der Lichtstrahlen und die Auswertung der so gewonnenen Signale eingegangen.

4. Detektion und Auswertung

Ziel der vorliegenden Erfindung ist es, die Lage und/oder Orientierung, insbesondere die Blickrichtung, eines Auges zu bestimmen. Im folgenden wird beschrieben, wie Merkmale des Auges aus von einem Teil des Auges abgestrahlten und anschließend vom Detektorsystem erfaßten Lichtstrahlen bestimmt und wie diese Merkmale zur Bestimmung der Orientierung des Auges verwendet werden können.

*4.1 Erfaßtes Bild*

Bevorzugt werden bei der Bestimmung erfindungsgemäß Punkte längs einer Trajektorie sequentiell, i.e. nacheinander abgetastet. Das heißt, Lichtstrahlen, die von diesen Punkten abgestrahlt werden, werden nacheinander im Detektorsystem erfaßt und wenigstens eine physikalische Größe dieser Lichtstrahlen, beispielsweise deren Intensität bzw. Helligkeit, Strahlleistung, Wellenlänge, Grauwerte oder dergleichen, wird bestimmt. Als Ergebnis erhält man eine Folge von Bildpunkten (d.h. eine Bildpunkt-Kurve), denen sich beispielsweise Werte für die physikalische Größe bzw. Größen und, beispielsweise anhand der Stellung der Detektor-Lichtleitanordnung, bestimmte Koordinaten (beispielsweise Abszisse x und Ordinate y; Radius R und Polarwinkel $\phi$) zuordnen lassen. Die Werte der physikalischen Größe bzw. Größen werden auch als ein Informationsgehalt des Licht(signals) bezeichnet.

Bei feinerer Abtastung, d.h. beispielsweise bei kleineren Schritten zwischen den einzelnen Stellungen (x,y) der Detektor-Lichtleitanordnung kann aus der Bildpunkt-Kurve ein immer schärferes zweidimensionales "Bild" des Teils des Auges ermittelt werden, von dem die Lichtstrahlen abgestrahlt wurden. Durch erfindungsgemäße Abtastung kann also ein zweidimensionales Bild wenigstens eines dreidimensionalen Bereichs des Auges, beispielsweise der Kornea, der Netzhaut oder dergleichen, erstellt werden.

Ist einer dieser Bereiche gekrümmt, so erscheint er im zweidimensionalen Bild verzerrt. Aus solchen Verzerrungen kann die Orientierung des Auges bestimmt werden. Beispielsweise kann eine kreisförmige Pupille je nach Orientierung des Auges zum Detektor als Ellipse erscheinen, aus deren Hauptachsen sich die Orinetierung des Auges ermitteln läßt.

In Blickrichtung des Detektors bzw. der Detektor-Lichtleitanordnung, d.h. entgegen der Richtung der Lichtstrahlen, die vom Detektor erfaßt werden, hintereinander liegende Elemente überdecken sich im zweidimensionalen Bild bzw. weisen einen bestimmten Abstand auf. Aufgrund solcher Überdeckungen bzw. Abstände kann wiederum die Orientierung des Auges ermittelt werden. Ist beispielsweise die dreidimensionale Lage des Pupillenmittelpunktes zur Fovea centralis bekannt, beispielsweise empirisch aus statistischen Untersuchungen oder vorhergehenden erfindungsgemäßen Bestimmungen, so kann aus dem Abstand der Bilder des Pupillenmittelpunktes und der Fovea centralis im zweidimensionalen Bild ihre räumliche Lage und damit die Orientierung und/oder Lage des Auges ermittelt werden.

Gleichermaßen kann die dreidimensionale Lage eines erfaßten Punktes beispielsweise aus der zweidimensionalen Lage seines Bildpunktes und der Laufzeit des an ihm reflektierten Signals bestimmt werden, wobei die Laufzeit beispielsweise bei einem frequenzmodulierten Lichtsignal anhand der Frequenz des erfaßten Lichtsignals bestimmt werden kann. Aus der Laufzeit kann beispielsweise ein Abstand des erfaßten Punktes zum Detektor und damit die Lage dieses Punktes relativ zur Vorrichtung bestimmt werden. Gleichermaßen kann statt der Laufzeit die Fokussierung, d.h. beispielsweise die Einstellung einer Fokussiervorrichtung, verwendet werden, die ebenfalls eine Information über den Abstand des erfaßten Punktes zum Detektor liefert.

Wird, beispielsweise anhand der Laufzeit, der Abstand des reflektierenden Punktes zum Detektor bestimmt, kann jedem Bildpunkt zusätzlich ein z-Wert zugeordnet werden, so daß die dreidimensionale Lage der abgetasteten Punkte relativ zur erfindungsgemäßen Vorrichtung bestimmbar ist. In diesem Fall kann quasi ein dreidimensionales Bild des Auges abgetastet werden. Im folgenden wird der Einfachheit halber meist auf zweidimensionale Abbildungen Bezug genommen, anhand der oben gezeigten dreidimensionalen Bildpunkte kann jedoch stets auch ein analoges dreidimensionales Verfahren verwendet sein. Wird beispielsweise von der Lage des Bildpunktes im zweidimensionalen Bild gesprochen, kann durch zusätzliche Messung des Abstandes des Punktes, der Licht abstrahlt, zum Detektor auch die dreidimensionale Lage des Punktes relativ zur Vorrichtung bzw. relativ zu anderen Punkten ermittelt werden.

Die zweidimensionalen Bilder derselben dreidimensionalen Struktur des Auges, das bei verschiedenen Orientierungen abgetastet wird, sind nicht nur gegeneinander verschoben bzw. verdreht (woraus rückwärts die Orientierungsänderung des Auges bestimmt werden kann), sondern auch unterschiedlich verzerrt. Bei einer bevorzugten hohen Abtastfrequenz im Vergleich zu Geschwindigkeit der Augenbewegung unterscheiden sich die Verzerrungen bei zeitlich rasch hintereinander abgetasteten Bildern jedoch nur geringfügig, so daß sie näherungsweise vernachlässigt und direkt die zweidimensionalen Bilder verwendet werden können, ohne die unterschiedlichen Verzerrungen zu berücksichtigen. Ändert sich die Orientierung des Auges merklich, so können sich auch die Verzerrungen in den verschiedenen zweidimensionalen Bildern deutlich voneinander unterscheiden, so daß solche Bilder beispielsweise nicht mehr geeignet miteinander verglichen werden können. Daher kann es vorteilhaft sein, in bestimmten Zeitabständen oder, falls eine deutliche Orientierungsänderung festgestellt wirdbestimmte Bilder erneut abzutasten.

*4.2. Strategie*

Anhand der erfaßten Bildpunkte kann die Lage bestimmter charakteristischer Merkmale bzw. Strukturen des Teils des Auges, der das erfaßte Licht abstrahlt, innerhalb des Bildes ermittelt werden.

Hierzu kann das Auge längs einer geeigneten Trajektorie abgetastet werden. Diese Trajektorie kann beispielsweise so gewählt sein, daß besonders signifikante Merkmale, beispielsweise Wechsel der Reflektivität oder dergleichen, mit einer hohen Wahrscheinlichkeit erfaßt werden.

Beispielsweise kann die Lage des Pupillenmittelpunktes und/oder des Makulamittelpunktes ermittelt werden. Dies kann für eine Bestimmung der Blickrichtung relevant sein.

Mehrere der beschriebenen Verfahren können auch nacheinander durchgeführt werden, wobei die in einem vorhergehenden Verfahren gewonnen Informationen bei nachfolgenden Verfahren verwendet werden können. Exemplarisch kann beispielsweise erst der Pupillenmittelpunkt grob bestimmt werden. Der so gefundene näherungsweise Mittelpunkt wird gespeichert und dient als Start- bzw. Referenzwert für die Feinbestimmung des tatsächlichen Pupillenmittelpunkts. Der näherungsweise oder der tatsächliche Pupillenmittelpunkt kann wiederum als Startwert bzw. vorläufiger Makulamittelpunkt bei der Bestimmung des Makulamittelpunkts dienen, da beispielsweise der Makulamittelpunkt mit einer gewissen Wahrscheinlichkeit in einer bestimmten Lage zum Pupillenmittelpunkt liegen kann. Diese Lage kann beispielsweise empirisch aus statistischen Untersuchungen und/oder vorhergehenden erfindungsgemäßen Bestimmungen bekannt bzw. geschätzt sein. Beispielsweise kann in Blickrichtung des Auges der Makulamittelpunkt mit hoher Wahrscheinlichkeit direkt hinter dem Pupillenmittelpunkt liegen.

Insbesondere können auch alle oben oder nachstehend beschriebenen Verfahren anhand bereits identifizierter Merkmale "nachjustiert" werden, d.h. bereits identifizierte Merkmale könne als Referenz- bzw. Startwerte für nachfolgende Verfahren dienen. Hierzu kann beispielsweise ein Bezugspunkt bestimmt und dann eine oder mehrere optische Vorrichtungen, also beispielsweise Detektor-Lichtleitanordnung und/oder Projektions-Lichtleitanordnung, bezüglich dieses Bezugspunktes ausgerichtet werden.

Vorteilhafterweise kann die Abtastung mit einer hohehn Abtastfrequenz durchgeführt werden. Dadurch ändert sich vorteilhafterweise die Orientierung des Auges zwischen zwei bzw. mehreren hintereinander abgetasteten Bildern nur wenig. Damit ändert sich auch die Lage signifikanter Merkmale innerhalb der abgetasteten Bilder nur wenig, was die Suche nach diesen Merkmalen vorteilhaft erleichtern kann.

*4.3 Auswertung*

Ist nach wenigstens einem der obigen Verfahren die zwei- bzw. dreidimensionale Lage eines bzw. mehrerer signifikanter Merkmale des Auges bestimmt, so lassen sich daraus auf verschiedene Weise Orientierungen des Auges relativ zur Vorrichtung und/oder zur Umgebung bestimmen. Dabei sei darauf hingewiesen, daß Orientierung auch die Bestimmung einer Änderung der Orientierung bezüglich einer als Referenz gewählten Orientierung umfassen kann.

Zur Verdeutlichung kann beispielsweise in einem einfachen Fall die Orientierung eines Auges zu einem bestimmten Zeitpunkt als Referenz gewählt und die Lage des Pupillenmittelpunkts in einem Bild des von der Lederhaut bzw. der Iris reflektierten Lichts bestimmt werden. Wird zu einem späteren Zeitpunkt erneut die Lage des Pupillenmittelpunkts in einem Bild des von der Lederhaut bzw. der Iris reflektierten Lichts bestimmt, so kann aus der neuen Lage des Pupillenmittelpunktes relativ zur alten Lage eine entsprechende Verdrehung des Auges bestimmt werden. Wird zusätzlich oder alternativ zur Lage des Bildpunktes beispielsweise der Abstand zum Detektor ermittelt, kann daraus auch die Lage des Punktes und damit des Auges realtiv zur erfindungsgemäßen Vorrichtung ermittelt werden.

Exemplarisch werden nachfolgend einige Möglichkeiten zur Bestimmung der Orientierung, insbesondere der Blickrichtung näher erläutert.

*4.3.1 Bestimmung der Sehachse*

Als Blickrichtung bzw. Sehachse kann beispielsweise eine Gerade durch den Drehpunkt des Auges und den Mittelpunkt der Pupille angenommen werden.

Bestimmt man die Lage des Pupillenmittelpunktes für verschiedene Stellungen des Auges, so liegen die Bilder des Pupillenmittelpunktes in einem zweidimensionalen abgetasteten Bild innerhalb eines Kreises (bzw. aufgrund der Verzerrung durch die zweidimensionale Abbildung des dreidimensionalen Auges innerhalb einer Ellipse) um das gedachte Bild des Drehpunktes, der dadurch bestimmt werden kann (beispielsweise als statistischer Mittelpunkt der Bilder des Pupillenmittelpunkts). Die Gerade durch Drehpunkt und Pupillenmittelpunkt stellt dann vereinfacht die Sehachse des Auges gemäß Annahme dar. Zur Verdeutlichung sei beispielsweise eine reine Drehung des Auges um eine vertikale Achse durch den Drehpunkt betrachtet. Das zweidimensionale Bild des Pupillenmittelpunkts bewegt sich dabei in einer zur Betrachtungsrichtung senkrechten und zur vertikalen Drehachse parallelen Bildebene auf einer horizontalen Geraden. Bei sehr vielen aufgenommenen Bildpunkten entspricht der Schwerpunkt dieser Geraden näherungsweise dem Bild des Drehpunktes.

Gleichermaßen kann beispielsweise aus der Verzerrung der Pupille, die bei einer nicht frontalen Draufsicht als Ellipse erscheint, durch eine Hauptachsentransformation die Ebene der Pupille ermittelt werden, deren Mittelsenkrechte als Sehachse angenommen werden kann..

Gleichermaßen kann die Sehachse als die Verbindungsgerade von Fovea centralis und Pupillenmittelpunkt bestimmt werden. Die dreidimensionale Position des Pupillenmittelpunktes und der Fovea centralis kann beispielsweise anhand empirischer Daten aus der Position des Pupillenmittelpunktes und der Lage der Fovea centralis relativ dazu geschätzt werden. Hierzu können beispielsweise gespeicherte Werte für diese Lage verwendet werden, die aus früheren Untersuchungen bekannt sind (beispielsweise die sogenannten Gullstrand'schen Werte) oder Werte, die mittels einer erfindungsgemäßen Vorrichtung ermittelt wurden. Gleichermaßen kann, wie oben beschrieben, beispielsweise der Abstand des Pupillenmittelpunktes und/oder der Fovea centralis vom Detektor anhand der Laufzeit des reflektierten Lichtstrahls zwischen Emittierung und Erfassung und/oder anhand der Fokussierung bestimmt werden und daraus zusammen mit der zweidimensionalen Lage der Bildpunkte die dreidimensionale Lage des Pupillenmittelpunktes und/oder der Fovea centralis bestimmt werden.

*4.3.2 Bestimmung der Orientierung mittels Karte*

Zur Bestimmung der Orientierung des Auges kann erfindungsgemäß ein Referenzbild von einem Teil des Auges erstellt werden, in dem signifikante Strukturen, beispielsweise die Makula, Blutzgefäße der Netzhaut, Verletzungen der Kornea oder dergleichen, erkennbar sind. Diese Merkmale können vorteilhafterweise mittels Bild- bzw. Mustererkennung auf einer entsprechenden Referenzkarte eingetragen werden.

Ein Referenzbild und/oder eine Referenzkarte kann beispielsweise mittels eines Speichers realisiert sein, wobei in jedem der Speicherplätze der Wert der charakteristischen Größe eines beispielsweise lagemäßig zugeordneten erfaßten Lichtstrahls gespeichert ist. Vorteilhafterweise können die charakteristischen Werte zuvor so gefiltert sein, daß nur Speicherplätze mit einem bestimmten charakteristischen Wert belegt werden, beispielsweise einem Wert, der höher ist als ein bestimmter Schwellenwert. In einer Referenzkarte können signifikanten Strukturen des abgetasteten Referenzbild bereits mit einem geringen Speicherbedarf abgebildet und gespeichert sein.

Wird nun zu einem späteren Zeitpunkt erfindungsgemäß ein Teil des Auges abgetastet, auf dem die wie oben erfaßten Strukturen zumindest teilweise vorhanden sind, vorteilhafterweise derselbe Bereich wie bei der Erstellung des Referenzbildes, so können Punkte eines solcherart erfaßten aktuellen Bildes mit dem Referenzbild bzw. der Referenzkarte mittels einer entsprechenden Routine zur Deckung gebracht werden. Aus den dabei auftretenden Verdrehungen und Verschiebungen des aktuellen Bildes kann die Orientierungsänderung des Auges gegenüber dem Zeitpunkt der Aufnahme des Referenzbildes ermittelt werden. Dabei kann das aktuelle Bild mittels einer anderen Trajektorie aufgenommen werden wie das Referenzbild. Insbesondere kann es deutlich weniger Bildpunkte aufweisen. Dabei ist besonders vorteilhaft, daß bereits die Zur-Deckung-Bringung weniger aktueller Bildpunkte mit wenigen Refernzpunkten eine rasche und einfache Bestimmung der Orientierung erlauben kann. Gleichermaßen kann auch anhand des aktuellen Bildes eine aktuelle Karte erstellt und mit der Referenzkarte zur Deckung gebracht werden, wobei wiederum aus den auftretenden Verdrehungen und Verschiebungen die Orientierungsänderung des Auges bestimmt werden kann.

Vorteilhafterweise können Bilder mit einer hohen Abtastfrequenz abgetastet werden, so daß sich die Orientierung des Auges zwischen zwei oder mehreren aufeinanderfolgenden Abtastungen wenig ändert. Dies kann vorteilhaft den Vergleich eines aktuellen Bildes mit einem Referenzbild bzw. einer Referenzkarte vereinfachen, da sich die Lage der Bildpunkte desselben Punktes im bzw. auf dem Auge nur wenig geändert hat und die Korrelation zwischen den Bildpunkten verschiedener Abtastzeitpunkte einfach herzustellen sein kann.

Wie oben beschrieben kann es vorteilhaft sein, ein Referenzbild bzw. eine Referenzkarte erneut zu erfassen, wenn festgestellt wird, daß sich die Orientierung des Auges so stark verändert hat, daß aufgrund der Verzerrung aufgrund der zweidimensionalen Abbildung einer dreidimensionalen Struktur ein Zur-Deckung-Bringung schwierig bzw. unmöglich wird. Eine entsprechend starke Orientierungsänderung des Auges kann beispielsweise dadurch festgestellt werden, daß es nicht mehr gelingt, ein aktuelles Bild und das Referenzbild bzw. die Referenzkarte zur Deckung zu bringen.

*4.3.3 Orientierung bezüglich der Umgebung und/oder der Vorrichtung*

Allgemein kann erfindungsgemäß die absolute und/oder relative Lage und/oder Orientierung, insbesondere die Blickrichtung, eines Auges relativ zur Umgebung und/oder zur erfindungsgemäßen Vorrichtung bestimmt werden.

Dabei ergeben sich verschiedene Möglichkeiten der Orientierungsbestimmung, von denen nachfolgend einige näher erläutert werden.

*4.3.3.1 Relativkinematik*

Vorstehend wurde unter anderem exemplarisch eine Reihe von Möglichkeiten erläutert, anhand eines oder mehrerer Bilder, die durch Erfassen von von einem Teil des Auges abgestrahlten Lichtstrahlen erstellt werden können, die Orientierung des Auges relativ zur Vorrichtung und insbesondere die Blickrichtung des Auges zu bestimmen.

Wird, wie oben angegeben, zusätzlich die Position der Vorrichtung relativ zur Umgebung bestimmt, beispielsweise mittels magnetischer, IR- oder RF-Peilung der Vorrichtung von einer inertial festen Vorrichtung aus oder mittels eines GPS-Empfängers auf der erfindungsgemäßen Vorrichtung, so läßt sich aus der relativen Orientierung des Auges zur Vorrichtung und der relativen Lage der Vorrichtung bezüglich der Umgebung kinematisch die Orientierung des Auges gegenüber der Umgebung errechnen.

Umgekehrt ergibt sich bei bekannter Orientierung des Auges relativ zur Umgebung und bei bekannter Orientierung der Vorrichtung relativ zur Umgebung analog die Orientierung des Auges gegenüber der Vorrichtung. Beschreibt man beispielsweise die Orientierung des Auges gegenüber der Umgebung mittels der Winkel $(\phi_{AU}, \eta_{AU}, \varphi_{AU})$, die Orientierung der Vorrichtung gegenüber der Umgebung mittels der Winkel $(\phi_{VU}, \eta_{VU}, \varphi_{VU})$ und die Orientierung des Auges gegenüber der Vorrichtung mittels der Winkel $(\phi_{AV}, \eta_{AV}, \varphi_{AV})$, so lassen sich die durch die jeweilige Abbildung $A_{xy}$ von x nach y ausgedrückten Orientierungen ineinander überführen :

$$A_{AU} = A_{VU}(\phi_{VU}, \eta_{VU}, \varphi_{VU}) \; * \; A_{AV}(\phi_{AV}, \eta_{AV}, \varphi_{AV})$$

$$A_{AV} = A_{VU}(\phi_{VU}, \eta_{VU}, \varphi_{VU}) \; * \; A_{AU}(\phi_{AU}, \eta_{AU}, \varphi_{AU})$$

Die zweite Gleichung besagt also beispielsweise, daß sich die Abbildung eines augenfesten Koordinatensystems auf ein vorrichtungsfestes Koordinatensystem und damit die Orientierung des Auges relativ zur Vorrichtung bestimmen läßt aus einer geeigneten Verknüpfung der Abbildung vom augen- auf ein umgebungsfestes Koordinatensystem und einer Abbildung von diesem umgebungs- auf das vorrichtungsfeste Koordinatensystem. Diese beiden Abbildungen sind durch die Orientierung des Auges relativ zur Umgebung und der Vorrichtung relativ zur Umgebung bestimmbar.

Im folgenden werden Bilder miteinander verglichen, die bezüglich der Umgebung (Umgebungsbild), bezüglich einer erfindungsgemäßen Vorrichtung (Vorrichtungsbild) oder bezüglich des Auges (Augenbild) fest sind. Diese Bilder werden vorzugsweise in einem gemeinsamem Koordinatensystem, beispielsweise einem bezüglich der Vorrichtung festen Koordinatensystem, miteinander verglichen, d.h., beispielsweise je wenigstens zwei Bilder werden in diesem Koordinatensystem dargestellt und signifikante Strukturen in den Bildern werden einander zugeordnet. D.h., beispielsweise wird ihre relative Lage und/oder Orientierung zueinander bestimmt. "Lage" von Merkmalen, Strukturen etc. umfaßt analog zu "Orientierung" eines Auges die Lage im engeren Sinne, gekennzeichnet beispielsweise durch Abstände zu Koordinatenachsen, und/oder ihre Orientierung im engeren Sinne als in der übrigen Anmeldung, gekennzeichnet beispielsweise durch Winkel gegenüber Koordiantenachsen.

Anhand der Änderung dieser relativen Lagen kann jeweils die Änderung der zugehörigen Bilder zueinander bestimmt werden. Aus dieser Änderung kann jeweils die entsprechende Orientierungsänderung der zugehörigen Systeme (Auge, Vorrichtung, Umgebung) zueinander bestimmt werden. Beispielsweise kann aus einer Verschiebung eines Augen- gegenüber einem Vorrichtungsbild eine entsprechende Verdrehung des Auges gegenüber der Vorrichtung ermittelt werden.

Alternativ oder zusätzlich kann aus den relativen Lagen auch eine absolute Zuordnung der zugehörigen Systeme ermittelt werden. Beispielsweise kann aus der relativen Lage einer signifikanten Struktur in einem Augenbild (beispielsweise der Makula) bezüglich einer signifikanten Struktur in einem Umgebungsbild (beispielsweise eine Kante, ein Baum oder dergleichen) die Lage des Auges zur Umgebung bestimmt werden, also beispielsweise festgestellt werden, wohin das Auge relativ zur signifikanten Struktur in der Umgebung blickt (beispielsweise direkt auf die Kante, auf den Baum oder dergleichen).

Im folgenden werden einige dieser möglichen Bestimmungen näher erläutert.

*4.3.3.2 Auge - Umgebung*

Vergleicht man ein relativ zur Umgebung festes Bild (Umgebungsbild), beispielsweise ein Bild der Umgebung, das von einer fest mit der Vorrichtung verbundenen Kamera von der Umgebung wird, mit einem relativ zum Auge festen Bild (Augenbild), beispielsweise einem Bild der Netzhaut oder der Kornea, das sich beispielsweise aus einer aktiven Abtastung ergibt, so kann daraus eine Orientierung des Auges relativ zur Umgebung bestimmt werden. Vergleicht man Bilder, die zu verschiedenen Zeitpunkten aufgenommen wurden, so kann daraus eine Orientierungsänderung des Auges relativ zur Umgebung bestimmt werden.

*4.3.3.2.1 Relative Orientierung Auge - Umgebung*

Zur Bestimmung der Orientierung des Auges relativ zur Umgebung werden ein Umgebungs- und ein Augenbild in einem gemeinsamen Koordinatensystem betrachtet, beispielsweise in einem bezüglich der Vorrichtung festen Koordinatensystem (vorrichtungsfestes Koordinatenssystem).

Dabei können die Bilder geeignet in dieses Koordinatensystem abgebildet sein. Beispielsweise kann aus der bekannten Geometrie der Anordnung einer Kamera, die das Umgebungsbild liefert, zur Vorrichtung, die das Augenbild liefert, bestimmt werden, wie das Umgebungsbild in ein vorrichtungsfestes Koordinatensystem des Augenbildes zu transformieren ist. Gleichermaßen kann beispielsweise eine signifikante Form der Vorrichtung, beispielsweise die geometrische Form eines Brillenglases, im Umgebungsbild erkennbar sein. Anhand der signifikante Form der Vorrichtung im Umgebungsbild kann dann bestimmt werden, wie das Umgebungsbild in das vorrichtungsfeste Koordinatensystem des Augenbildes zu transformieren ist

Vorteilhafterweise kann als Umgebungsbild ein von einem Teil des Auges reflektiertes Bild der Umgebung verwendet sein, beispielsweise das Umgebungsreflexbild der Netzhaut oder der Kornea, da dann Umgebungs- und Augenbild bereits im selben vorrichtungsfesten Koordiantensystem vorliegen.

Diese Bilder werden miteinander verglichen, d.h. bestimmte signifikante Strukturen innerhalb des Umgebungsbildes (Kanten, Gegenstände, Buchstaben etc.), die beispielsweise mittels Mustererkennung identifiziert sein können, werden bestimmten signifikanten Strukturen des Augenbildes (beispielsweise der Makula, dem Pupillenmittelpunkt etc.) zugeordnet und ihre Lage relativ zueinander bestimmt.

Anhand dieser relativen Lage läßt sich die Orientierung, insbesondere die Blickrichtung, des Auges relativ zur Umgebung bestimmen.

Beispielsweise kann im Augenbild bestimmt werden, an welcher Stelle die Makulamitte liegt. Diese Stelle kann im Umgebungsbild festgestellt werden. Dann kann bestimmt werden, daß der Betrachter mit dem Auge gerade diesen Punkt in der Umgebung anblickt. Damit kann die Blickrichtung des Auges relativ zur Umgebung bestimmt werden. Vorteilhafterweise kann eine Mustererkennung im Umgebungsbild identifizieren, was der Betrachter gerade anblickt, beispielsweise welchen Schalter, welches Ziel oder welchen Gegenstand.

Vorteilhafterweise kann ein Umgebungsbild, das mit einer Kamera aufgenommen wird (Kamerabild), mit einem Umgebungsbild, das vom Auge reflektiert und abgetastet wird (Umgebungsreflexbild), korreliert werden. Die beiden Bilder können einander so zugeordnet sein, daß ein in einem Bild festgelegter Punkt der Umgebung im anderen Bild bestimmbar ist. Dann kann beispielsweise in einem relativ unscharfen und/oder lichtschwachen Umgebungsreflexbildes mittels eines erfindungsgemäßen Verfahrens der Punkt der Umgebung festgestellt werden, auf den das Auge blickt, und dieser Punkt bzw. der zugehörige Gegenstand, Schalter etc. kann in einem schärferen und/oder lichtstärkeren Kamerabild mittels Mustererkennung besser identifiziert werden. Dies ist beispielsweise vorteilhaft bei diffusem Licht, bei dem eine Infrarotkamera ein genaueres Bild der Umgebung abtasten kann.

*4.3.3.2.2 Relative Orientierungsänderung Auge - Umgebung*

Gleichermaßen kann auch eine Änderung der Orientierung des Auges relativ zur Umgebung bestimmt werden, indem analog zu einem erfindungsgemäßen Verfahren zu verschiedenen Zeitpunkten jeweils eine Orientierung des Auges gegenüber der Umgebung bestimmt und die Änderung zwischen den Orientierungen ermittelt wird, die zu verschiedenen Zeitpunkten bestimmt worden sind.
Beispielsweise können signifikante Merkmale im Umgebungsbild (Kanten etc.) und im Augenbild (Makula etc.) einander zugeordnet werden. Beispielsweise können die beiden Bilder jeweils so zu einem Gesamtbild überlagert werden, daß ihre Bildbegrenzungen in einem festen Verhältnis zueinander angeordnet sind (bei gleich großen Umgebungs- und Netzhautreflexbildern beispielsweise deckungsgleich). D.h., beide Bilder können in einem gemeinsamen Koordinatensystem dargestellt werden, vorzugsweise in einem bezüglich der erfindungsgemäßen Vorrichtung festen Koordinatensystem. In dem Gesamtbild können Abständen bzw. Relativlagen zwischen signifikanten Merkmalen des Umgebungs- und des Augenbildes bestimmt werden. Aus der Änderung der relativen Lage der signifikanten Merkmale zueinander im Gesamtbild kann die Änderung der Orientierung des Auges bezüglich der Umgebung bestimmt werden.
Gleichermaßen können auch die beide Bilder (beispielsweise durch ein Korrelationsverfahren oder durch Drehung und Verschiebung) zur Deckung gebracht werden. Aus den hierbei auftretenden Werten (beispielsweise Korrelationsfaktoren oder Drehwinkel und Verschiebung) läßt sich wiederum die Orientierungsänderung des Auges gegenüber der Umgebung bestimmen.

*4.3.3.3 Orientierung Vorrichtung - Umgebung*

Aus der Änderung eines relativ zur Umgebung festen Bildes (Umgebungsbild), beispielsweise eines Bildes, das von einer fest mit einer Vorrichtung verbundenen Kamera aufgenommen oder das von der Netzhaut oder der Kornea reflektiert und von einer erfindungsgemäßen Vorrichtung erfaßt wird, läßt sich eine Orientierungsänderung der Vorrichtung gegenüber der Umgebung bestimmten.
Hierzu werden beispielsweise zwei Umgebungsbilder im selben, vorzugsweise bezüglich der Vorrichtung festen, Koordinatensystem miteinander verglichen, die in einem zeitlichen Abstand voneinander aufgenommen wurden. Hat sich die Orientierung der Vorrichtung gegenüber der Umgebung in der Zwischenzeit verändert, so weisen die selben signifikante Muster innerhalb des Umgebungsbilds zu verschiedenen Zeitpunkten verschiedene Positionen innerhalb des Koordinatensystems auf. Daraus ergibt sich die Orientierungsänderung. Alternativ können die beiden Bilder (beispielsweise durch ein Korrelationsverfahren oder durch Drehung und Verschiebung) zur Deckung gebracht werden. Aus den hierbei auftretenden Werten (beispielsweise Korrelationsfaktoren oder Drehwinkel und Verschiebung) läßt sich wiederum die Orientierungsänderung der Vorrichtung gegenüber der Umgebung bestimmen.
Gleichermaßen kann die Orientierung der Vorrichtung zur Umgebung auch durch einen Vergleich eines Vorrichtungs- mit einem Umgebungsbild bestimmt werden, indem beispielsweise beide Bilder in einem gemeinsamen, vorzugsweise einem vorrichtungsfesten, Koordinatensystem betrachtet werden und signifikante Strukturen im Vorrichtungsbild und im Umgebungsbild einander zugeordnet werden. Aus der relative Lage dieser Strukturen zueinander, beispielsweise einer Markierung an der Vorrichtung oder der geometrischen Form eines Brillenglases, zu einer signifikanten Kante, einem Muster etc. im Umgebungsbild, kann dann eine Orientierung der Vorrichtung relativ zur Umgebung bestimmt werden.
Durch mehrmaliges, zeitlich versetztes Feststellen einer solchen Orientierung kann ebenfalls eine Orientierungsänderung der Vorrichtung gegenüber der Umgebung bestimmt werden.
Ist zusätzlich die Orientierung des Auges gegenüber der Vorrichtung bestimmt, kann daraus relativkinematisch eine Orientierungsänderung des Auges gegenüber der Umgebung bestimmt werden.
Gleichermaßen kann bei bekannter Orientierung der Vorrichtung gegenüber der Umgebung, die beispielsweise mittels einer Orientierungsbestimmungsvorrichtung bestimmt sein kann, und bekannter Orientierung des Auges gegenüber der Vorrichtung relativkinematisch eine Orientierungs des Auges gegenüber der Umgebung bestimmt werden.

*4.3.3.4 Orientierung Auge - Vorrichtung*

Mittels einem bezüglich des Auges festen Bild (Augenbild), beispielsweise einem Bild der Netzhaut oder der Kornea, in dem Strukuren des Auges wie beispielsweise Narben, Blutgefäße etc. erkennbar sind, kann die Orientierung des Auges gegenüber der Vorrichtung bestimmt werden.

Ist zusätzlich die Orientierung bzw. Orientierungsänderung der Vorrichtung gegenüber der Umgebung bekannt, können daraus zusammen mit der Orientierung bzw. Orientierungsänderung des Auges gegenüber der Vorrichtung relativkinematisch die Orientierung bzw. Orientierungsänderung des Auges gegenüber der Umgebung berechnet werden.

*4.3.3.4. 1 Relative Orientierung Auge - Vorrichtung*

*4.3.3.4.1.1 Markierung*

Vergleicht man ein relativ zur Vorrichtung festes Bild (Vorrichtungsbild), beispielsweise ein Bild, in dem eine relativ zur Vorrichtung feste Markierung erkennbar ist, mit einem relativ zum Auge festen Bild (Augenbild), beispielsweise einem Bild der Netzhaut oder der Kornea, das sich beispielsweise aus einer aktiven Abtastung ergibt, so kann daraus eine Orientierung des Auges relativ zur Vorrichtung bestimmt werden. Vergleicht man Bilder, die zu verschiedenen Zeitpunkten aufgenommen wurden, so kann daraus eine Orientierungsänderung des Auges relativ zur Vorrichtung bestimmt werden.

Zur Bestimmung der Orientierung des Auges relativ zur Vorrichtung kann ein Vorrichtungs- und ein Augenbild in einem gemeinsamen Koordinatensystem betrachtet werden, vorzugsweise in einem bezüglich der Vorrichtung festen Koordinatensystem (vorrichtungsfestes Koordinatenssystem).

Vorteilhafterweise kann als Vorrichtungsbild ein von einem Teil des Auges abgestrahltes Bild verwendet sein, beispielsweise das Umgebungsreflexbild der Netzhaut oder der Komea oder ein Netzhautreflexbild, in dem eine bezüglich der Vorrichtung feste Markierung erkennbar ist. Besonders bevorzugt können als Vorrichtungsbild bezüglich der Vorrichtung feste Strukturen (Markierung, geometrische Form des Brillenglases etc.) innerhalb des Augenbilds verwendet sein, da dann Vorrichtungs- und Augenbild bereits im selben vorrichtungsfesten Koordinatensystem vorliegen bzw. vorteilhafterweise bereits im selben Bild überlagert sind.

Diese Bilder werden miteinander verglichen, d.h. bestimmte signifikante Strukturen innerhalb des Vorrichtungsbildes (Markierung, Kanten des Brillenglases etc.), die beispielsweise mittels Mustererkennung identifiziert sein können, werden bestimmten signifikanten Strukturen des Augenbildes (beispielsweise der Makula, dem Pupillenmittelpunkt etc.) zugeordnet und ihre Lage relativ zueinander bestimmt.

Anhand dieser relativen Lage läßt sich die Orientierung des Auges relativ zur Vorrichtung bestimmen.

Beispielsweise kann im Vorrichtungsbild bestimmt werden, an welcher Stelle eine Markierung liegt. Im Augenbild kann festgestellt werden, an welcher Stelle die Makula- und/oder Pupillenmitte liegt. Anhand dieser relativen Lage läßt sich die Orientierung des Auges bezüglich der Vorrichtung bestimmen.

Die Orientierungsänderung des Auges relativ zur Vorrichtung kann also bestimmt werden, wenn ein signifikantes Muster der Vorrichtung in einem abgetasteten Bild eines Teils des Auges erkennbar ist.

Hierzu kann beispielsweise eine Markierung an einer optischen Vorrichtung einer erfindungsgemäßen Vorrichtung, beispielsweise auf einem Spiegel, auf einer Linse, in einem Hologramm, an einem Brillenglas vor dem Auge etc. so vorhanden sein, daß Lichtstrahlen besonders stark ("helle" Markierung) oder besonders schwach ("dunkle" Markierung) reflektiert werden. Die Markierung kann beispielsweise holographisch erzeugt sein, vorzugsweise in einem vom Auge nicht wahrgenommenen Wellenlängenbereich, etwa im Infrarotbereich. Als Markierung kann gleichermaßen beispielsweise auch ein Rand eines Brillenglases dienen.

Ein Detektor erfaßt Lichtstrahlen, die von einem Teil des Auges abgestrahlt werden, beispielsweise die Wärmestrahlung von Blutgefäßen der Netzhaut oder von der Netzhaut reflektiertes Licht. In einem daraus erstellten Bild können signifikante Strukturen des Auges erfaßt werden, die relativ zum Auge fest sind, beispielsweise Blutgefäße oder die Makula. In dem Bild ist auch die relativ zur Vorrichtung feste Markierung sichtbar, die beispielsweise auf einem festen Spiegel einer Detektor-Lichtleitanordnung so vorhanden ist, daß ein aus dem Auge abgestrahlter Lichtstrahl, der an der Stelle der Markierung auf den festen Spiegel fällt, nicht oder nur wenig in Richtung des Detektors geleitet wird. Die Markierung erscheint im erfaßten Bild dann dunkel. Aus der relativen Lage der Bilder wenigstens einer signifikanten Struktur des Auges relativ zu einem Bild der Markierung läßt sich die Orientierung der Struktur und damit des Auges relativ zur Markierung und damit zur Vorrichtung bestimmen.

Weitere erfindungsgemäße Markierungen bzw. deren Verwendung sind im Abschnitt "Markierungen" im Abschnitt "optische Vorrichtungen" offenbart.

*4.3.3.4.1.2 Stellung der Vorrichtung*

Zur Bestimmung der Orientierung des Auges relativ zur Vorrichtung kann ein Augenbild in einem bezüglich der Vorrichtung festen Koordinatensystem (vorrichtungsfestes Koordinatenssystem) betrachtet werden.

Beispielsweise kann ein vorrichtungsfestes Korrdinatenssystem durch eine Neutralstellung des Detektorsystems bestimmt sein. In einem in einem solchen Koordinatensystem betrachteten Augenbild können bestimmte signifikante Strukturen (Makula-, Pupillenmittelpunkt etc.) bestimmt und ihre Lage bezüglich des Koordinatensystems festgestellt werden. Anhand dieser Lage kann dann eine Orientierung des Auges relativ zur Vorrichtung bestimmt werden.

*4.3.3.4.2 Relative Orientierungsänderung- Vorrichtung*

Gleichermaßen kann auch eine Änderung der Orientierung des Auges relativ zur Vorrichtungung bestimmt werden, indem analog zu einem oben beschriebenen Verfahren zu verschiedenen Zeitpunkten jeweils eine Orientierung des Auges gegenüber der Vorrichtung bestimmt wird.

Hierzu kann also beispielsweise die relative Lage wenigstens einer bestimmten signifikanten Strukur eines Augenbildes, das zu verschiedenen Zeitpunkten abgetastet wurde, innerhalb eines vorrichtungsfesten Korrdinatensystems und/oder relativ zu einem Vorrichtungsbild, insbesondere etwa einer Markierung, bestimmt werden. Aus der Änderung dieser relativen Lage zwischen den verschiedenen Zeitpunkten der Abtastung kann dann eine Orientierungsänderung des Auges gegenüber der Vorrichtung ermittelt werden.

Gleichermaßen können auch zwei Augenbilder, die zu verschiedenen Zeitpunkten abgetastet wurden, (beispielsweise durch ein Korrelationsverfahren oder durch Drehung und Verschiebung) zur Deckung gebracht werden. Aus den hierbei auftretenden Werten (beispielsweise Korrelationsfaktoren oder Drehwinkel und Verschiebung) läßt sich wiederum die Orientierungsänderung des Auges gegenüber der Vorrichtung bestimmen.

*Vollinhaltliche Bezugnahme auf andere Anmeldungen. Offenbarungen*

Wie in dieser Beschreibung verdeutlicht, läßt sich die vorliegende Erfindung in vorteilhafter Weise im Zusammenhang mit den in den Offenlegungsschriften WO 0231580, WO 0231577, WO 0231578, WO 0231579, DE 101 27 826, WO 02097511, DE 196 31 414 A1 und DE 197 28 890 A1 beschriebenen Systemen, Vorrichtungen und Verfahren verwenden. Auch im Zusammenhang mit der in der am 8. Oktober 2001 von der Anmelderin dieser Anmeldung eingereichten Anmeldung mit dem Titel "Informationssystem und Verfahren zum Zurverfügungstellen von Informationen unter Verwendung eines holographischen Elements" offenbarten Erfindung läßt sich die vorliegende Erfindung in vorteilhafter Weise anwenden.

*Bevorzugte Ausführungsformen*

Bevorzugt erfolgt die Bestimmung der Lage und/oder Orientierung, insbesondere der Blickrichtung, des Auges wahrnehmungslos. Dadurch wird eine Störung des Anwenders durch virtuelle Punkte in seinem Blickfeld vermieden.

Bevorzugt ist einen erfindungsgemäße Vorrichtung tragbar, insbesondere in Form einer Brille, ausgebildet. Dadurch ist es beispielsweise möglich, die Vorrichtung auch in Autos, Flugzeugen etc. einzusetzen und jeweils die Blickrichtung des Auges zu bestimmen.

Bevorzugt projiziert ein erfindugsgemäßes Projektionssystem ein Infrarotlichtstrahl auf das Auge, dessen Durchmesser im Vergleich zum Pupillendurchmesser sehr klein ist. Bevorzugt wird der okulare, insbesondere der retinale, Reflex des Strahls erfaßt. Entgegen einem Vorurteil im Stand der Technik reicht bereits ein Lichstrahl mit einem solch kleinen Durchmesser aus, um ein ausreichenden reflektierten Strahl zu erzeugen. Vorteilhafterweise spielt bei der Abtastung mit Lichtstrahlen kleiner Durchmesser die Krümmung abgetasteter Teile des Auges keine wesentliche Rolle, verursacht insbesondere keine wesentlichen Verzerrungen des Bildes.

Bevorzugt umfaßt eine erfindungsgemäße Vorrichtung einen in einem Infrarotlichtstrahl angeordneten Teilerspiegel, der nur einen geringen Anteil des Infrarotlichtstrahls durchläßt und einen entsprechend großen Anteil des darauffallenden okularen Reflexes in Richtung einer Detektorvorrichtung reflektiert. Dadurch wird Licht auf demselben Strahlengang auf bzw. ins Auge projiziert bzw. vom bzw. aus dem Auge reflektiert, wodurch beispielsweise gewährleistet sein kann, daß der Detektor stets ein Signal empfängt und nicht erst auf den beleuchteten Punkt eingestellt werden muß.

Bevorzugt projiziert ein erfindungsgemäßes Projektionssystem Licht pixelweise mit einer vorgegebenen Pixelfrequenz auf das Auge. Besonders bevorzugt moduliert ein erfindungsgemäßes Projektionssystem das projizierte Licht mit einer Frequenz, die höher als die Pixelfrequenz ist. Hierdurch ist es vorteilhaft möglich, innerhalb eines an einem Punkt bzw. Pixel reflektierten Lichtstrahls Informationen zu übertragen, beispielsweise über den Zeitpunkt der Emittierung oder um den Lichtstrahl vom Ungebungslicht unterscheiden zu können.

Bevorzugt erfolgt keine aktive Beleuchtung des Auges und das Detektorsystem führt eine pixelweise Abtastung des vom Auge zurückreflektierten Umgebungslichtes und/oder des vom Auge emittierten Lichts durch. Dabei ist vorteilhafterweise kein Projektionssystem notwendig.

Bevorzugt weist eine erfindungsgemäße Vorrichtung eine vor dem Auge positionierbaren Fläche auf, die Markierungsbereiche, die einen darauffallenden, vom Projektionssystem stammenden Projektionsstrahl vollständig in Richtung des Detektorsystems zurückreflektieren, sowie Normalbereiche, die einen darauffallenden, vom Projektionssystem stammenden Projektionsstrahl in Richtung Augenmitte lenken. Anhand der Markierungsbereich kann beispielsweise die Orientierung des Auges bezüglich der Markierungsbereiche und damit der erfindungsgemäßen Vorrichtung bestimmt werden.

Bevorzugt bestimmt eine erfindungsgemäße Vorrichtung die Lage und/oder Orientierung des Auges bezüglich seiner Umgebung dadurch, daß das Detektorsystem sowohl die Netzhautstruktur des Auges als auch das darauf überlagerte Umgebungsreflexbild erfaßt, die Position der Fovea anhand der Netzhautstruktur ermittelt und den von der Fovea anvisierten Bereich der Umgebung mittels einer Mustererkennung identifiziert.

Bevorzugt erfaßt eine erfindungsgemäße Vorrichtung eine Darstellung zumindest ausgewählter Bereiche der Retina und legt diese in einem Zwischenspeicher ab. Besonders bevorzugt nimmt eine erfindungsgemäße Vorrichtung zur Bestimmung einer Veränderung der räumlichen Position des Auges einen Vergleich der abgelegten Darstellung mit Informationen vor, die die Vorrichtung aus von der Retina abgetastetem, während einer aktuellen Scanbewegung erfaßtem Licht gewonnen hat. Hierduch kann beispielsweise vorteilhaft ein rascher Vergleich möglich sein.

Bevorzugt umfaßt eine erfindungsgemäße Vorrichtung eine eine vorbestimmte geometrische Form aufweisenden, vor dem Auge positionierbaren Fläche, über die Licht vom Projektionssystem in das Auge projiziert werden kann, wobei die geometrischen Form der Fläche dazu herangezogen wird, die Relativlage mindestens eines charakteristischen Bereichs der Retina bezüglich des optischen Detektor- und/oder Projektionssystems zu bestimmen. Vorteilhafterweise ist dabei keine zusätzliche Markierung notwendig.

### Ausführungs- und Anwendungsbeispiele

Im folgenden werden zunächst einige der oben allgemein dargestellten Merkmale bzw. Begriffe näher erläutert bzw. anhand von Ausführungen der vorliegenden Erfindung näher erklärt. Zur besseren Übersichtlichkeit wird dabei auf die Gliederung der oben gegebenen Zusammenfassung der Erfindung Bezug genommen. Dabei wird expressis verbis darauf hingewiesen, daß die im folgenden gemachten Ausführungen exemplarisch illustrieren sollen und keinerlei Einschränkung der in der Zusammenfassung allgemein dargestellten Merkmale bzw. Begriffe darstellen. Im Anschluß werden einige bevorzugte Ausführungen der vorliegenden Erfindung angegeben.

Hierzu zeigt:

Fig. 1 eine Ausführung einer erfindungsgemäßen Vorrichtung in Form einer Brille;
Fig. 2 ein menschliches Auge im Längsschnitt;
Fig. 3a einen Verlauf der Strahlstärke $I_e$ über der Zeit;
Fig. 3b einen anderen Verlauf der Strahlstärke $I_e$ über der Zeit;
Fig. 4 einen steuerbaren Laser als Strahler;
Fig. 5 einen Anordnung von Strahler und Detektor mit gemeinsamer Lichtleitvorrichtung;
Fig. 6 eine Ausführung einer erfindungsgemäßen Vorrichtung in Form einer Brille;
Fig. 7a eine Ausführung einer erfindungsgemäßen Vorrichtung in Form einer Brille;
Fig. 7b vergrößert die Strahler-Detektor-Kombination aus Fig. 7a;
Fig. 8 ein Bewegungsmuster zur Bestimmung des Pupillenmittelpunkts;
Fig. 9 ebenfalls ein Bewegungsmuster zur Bestimmung des Pupillenmittelpunkts;
Fig. 10 ein Bewegungsmuster zur Bestimmung des Makulamittelpunkts;
Fig. 11 ebenfalls ein Bewegungsmuster zur Bestimmung des Makulamittelpunkts;
Fig. 12 ein schematisches Bild der Netzhaut mit spiralförmigem Abtastmuster;
Fig. 13 ein abgetastetes Bild der Netzhaut aus Fig. 12;
Fig. 14 ein schematisches Bild der Netzhaut mit alten und neuen Achsen;
Fig. 15 schematisch ein Auge mit Pupille in Referenz- und verdrehter Lage sowie das entsprechende abgetastete Bild;
Fig. 16 schematisch ein Auge in Referenz- und verdrehter Lage sowie eine Umgebung;
Fig. 16A ein Umgebungsreflexbild der Netzhaut;
Fig. 16B ein Netzhautreflexbild;
Fig. 16C ein Umgebungsreflexbild der Netzhaut bei verdrehtem Auge;
Fig. 16D ein Netzhautreflexbild bei verdrehtem Auge;
Fig. 16E ein Umgebungsreflexbild der Netzhaut bei verdrehter Vorrichtung;
Fig. 17A ein Brillenglas mit Markierung vor einem Auge;
Fig. 17B ein Umgebungs- und ein überlagertes Netzhautreflexbild des Auges aus Fig. 17A;

Fig. 17C die Anordnung aus Fig. 17A bei verdrehtem Auge;
Fig. 17D ein Umgebungs- und ein überlagertes Netzhautreflexbild des Auges aus Fig. 17C;
Fig. 18 einen reflektierten Hauptstrahl und Randstrahlen.

Eine Vorrichtung bzw. ein Verfahren zur Bestimmung der Lage und/oder Orientierung eines Auges nach der vorliegenden Erfindung besteht darin, daß ein von einem Teil des Auges abgestrahltes Lichtsignal mit Hilfe eines Detektorsystems erfaßt und ausgewertet wird.

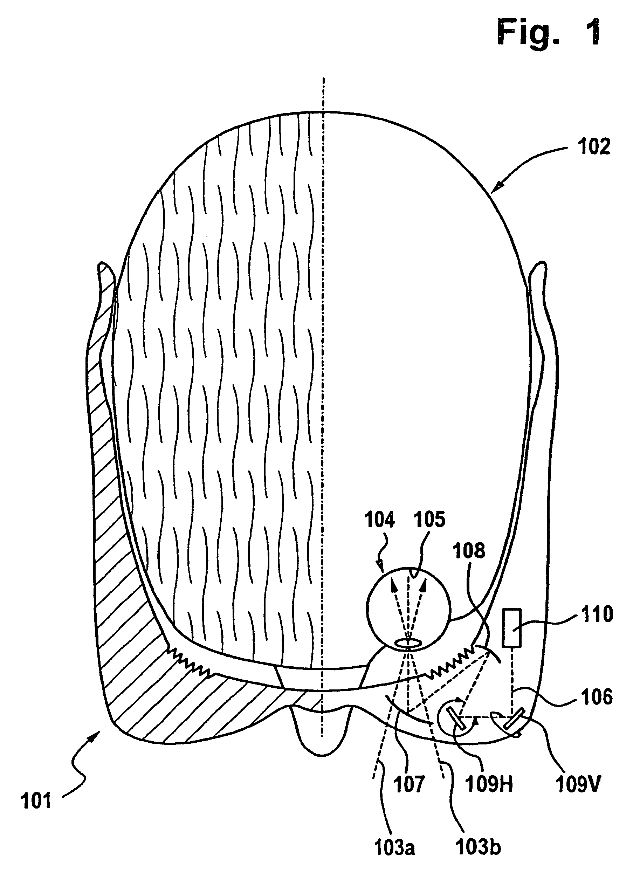

Dies sei zum besseren Verständnis anhand einer Ausführung der vorliegenden Erfindung exemplarisch erläutert. Hierzu zeigt Fig. 1 eine erfindungsgemäße Vorrichtung in einer Ausführung als Brille 101 auf dem Kopf 102 eines Benutzers, wobei die rechte Bildhälfte die Draufsicht von oben auf den Kopf 102 und die linke Bildhälfte einen Schnitt in Höhe etwa des Augenmittelpunktes darstellt. Lichtstrahlen 103a, 103b ("Lichtsignal" in der vorliegenden Ausführung) gelangen aus der Umgebung in das linke Auge 104 des Benutzers und werden an dessen Netzhaut 105 ("Teil des Auges" in der vorliegenden Ausführung) teilweise reflektiert. Ein an einem bestimmten Punkt der Netzhaut reflektierter Lichtstrahl 106 trifft auf einen ersten festen konkaven Spiegel 107 und wird von diesem auf einen zweiten festen konkaven Spiegel 108 gelenkt. Von diesem Spiegel 108 wird der Strahl 106 auf einen horizontal beweglichen Flachspiegel 109H und von diesem auf einen vertikal beweglichen Flachspiegel 109V gelenkt. Vom vertikal beweglichen Flachspiegel 109V wird der Strahl 106 auf einen Photodetektor 110 gelenkt, der den auftreffenden Strahl 106 bezüglich seiner Intensität, Wellenlänge oder dergleichen erfaßt ("Detektorsystem" umfaßt in der vorliegenden Ausführung den Photodetektor sowie die Spiegelanordnung). Durch geeignete Bewegungen der Flachspiegel 109H bzw. 109V können nacheinander Lichtstrahlen in den Photodetektor 110 gelenkt werden, die an verschiedenen Punkten der Netzhaut reflektiert wurden. Die Netzhaut kann so entlang eines vorgegebenen Bewegungsmusters (Scanmuster) abgescannt bzw. abgetastet werden. Aus den dabei erhaltenen Informationen kann eine Orientierung des Auges bestimmt werden ("Auswertung" in der vorliegenden Ausführung).

Zum besseren Verständnis wird im folgenden zunächst in aller Kürze die Funktionsweise und Fachterminologie des Auges vorangestellt.

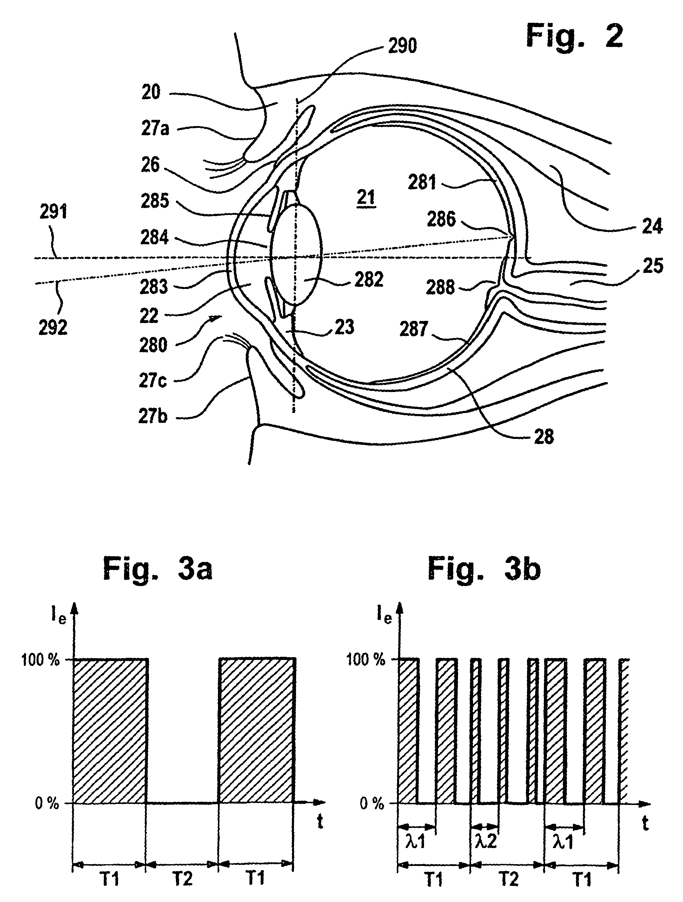

Das in Fig. 2 dargestellte Auge bzw. der Augapfel 280 wird durch die sechs Muskeln 24 in der Augenhöhle (Orbita) 20 bewegt und dadurch seiner Orientierung relativ zum Schädel eingestellt. Oberlied 27a und Unterlied 27b mit Wimpern 27c gehen in die Bindehaut 26 über, die den Raum zwischen Orbita 20 und dem Augapfel 280 verschließt.

Der Augapfel 280 besteht aus einem durchsichtigen, näherungsweise kugelförmigen Glaskörper 21, auf dem in Blickrichtung vorne eine verformbare Linse 282 aufliegt, deren Brennpunkt durch Anspannen bzw. Entspannen von am Umfang angeordneten Ziliarmuskeln 23 veränderbar ist. Unmittelbar vor der Linse 282 ist eine Blende (Regenbogenhaut, Iris) 285 aus gefärbtem Material angeordnet, deren Pupille 284 im Durchmesser muskulär veränderbar ist.

Den hinteren Teil des Glaskörpers umgibt die Lederhaut (Sklera) 28, deren Innenseite von der Aderhaut (Choroidea) 287 überzogen ist. Zwischen Glaskörper 21 und Aderhaut 287 befindet sich die Netzhaut (Retina) 281, die vom Glaskörper gestützt und von der Aderhaut mit Blut versorgt wird. Die Netzhaut umfaßt stark lichtempfindliche Stäbchen zum skotopischen Sehen (im Dämmerlicht) sowie weniger lichtempfindliche Zapfen zum photopischen Sehen (Farbsehen bei Tageslicht). Innerhalb der Netzhaut liegt der sogenannte gelbe Fleck (Makula) mit der Sehgrube (Fovea centralis) 286, dem Bereich des schärfsten Sehens mit einer sehr hohen Zapfendichte. In einem anderen Bereich liegt der blinde Fleck 288, in dem der Sehnerv 25 in die Netzhaut mündet und wo keine Bildinformation aufgenommen werden kann.

Den vorderen Teil des Glaskörpers umgibt die durchsichtige Hornhaut (Kornea) 283 so, daß zwischen Komea und Iris die vordere Augenkammer 22 gebildet wird, die mit einer durchsichtigen Flüssigkeit gefüllt ist. Die Hornhaut geht an ihrem Umfang in die Lederhaut über.

Im optischen System Auge wird beim normalsichtigen entspannten Auge einfallendes paralleles Licht im wesentlichen durch die Hornhaut auf die Netzhaut fokussiert. Durch Veränderung der Brechung der Linse 282 werden Gegenstände in unterschiedlicher Entfernung scharf abgebildet. Als optische Achse 291 des Auges 280 wird im folgenden die Mittelsenkrechte auf die Hauptebene 290 der Linse 282 bezeichnet. Als Blickrichtung 292 hingegen wird die Gerade durch den Pupillenmittelpunkt und die Fovea centralis 286 definiert, da ein Mensch im Regelfall sein Auge so auf einen zu betrachtenden Punkt richtet, daß dieser im Bereich des schärfsten Sehens abgebildet wird. Während im Normalfall optische Achse 291 und Blickrichtung oder Sehachse 292 kaum voneinander abweichen, kann es durchaus vorkommen, daß die Fovea centralis nicht der Pupille gegenüber liegt. Das Auge scheint dann zu schielen.

*zu 1.2 Passive Abtastung*

Aus dem polyspektralen Umgebungslicht können Lichtstrahlen besonders geeigneter Wellenlängen herausgefiltert werden, beispielsweise Licht im grün-gelben Bereich des schärfsten Sehens zwischen 500 nm und 600 nm, um so die Makula besonders gut detektieren zu können, oder Licht im Bereich von ca. 750 nm, bei dem die Reflektivität der Netzhaut am höchsten ist.

*zu 1.3.1.1 Amplitudenmodulation*

Als Amplitudenmodulation wird eine Modulation bezeichnet, bei der wenigstens eine der oben angegebenen charakteristischen Größen in verschiedenen Zeitabschnitten bzw. zu verschiedenen Zeitpunkten jeweils bestimmte Werte aufweisen. Fig. 3a zeigt hierzu exemplarisch die Strahlstärke $I_e$ eines aktiv eingestrahlten Lichts über der Zeit, die in einer ersten Zeitperiode T1 einen Referenzwert von 100% und in einer anschließenden zweiten Zeitperiode T2 einen Wert von 0% aufweist, wobei die Perioden T1 und T2 periodisch aufeinander folgen. Wird ein solches aktiv eingestrahltes Licht zusammen mit dem Ungebungslicht an einem Punkt der Netzhaut reflektiert und in einem Detektor die Strahlstärke des gesamten an diesem Punkt reflektierten Lichtstrahl erfaßt, so ergibt sich aus der Differenz des in der Periode T1 und des in der Periode T2 aufgefangenen Lichts näherungsweise gerade das aktiv eingestrahlte Licht ohne Umgebungslichtanteile.

*zu 1.3.1.2 Frequenzmodulation*

Als Frequenzmodulation wird die Änderung einer Periodizität wenigstens einer der oben angegebenen charakteristischen Größen bezeichnet. Fig. 3b zeigt hierzu wiederum exemplarisch die Strahlstärke $I_e$ eines aktiv eingestrahlten Lichts über der Zeit, der in einer ersten Zeitperiode T1 gepulst mit einer Periodendauer $\lambda 1$ und in einer anschließenden zweiten Zeitperiode T2 gepulst mit einer Periodendauer $\lambda 2 \neq \lambda 1$ eingestrahlt wird. Wird ein solches aktiv eingestrahltes Licht zusammen mit dem Umgebungslicht an einem Punkt der Netzhaut reflektiert und in einem Detektor erfaßt, so kann anhand der Periodendauer $\lambda 1$ bzw. $\lambda 2$ bestimmt werden, ob der Lichtstrahl während der Periode T1 oder der Periode T2 ausgestrahlt wurde. Allgemein kann beispielsweise die Periodizität zwischen einem nicht dargestellten Zeitpunkt TA und einem nicht dargestellten Zeitpunkt TE gemäß einer vorgegebenen Relation geändert werden, also beispielsweise zwischen TA und TE linear abnehmen. Dann kann aus der Periodizität des reflektierten und erfaßten Lichtstrahls der Zeitpunk zwischen TA und TE bestimmt werden, zu dem der Strahl emittiert wurde. Dies ermöglicht beispielsweise eine Laufzeitmessung und damit beispielsweise bei bekannter Geometrie von Detektor- bzw. Projektionssystem die Bestimmung der Entfernung zwischen dem reflektierenden Punkt des Auges und dem Detektor- bzw. Projektionssystem als Produkt der Lichtgeschwindigkeit und der Laufzeit. Gleichermaßen ermöglicht es, aktiv eingestrahltes Licht von Umgebungslicht zu unterscheiden.

Es können auch eine oder mehrere charakteristische Größen des aktiv eingestrahlten Lichtstrahls gleichzeitig oder nacheinander amplituden- und/oder frequenzmoduliert sein. In Fig. 3b ist zwischen der Periode T1 und der Periode T2 sowohl die Periodendauer (Frequenzmodulation) als auch die Pulsbreite (Pulscodemodulation) verändert.

*zu 1.3.2 Anpassung der aktiven Lichtstärke*

Die aktive Abtastung weist gegenüber der passiven Abtastung den Vorteil auf, daß sichergestellt werden kann, daß stets Licht ausreichender Intensität aus dem Auge abgestrahlt wird, indem aktiv entsprechend viel Licht eingestrahlt wird. Beispielsweise kann die Strahlstärke des Strahlers, der das Licht aktiv aussendet, so angepaßt werden, daß der Detektor den daraus resultierenden reflektierten Lichtstrahl ausreichend deutlich erfassen kann. Kann der Detektor den aktiv eingestrahlten und reflektierten Lichtstrahl nicht mehr erfassen, kann die Lichtstärke entsprechend erhöht werden, registriert der Detektor eine sehr hohe Lichtstärke des aktiv eingestrahlten und reflektierten Strahls, wird die Lichtstärke entsprechend reduziert. Somit ist die Bestimmung der Orientierung des Auges auch bei Dunkelheit (virtual-reality-Brille, Nachtsichtgerät etc.) oder stark wechselnden Lichtverhältnissen (Tunneldurchfahrt etc.) möglich.

*zu 1.3.3.1 Punktuelle Beleuchtung*

Erfindungsgemäß kann ein Lichtstrahl von einem Strahler, beispielsweise einem Laser, emittiert und mittels einer Projektions-Lichtleitanordnung so gelenkt werden, daß nacheinander, i.e. sequentiell verschiedene Punkte auf dem Teil des Auges beleuchtet werden. Dies kann beispielsweise mittels einer Vorrichtung durchgeführt werden, die analog zu der in Fig. 1 dargestellten aufgebaut ist, wobei der Detektor 110 durch einen Strahler ersetzt ist (Umkehrbarkeit des Strahlengangs). Durch eine entsprechende Bewegung der beiden Flachspiegel 109H bzw. 109V wird der vom Strahler emittierte Lichtstrahl dann sequentiell auf verschiedene Punkte der Netzhaut 105 gelenkt. Dabei folgt der Endpunkt des Lichtstrahls auf dem Auge einem Bewegungsmuster (Projektionsbewegung).

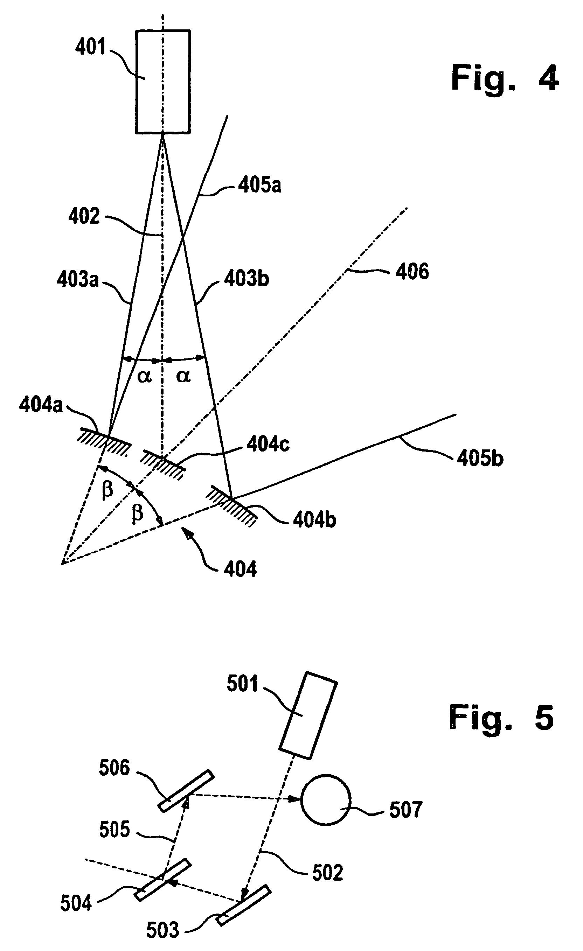

Alternativ kann auch der Strahler selbst den emittierte Lichtstrahl auf verschiedene Punkt des Teils des Auges lenken. Beispielsweise kann ein steuerbarer Laser den emittierten Lichtstrahl so auf einen gekrümmten oder mehrteiligen Spiegel aussenden, daß jeweils ein bestimmter Spiegelbereich unter einem bestimmten Winkel getroffen wird. Die Spiegelbereich sind gegeneinander um verschiedene Winkel geneigt, so daß ein im gleichen Winkel zum gesamten Spiegel auftreffender Lichtstrahl an verschiedenen Spiegelbereichen in verschiedene Richtungen abgelenkt wird. Fig. 4 zeigt hierzu schematisch einen steuerbaren Laser 401, der einen Lichtstrahl maximal um den Winkel $\alpha$ von der

Hautachse 402 ablenken kann. Damit ergeben sich bei maximaler Ablenkung α in der Zeichenebene die Randstrahlen 403a bzw. 403b. Diese werden an den entsprechenden Spiegelbereichen 404a bzw. 404b eines Spiegels 404 reflektiert, wobei diese Spiegelbereiche geeignet gegeneinander geneigt sind. Die reflektierten Randstrahlen 405a bzw. 405b weisen gegenüber dem an einem gegenüber den Spiegelbereichen 404a bzw. 404b geneigten Spiegelbereich 404c reflektierten Hauptstrahl 406 einen Winkel β auf, der größer ist als der ursprüngliche Winkel α. Auf diese Weise können auch mit einem Strahler, der nur eine kleine Ablenkung des emittierten Strahls realisieren kann, ausreichend große Abtastbereiche verwirklichen. Die Oberfläche des Spiegels 404 mit den entsprechenden Spiegelbereichen 404a, 404b bzw. 404c ergibt sich geometrisch aus den entsprechenden Winkeln und Abständen.

*zu 1.3.3.1. Erfassung von Licht aus der ganzen Pupille*

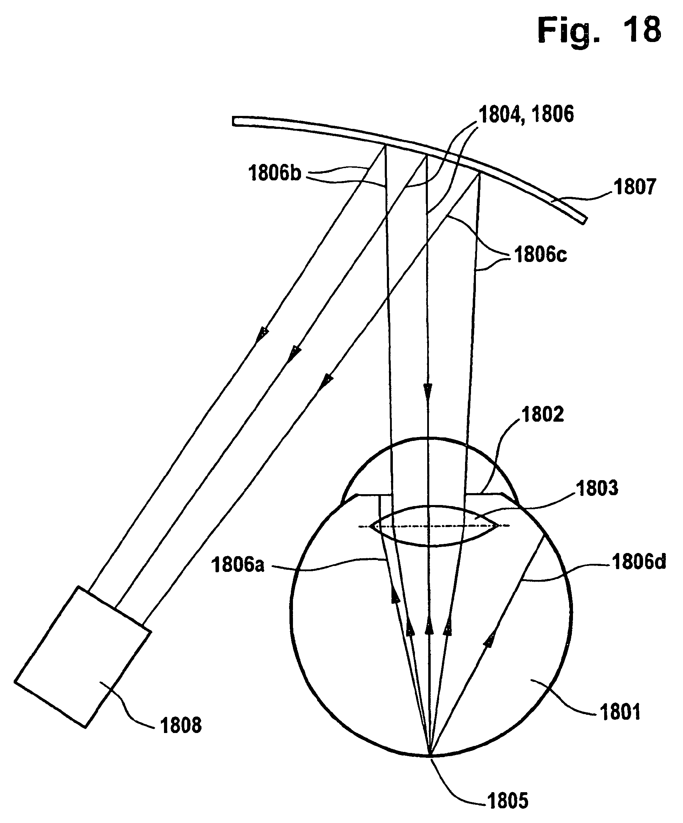

Fig. 18 zeigt einen in ein Auge 1801 mit Pupille 1802 und Linse 1803 eingestrahlten Lichtstrahl 1804, der einen sehr kleinen Durchmesser aufweist. Dieser wird an einem Punkt 1805 der Netzhaut des Auges 1801 größtenteils als Hauptstrahl 1806 zurückgestrahlt (reflektiert bzw. gestreut), der im dargestellten Fall zufälligerweise dem eingestrahlten Lichtstrahl 1804 entspricht. Diese besondere Konfiguration ist hier nur der übersichtlicheren Darstellung wegen gewählt und bedeutet keine Einschränkung der Ausführungen. In diese Richtung wird nicht der gesamte Lichtstrahl 1804 parallel als Hauptstrahl 1806 zurückreflektiert, ein Teil wird auch als Strahlen 1806a, 1806b, 1806c, 1806d in andere Richtungen reflektiert, wobei sich beispielsweise eine keuleförmige Intensitätsverteilung des zurückgestrahlten Lichtes um den Hauptstrahl ergibt. Ein Teil dieser in andere als der Reflexionsrichtung reflektierten Lichtstrahlen gelangt durch die Pupille 1802 aus dem Auge heraus und verläuft näherungsweise koaxial zum reflektierten Hauptstrahl 1806. Hierzu sind die beiden Randstrahlen 1806b, 1806c eingezeichnet, die gerade noch die Pupille 1802 passieren. Die Strahlen 1806a, 1806d werden so reflektiert, daß sie die Pupille 1802 nicht mehr passieren. Eine Detektor-Lichtleitanordnung 1807 (vereinfacht in Form eines Spiegels dargestellt) ist so ausgebildet, daß auch diese Randstrahlen zu einem Detektor 1808 geleitet und von diesem erfaßt werden, wodurch sich vorteilhaft die erfaßte Lichtmenge erhöht.

*zu 3.2.1 Spiegel*

In einer bevorzugten Ausführung umfaßt die Detektor-Lichtleitanordnung einen oder mehrere Spiegel, deren Orientierung zueinander, zum Auge, dessen Orientierung bestimmt werden soll, und zum Detektor so einstellbar ist, daß Lichtstrahlen, die an bestimmten Punkten des Auges reflektiert wurden, sequentiell auf den Detektor gelenkt werden.

Die Orientierung der beweglichen Spiegel ist so steuerbar, daß ein gewünschtes Bewegungsmuster abgetastet werden kann. Für ein in der Regel zweidimensionales Muster (beispielsweise ein Gitter, eine Spirale oder dergleichen) können beispielsweise, wie in Fig. 1 gezeigt, zwei Flachspiegel vorhanden sein, die um zwei voneinander verschiedene Achsen bewegbar sind. Alternativ kann beispielsweise auch ein Taumelspiegel verwendet werden.

Neben den beweglichen Spiegeln können, wie exemplarisch in Fig. 1 gezeigt, auch feste Spiegel vorhanden sein. Ein erster fester Spiegel kann beispielsweise ein halbdurchlässiger Spiegel sein, der Licht in Richtung auf das Auge passieren läßt und aus dem Auge austretendes Licht ablenkt. Dadurch ist es möglich, den Detektor außerhalb des Blickfeldes des Auges anzuordnen. Weitere feste Spiegel können, wie ebenfalls in Fig. 1 exemplarisch gezeigt, so angeordnet sein, daß der Detektor bzw. die beweglichen Spiegel besonders günstig, beispielsweise außerhalb des Blickfelds des Auges bzw. an einer geeigneten Stelle der Vorrichtung anordenbar sind.

*zu 3.3.1 Strahler*

Vorteilhafterweise ist wenigstens eine der physikalischen Größen des vom Strahler emittierten Lichts einstellbar. Beispielsweise kann ein Strahler Lichtstrahlen mit einem bestimmten zeitlichen Muster bezüglich Intensität, Wellenlänge oder dergleichen aussenden, wie dies exemplarisch in Fig. 3a, 3b angedeutet ist.

*zu 3.3.3 Projektions-Lichtleitanordnung*

Um vom Strahler emittiertes Licht auf den Bereich des Auges zu lenken, von dem es reflektiert werden und anschließend im Detektorsystem erfaßt werden soll, kann eine Projektions-Lichtleitanordnung vorhanden sein, in die vom Strahler emittiertes Licht eintritt und die dieses Licht auf den gewünschten Bereich lenkt.

Insbesondere kann die Detektor-Lichtleitvorrichtung selbst einen Teil der Projektions-Lichtleitanordnung bilden, indem das vom Strahler emittierte Licht gegensinnig parallel in den Strahlengang vor, in bzw. nach der Detektor-Lichtleitvorrichtung eintritt.

Exemplarisch zeigt hierzu Fig. 5 einen Strahler 501, der einen Lichtstrahl 502 emittiert. Dieser wird von einem Spiegel 503 auf einen halbdurchlässigen Spiegel 504 gelenkt, der den Lichtstrahl 502 möglichst unverändert passieren läßt. Ein halbdurchlässige Spiegel kann ein Teilerspiegel sein, der bevorzugt Licht in einer Richtung durch den Spiegel möglichst unverändert passieren lassen und Licht in der Gegenrichtung möglichst vollständig reflektieren. Der Lichtstrahl 502 tritt dann in eine Detektor-Lichtleitanordnung ein, wie sie beispielsweise in Fig. 1 durch die beweglichen Flachspiegel 109V, 109H und die beiden konkaven Spiegel 107, 108 gebildet wird. Von dieser wird der Lichtstrahl auf einen bestimmten Bereich des Auges gelenkt und/oder fokussiert, an diesem Bereich reflektiert und trifft auf dem gleichen Weg als reflektierter Lichtstrahl 505 wieder auf den halbdurchlässigen Spiegel 504. Dieser reflektiert einen möglichst großen Anteil des eintreffenden Lichtstrahls 505 auf einen weiteren Spiegel 506, der den an einem Teil des Auges reflektierten Lichtstrahl 505 in einen Detektor 507 lenkt, wo wenigstens eine charakteristische Größe dieses Lichtststrahls erfaßt wird.

*Ausfhührunsgbeispiele zur optischen Vorrichtung*

Nachfolgend werden zur Verdeutlichung der oben dargestellten Merkmale anhand der Figuren einige mögliche Ausführungen der optischen Vorrichtung nach der vorliegenden Erfindung anhand einer bevorzugten Ausführung in Form einer Brille beschrieben. Die Erfindung ist nicht auf eine Brille beschränkt, sie kann beispielsweise auch an einem am Kopf tragbaren Gestell, einem Helm oder dergleichen angeordnet sein. Gleichermaßen kann sie beispielsweise auch an einem ortsfesten Gerät befestigt sein, das die von der Vorrichtung gelieferten Informationen bezüglich der Orientierung des Auges verwendet, etwa an einem Gerät zur Laserchirurgie oder dergleichen. Gleichermaßen kann sie an einem relativ zur Umgebung und relativ zum Träger beweglichen Objekt wie beispielsweise einem tragbaren elektronischen Notizbuch, einem Laptop oder dergleichen befestigt sein. Weiters wird bei den beschriebenen Ausführungen als reflektierender Teil des Auges die Netzhaut verwendet, wie oben beschrieben können gleichermaßen auch andere Teile des Auges (Hornhaut, Iris etc.) verwendet sein.

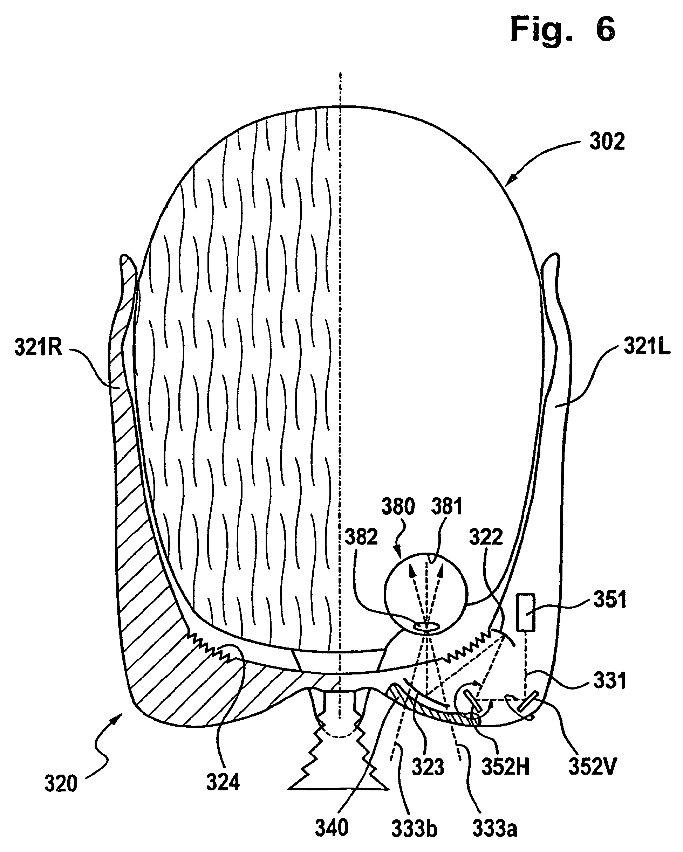

In Fig. 6 trägt der Anwender 302 eine erfindungsgemäße Vorrichtung in Form einer Brille 320 mit einem linken bzw. rechten Bügel 321L bzw. 321R. Die linke Bildhälfte zeigt die Draufsicht von oben, die rechte Bildhälfte einen durch den linken Bügel 321 L verlaufenenden Schnitt.

Aus der Umgebung fallen Lichtstrahlen 333a, 333b in das Auge 380 und werden von dessen Hornhaut und Linse 382 auf die Netzhaut 381 fokussiert. Die Netzhaut reflektiert diese Strahlen teilweise so, daß diese durch die Linse 382 wieder aus dem Auge 380 austreten. Die Reflektivität der Netzhaut beträgt dabei je nach Wellenlänge des einfallenden Lichts ca. 0.2-10%. Ein so reflektierter Strahl 331 wird durch einen ersten halbdurchlässigen konkaven Spiegel 323, der Licht in Richtung auf das Auge möglichst unverändert passieren läßt und aus dem Auge austretendes Licht möglichst vollständig reflektiert, und einen zweiten konkaven Spiegel 322 auf einen horizontal beweglichen ersten Flachspiegel 352H, von diesem auf einen vertikal beweglichen zweiten Flachspiegel 352V und von diesem auf einen Photodetektor 351 gelenkt. Je nach Stellung der beiden Flachspiegel 352H, 352V wird jeweils ein ganz bestimmter Lichtstrahl 331, der an einem bestimmten Punkt der Netzhaut reflektiert wurde, in den Detektor 351 gelenkt und in diesem mittels einer Fokussiereinrichtung wie beispielsweise einer Sammellinse auf eine Photozelle, die die Intensität des Lichtstrahls erfaßt. Mit Intensität des Lichtstrahls ist stets eine geeignete, von einem Detektor meßbare Größe des reflektierten Lichtstrahls wie beispielsweise die Bestrahlung(sstärke), Strahldichte, Lichtstärke, Helligkeit oder dergleichen bezeichnet. Durch eine entsprechende Bewegung der beiden Flachspiegel werden somit sequentiell diejenigen Lichtstrahlen erfaßt, die an Punkten längs einer entsprechenden Trajektorie auf der Netzhaut reflektiert wurden, das Umgebungsreflexbild wird als Bildpunktfolge abgetastet.

Beispielsweise werden bei festem ersten Flachspiegel 352H und gleichförmig um seine horizontale Achse gedrehtem zweiten Flachspiegel 352V die Bildpunkte einer vertikalen Geraden auf der Netzhaut punktuell abgetastet. Bei gleichförmig um seine vertikale Achse gedrehtem ersten Flachspiegel 352H und festem zweiten Flachspiegel 352V werden horizontale Geraden auf der Netzhaut punktuell abgetastet. In einer bevorzugten Ausführung der vorliegenden Erfindung werden die beiden Flachspiegel sinusförmig auf- und ab- bzw. hin- und herbewegt, so daß die auf der Netzhaut 381 abgetastete Kurve Kreise (bei gleicher Amplitude in beiden Spiegeln), Ellipsen (bei unterschiedlichen Amplituden in beiden Spiegel), Spiralen (bei wachsenden Amplituden in beiden Spiegeln) oder andere geeignete Kurven umfassen können.

In einer weiteren Ausführung sind die beiden Flachspiegel 352H, 352V durch einen um wenigstens zwei Achsen beweglichen Taumelspiegel ersetzt, der so angeordnet ist, daß er Lichtstrahlen vom zweiten konkaven Spiegel 322 in den Phtotodetektor 351 lenkt. Durch entsprechende Ansteuerung des Taumelspiegels können ebenfalls Bildpunktfolgen längs beliebiger Trajektorien auf der Netzhaut vom Photodetektor 351 erfaßt werden.

Eine Lichtfalle 324 verhindert, daß Licht aus unerwünschten Einfallsrichtungen auf das Auge bzw. die Spiegel fällt.

Statt des ersten konkaven Spiegels 323 kann das Brillenglas 340 so verspiegelt sein, daß es zumindest die vom Auge reflektierten Lichtstrahlen möglichst vollständig reflektiert. Um auch bei verdrehten Augenstellungen vom Auge reflektierte Lichtstrahlen geeignet zum zweiten Hohlspiegel 322 umzulenken, muß die spiegelnde, dem Auge zugewandte Oberfläche des Brillenglases eine entsprechende Form aufweisen. Insofern wird vollinhaltlich auf die am 8.10.2001 vom gleichen Anmelder eingereichte PCT-Anmeldung "Informationssystem und Verfahren zum Zurverfügungstellen von Informationen unter Verwendung eines holographischen Elements" genommen. Die reflektierenden Oberfläche der gewünschten Form kann bevorzugt mittels einer entsprechend ausgebildeten holographischen Beschichtung des Brillenglases 340 emuliert sein.

An Stelle der hier und im folgenden beschriebenen verschiedenen reflektierenden Anordnungen (Flach-, Taumel- bzw. konkave Spiegel, spiegelnde Oberflächen, holographische Beschichtungen etc.) können in Ausführungen der vorliegenden Erfindung stets auch alle weiteren bekannten Lichtbrechungs- bzw. - reflektionsmechanismen, beispielsweise elektroholographische oder dergleichen, verwendet sein, um die Lichtstrahlen vom Strahler zum Auge bzw. vom Auge zum Detektor zu lenken.

Bei einer Vorrichtung zur aktiven Abtastung ist zusätzlich ein Strahler vorhanden, der Licht ausstrahlt, beispielsweise ein Laser, eine Infrarot-Lampe, eine Leuchtdiode oder dergleichen. In einer Ausführung ist der Photodetektor 351 im Zentrum eines Strahlers angeordnet. Somit wird zur Projektion von Licht auf das Auge die selben Vorrichtungen (Flach-bzw. Taumelspiegel 352H, 352V; zweiter konkaver Spiegel 322; erster konkaver Spiegel 323 bzw. verspiegeltes Brillenglas 340 bzw. holographisch beschichtetes Brillenglas 340) und somit der selbe Strahlengang wie bei der Erfassung der reflektierten Lichtstrahlen verwendet. Eine solche Anordnung weist den Vorteil auf, daß möglicherweise vorhandene systematische Fehler durch Abweichungen der Spiegeloberflächen oder dergleichen bei Projektion und Abtastung identisch sind. Nutzt man das Projektionssystem zusätzlich oder alternativ zur Projektion von Signalen auf die Netzhaut, so wird die Korrelation zwischen der durch den Photodetektor 351 erfaßten Bildpunktfolge auf der Netzhaut, die beispielsweise das Umgebungsflexbild des wahrgenommenen Bildes im Auge sein kann, und der hineinprojizierten Information, die beispielsweise eine virtuelle Markierung eines Gegenstandes in diesem Bild sein kann, nicht durch diese systematischen Fehler beeinträchtigt. Darüber hinaus verringert die gemeinsame Nutzung der selben Vorrichtungen das Gewicht der optischen Vorrichtung und es ist sichergestellt, daß von Strahler emittiertes Licht auch stets zumindest zum Teil zum Detektor zurück gelangt.

Alternativ kann in einer (nicht dargestellten) Ausführung der vorliegenden Erfindung eine zusätzliche zweite Anordnung von Flach-bzw. Taumelspiegel, zweitem konkaven Spiegel und erstem konkaver Spiegel bzw. verspiegeltem Brillenglas bzw. holographisch beschichtete Brillenglas analog zu einer der oben beschriebenen Ausführungen vorhanden sein, bei der an Stelle des Photodetektors 351 ein Strahler vorhanden ist, so daß reflektiertes Licht durch die oben beschriebene Anordnung mit dem Detektor 351 erfaßt und gleichzeitig durch die zweite Anordnung Licht auf das Auge eingestrahlt werden kann.

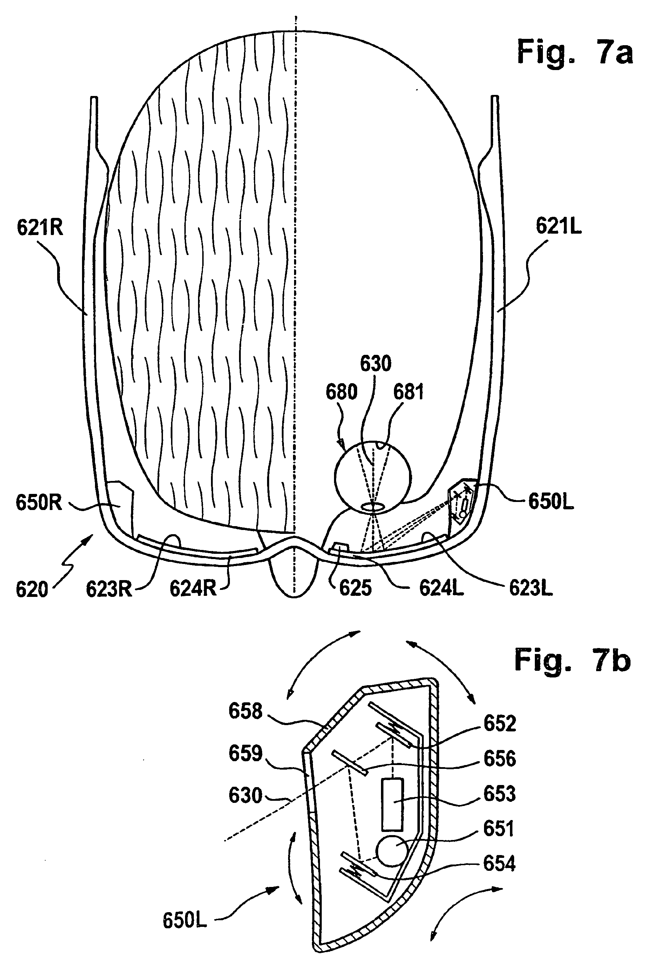

Alternativ kann zur aktiven Abtastung eine der im folgenden anhand Fig. 7a, 7b beschriebenen Ausführungen verwendet werden.

In der Detailzeichnung 7b ist ein kombiniertes Projektions- und Detektorsystem 650L gezeigt, das zwischen dem linken Brillenglas 624L und dem linken Bügel 621 L angeordnet ist. Selbstverständlich kann diese oder eine andere Ausführung der vorliegenden Erfindung stets auch alternativ oder zusätzlich analog auf der rechten Seite angeordnet sein, in diesem Fall also zwischen rechtem Brillenglas 624R und rechtem Bügel 621R. Das linke Brillenglas 624L ist bei der vorliegenden Ausführung auf der dem Auge zugewandten Seite mit einer holographischen Beschichtung 623L versehen, die wie oben beschrieben eine Reflektions- bzw. Fokussieroberfläche emuliert.

Das kombinierte Projektions- und Detektorsystem 650L umfaßt ein abgeschlossenes Gehäuse 658 mit einem lichtdurchlässigen Fenster 659. Dadurch können zwar Lichtstrahlen in das Gehäuse ein- und aus dem Gehäuse austreten, störende Fremstoffe wie Staub oder dergleichen werden jedoch am Eintritt gehindert. In dem Gehäuse 658 ist ein Strahler 653 und ein Photodetektor 651 angeordnet. Ein vom Strahler 653 ausgestrahlter Lichtstrahl 630 trifft auf einen ersten Taumelspiegel 652, der durch einen geeigneten Antrieb so positioniert wird, daß er den Lichtstrahl durch einen Teilerspiegel 656 so auf die holographische Beschichtung 623L lenkt, daß der Lichtstrahl von dort auf einen gewünschten Punkt auf der Netzhaut 681 des Auges 680 reflektiert wird. Von diesem Punkt wird der Strahl 630 zumindest teilweise auf dem selben Weg zurück auf die holographische Beschichtung 623L und von dieser auf dem selben Weg auf den Teilerspiegel 656 reflektiert. Der Teilerspiegel 656 lenkt einen Teil des ankommenden Strahls 630 auf einen zweiten Taumelspiegel 654, der wiederum durch einen geeigneten Antrieb so positioniert wird, daß der den Strahl 630 in den Photodetektor 651 ablenkt, der diesen erfaßt.

In einer vorteilhaften Ausführung ist der Teilerspiegel 656 die dem zweiten Taumelspiegel 654 zugewandte Seite stark reflektierend, so daß ein möglichst hoher Prozentsatz des vom Auge zurückreflektierten Lichtstrahls 630 in den Photodetektor 651 gelenkt wird.

Vorteilhafterweise ist am Brillenglas 624L eine Markierung 625 angebracht. Diese kann zum einen dazu verwendet werden, einen Referenzwert bezüglich der Lichtverhältnisse und der Reflektivität der einzelnen Komponenten im Strahlengang festzulegen. Hierzu wird durch den ersten Taumelspiegel 652 der Lichtstrahl 630 mit bekannter Intensität auf die Markierung 625 gelenkt und von dieser teilweise in die entgegengesetzte Richtung zurückreflektiert. Hierzu kann die dem kombinierten Projektions- und Detektorsystem 650L zugewandte Oberfläche der Markierung 625 entsprechend ausgebildet sein, beispielsweise voll reflektierend. Der reflektierte Strahl 630 wird am Teilerspiegel 656 auf den zweiten Taumelspiegel 654 und von diesem in den Photodetektor 651 abgelenkt, wo die Intensität registriert und als Referenzwert beispielsweise für 100% Reflektion festgelegt wird. Damit ist es möglich, die optische Vorrichtung auch an wechselnde Lichtverhältnisse anzupassen.

Der Strahler und/oder der Detektor kann bei der vorliegenden Erfindung stets durch eine Lichtaustritts- bzw. Lichteintrittsvorrichtung ersetzt sein, die an Stelle der Lichtaustritts- bzw. Lichteintrittsöffnung des Strahlers bzw. Detektors angeordnet und mit einem geeigneten Lichtleiter, insbesondere einem Glasfaserkabel oder dergleichen lichtleitend verbunden ist, der an seinem anderen Ende wiederum mit einem Strahler bzw. Detektor lichtleitend verbunden ist. Dadurch können Strahler bzw. Detektor vorteilhaft an einer beliebigen Stelle entfernt von der Lichtleitanordnung angeordnet sein, beispielsweise am Gürtel des Trägers einer erfindungsgemäßen Vorrichtung in Form einer Brille, so daß das Gewicht von Projektor bzw. Photodetektor nicht auf die Brille wirken.

*zu 4.1 Erfaßtes Bild*

Erfindungsgemäß können bei der Bestimmung stets Punkte längs einer Trajektorie sequentiell, i.e. nacheinander abgetastet werden. Als Ergebnis erhält man eine Folge von Bildpunkten, denen sich beispielsweise anhand der Stellung der Detektor-Lichtleitanordnung bestimmte Koordinaten (beispielsweise Abszisse x und Ordinate y; Radius R und Polarwinkel $\phi$) und Werte für die physikalische Größe zuordnen lassen. Zusätzlich kann jedem Punkt ein Abstand zum Detektor, beispielsweise aufgrund einer gemessenen Laufzeit und/oder Fokussierung und/oder Intereferenz zugeordnet sein, so daß man als "Bild" eine Relation "physikalischer Wert(x,y)" bzw. "physikalischer Wert(x,y,z)" erhält.

Zum besseren Verständnis kann beispielsweise bei der Anordnung in Fig. 1 jeder Stellung des horizontal beweglichen Flachspiegels 109H ein x-Wert und jeder Stellung des vertikal beweglichen Flachspiegels 109V ein y-Wert zugeordnet sein. Als physikalische Größe kann beispielsweise die Strahlstärke $I_e$ bzw. Helligkeit eines bei dieser Stellung der Detektor-Lichtleitanordnung vom Detektor 110 erfaßten Lichtstrahls, der an einem Punkt der Netzhaut 105 reflektiert wurde, quantitativ bestimmt werden. Werden nacheinander für verschiedene Stellungen der beiden Flachspiegel die Helligkeit eines einfallendes Lichtstrahls ermittelt und gespeichert, so erhält man eine Menge von Wertetrippeln (x, y, $I_e$), die in einem x-y-Koordinatensystem eine Kurve ergeben. Durch entsprechende Analog/Digital-Wandlung können dabei den einzelnen Punkten der Kurve spezifische Helligkeits- bzw. Graustufenwerten zugeordnet sein. Zusätzlich kann, beispielsweise mittels eines frequenzmodulierten Lichtstrahls, die Laufzeit und damit die Entfernung z des reflektierenden Punktes zum Detektor bestimmt sein, man erhält dann analog eine Menge von Wertetupeln (x, y, z, $I_e$), die in einem x-y-z-Koordinatensystem eine Bildpunktkurve ergeben.

Bei feinerer Abtastung, d.h. beispielsweise bei kleineren Schritten zwischen den einzelnen Stellungen (x,y) der Flachspiegel ergibt die Kurve ein immer schärferes zweidimensionales bzw. dreidimensionales "Bild" des Teils des Auges, an dem die Lichtstrahlen reflektiert wurden.

*zu 4.2. Detektion*

*4.2.1 Grobbestimmung des Pupillenmittelpunktes*

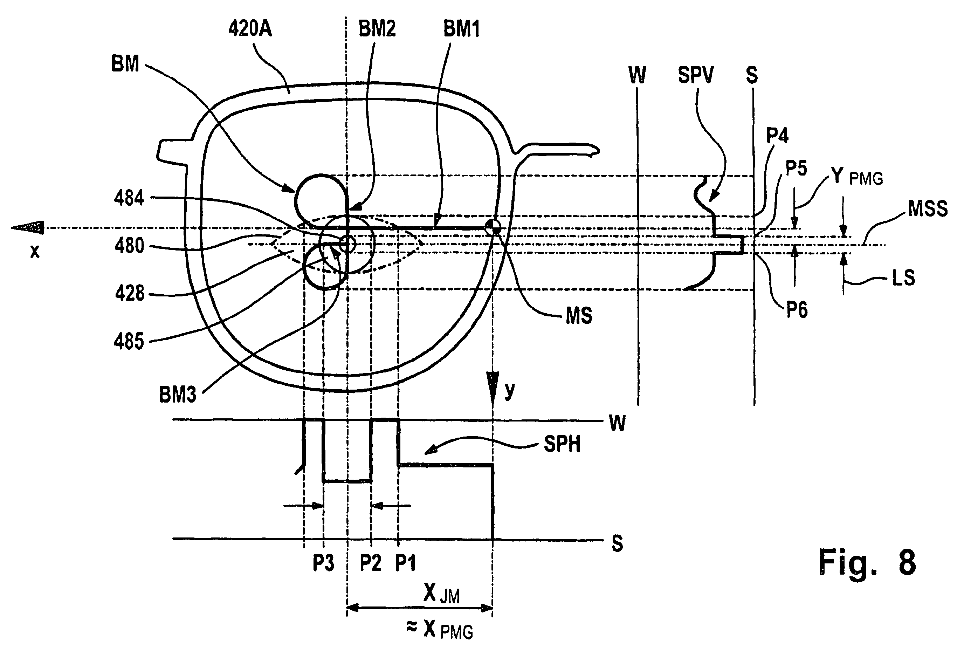

Ein charakteristisches Merkmal des Auges ist der Pupillenmittelpunkt, der beispielsweise zusammen mit der Fovea centralis eine Blickrichtung des Auges festlegt. Anhand von Fig. 8 eine Grobbestimmung des Pupillenmittelpunktes bezüglich der Vorrichtung 420A gemäß der vorliegenden Erfindung erläutert. Fig. 8 zeigt in der Draufsicht das Bild eines Auges 480 mit der Lederhaut 428, der Iris 485 und der Pupille 484 hinter einem Brillengestell 420A.

Für das Brillengestell 420A wird ein ebenes Koordinatensystem (MS, x, y) festgelegt, bezüglich dem der Pupillenmittelpunkt ($X_{PMG}$, $y_{PMG}$) bestimmt wird. Dabei wird als Ursprung eine Markierung MS auf dem Brillengestell gewählt, durch die die horizontale x-Achse und die dazu senkrechte y-Achse gehen. Die x-Achse liegt dabei vorteilhafterweise in etwa in Höhe der Pupille.

Zunächst wird durch entsprechende Bewegung der Detektorspiegel (s. den Abschnitt "optische Vorrichtungen") eine Folge von Punkten längs einer ersten Geraden BM1 auf der x-Achse aktiv oder passiv abgetastet. Die Helligkeit SPH der Bildpunkte längs dieser Geraden ändert sich dabei beim Übergang des Abtaststrahls vom Gesicht zur Lederhaut an der Stelle P1 auf einen höheren Wert W ("weiß"), beim Übergang von der Lederhaut zur Iris an der Stelle P2 auf einen niedrigeren Wert und beim erneuten Übergang von Iris zur Lederhaut an der Stelle P3 wieder auf den Wert W. Das geometrische Mittel der x-Koordinaten der Punkte P2 und P3 wird als vorläufiger x-Koordinate $x_{JM}$ des Pupillenmittelpunkt festgelegt.

Anschließend wird eine Folge von Punkten längs einer zweiten Geraden BM2, die parallel zur y-Achse durch den Punkt ($x_{JM}$, 0) und damit stets durch die Pupille verläuft, aktiv oder passiv abgetastet. Vorzugsweise geht der Abtatsstrahl dabei längs eines Dreiviertelkreises von der Geraden BM1 auf die Gerade BM2 über, um die Schwingungsanregung der Vorrichtung gering zu halten. Die Helligkeit SPV der Bildpunkte längs der zweiten Geraden BM2 ändert sich beim Übergang von der Iris zur näherungsweise schwarzen Pupille an der Stelle P5 näherungsweise auf einen niedrigeren, für eine individuelle Pupille charakteristischen Wert S und beim erneuten Übergang von der Pupille zur Iris an der Stelle P6 wieder auf einen höheren Wert. Als y-Koordinate $y_{PMG}$ des Pupillenmittelpunktes wird das geometrische Mittel zwischen den y-Koordinaten der Punkte P5 und P6, zwischen denen der Abtaststrahl näherungsweise einen, beispielsweise empirisch ermittelten, charakteristischen Helligkeitswert S liefert, bestimmt.

Abschließend wird eine Folge von Punkten längs einer dritten Geraden BM3 aktiv oder passiv abgetastet, die parallel zur x-Achse durch den Punkt (0, $y_{PMG}$) und damit stets durch die Pupille verläuft. Vorzugsweise geht der Abtaststrahl dabei längs eines Dreiviertelkreises von der Geraden BM2 auf die Gerade BM3 über, um die Schwingungsanregung der Vorrichtung gering zu halten. Die Helligkeit der Bildpunkte längs der dritten Geraden BM3 ändert sich analog zur zweiten Geraden beim Übergang von der Iris zur näherungsweise schwarzen Pupille näherungsweise auf einen niedrigeren Wert S und beim erneuten Übergang von der Pupille zur Iris wieder auf einen höheren Wert. Als x-Koordinate $x_{PMG}$ des Pupillenmittelpunktes wird das geometrische Mittel zwischen den x-Koordinaten der Punkte bestimmt, zwischen denen der Abtaststrahl näherungsweise den Helligkeitswert S liefert.

Eine geeignet ausgelegte Auswertevorrichtung bestimmt anhand oben dargestellten Verfahrens zuverlässig die Lage ($x_{PMG,}$ $y_{PMG}$) des Pupillenmittelpunkts bezüglich des Brillengestells 420A und speichert diese Daten ab, so daß beispielsweise die im folgenden beschriebenen Verfahren ausgehend vom gefundenen Pupillenmittelpunkt verwendet werden können, um die Abstastvorrichtung entsprechend nachjustiert wird.

Falls bei der Abtastung längs einer der drei Geraden BM1, BM2 oder BM3 nicht die erwarteten Helligkeitsverläufe registriert werden, da beispielsweise das Auge durch das Lied verschlossen ist, erkennt die Auswertevorrichtung, daß die Bestimmung gescheitert ist und wiederholt wenigstens die gescheiterten Abtastschritte solange, bis eine fehlerfreie Bestimmung des Pupillenmittelpunkts erfolgreich durchgeführt wurde und/oder meldet das Scheitern der Bestimmung, aus dem beispielsweise geschlossen werden kann, daß gerade ein Lidschlag erfolgt. Damit kann also gleichzeitig sichergestellt sein, daß das Auge zum Zeitpunkt der Bestimmung der Pupillenmitte geöffnet ist und beispielsweise Informationen auch in das Auge hineinprojiziert oder die Netzhaut abgetastet werden können.

Zusätzlich kann beispielsweise die Entfernung des Punktes P2 zum Detektor ermittelt und damit näherungsweise die dreidimensionale Lage des Pupillenmittelpunktes relativ zur erfindungsgemäßen Vorrichtung bzw. zum Detektor bestimmt werden.

*4.2.2 Feinbestimmung des Pupillenmittelpunktes*

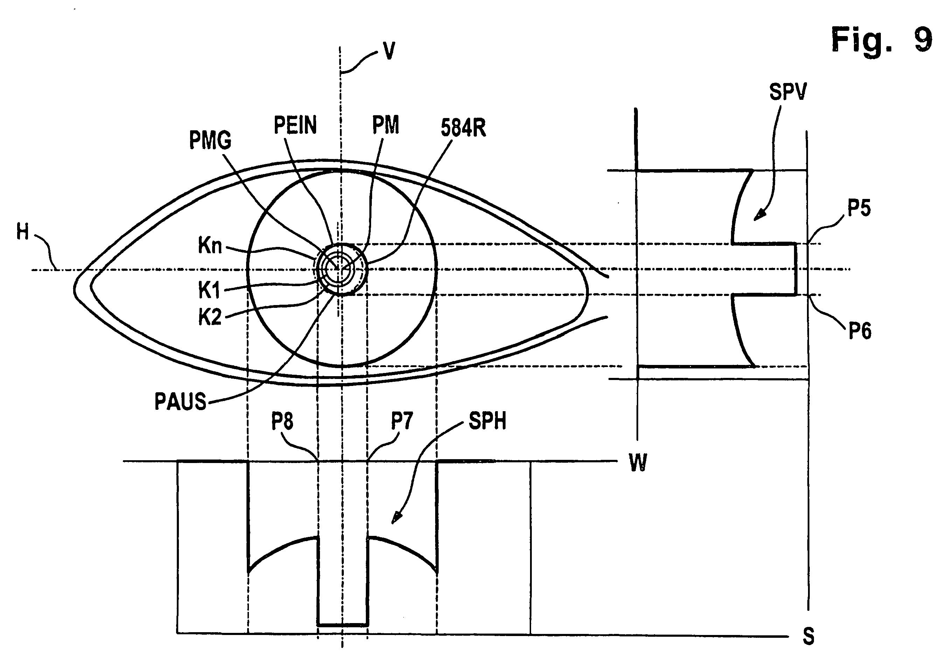

Es kann eine Feinbestimmung des Pupillenmittelpunktes durchgeführt werden, die nachfolgend anhand von Fig. 9 schematisch erläutert wird. Vorteilhafterweise kann diese Feinbestimmung durchgeführt werden, nachdem anhand der oben dargestellten Grobbestimmung die Lage des Pupillenmittelpunktes ungefähr bestimmt worden ist.

Fig. 9 zeigt die Draufsicht auf eine Auge mit einer horizontalen Linie H und einer dazu senkrechten vertikalen Linie V, die beide durch den (exakten) Pupillenmittelpunkt PM gehen. Bei aktiver oder passiver Abtastung längs dieser Linien ergeben sich näherungsweise die unten bzw. rechts dargestellten Helligkeitsverläufe SPH bezüglich der horizontalen Linie H bzw. SPV bezüglich der vertikalen Linie V. Dabei verringert sich der Helligkeitswert bei Übergang von der weißen Lederhaut auf die farbige Iris von einem für die Lederhaut charakteristischen Wert W und sinkt beim Übergang von der Iris zur schwarzen Pupille an den Stellen P7, P8 bzw. P5, P6 näherungsweise auf einen für die Pupille charakteristischen Wert S.

Wird nun die Pupille nacheinander längs konzentrischer Kreisen K1, K2,... Kn um den zuvor grob bestimmten Pupillenmittelpunkt PMG abgetastet, so ergibt die Abtastung längs der Kreise, die vollständig innerhalb der Pupille liegen, stets näherungsweise einen konstanten Helligkeitswert S über dem gesamten Umfang des Kreises, die Abtastung längs der Kreise, die vollständig außerhalb der Pupille liegen, stets näherungsweise einen konstanten Helligkeitswert W über dem gesamten Umfang des Kreises.

Der Radius r1 eines ersten Kreises K1 um den grob bestimmten Pupillenmittelpunkt PMG wird so gewählt, daß der Kreis K1 vollständig innerhalb der Pupille liegt. Dazu wird beispielsweise mit einem Startwert für den Radius rl begonnen und die Helligkeitswerte längs des zugehörigen Startkreises mit dem Referenzwert S verglichen. Anschließend wird der Radius solange verkleinert wird, bis die Helligkeitswerte an keiner Stelle signifikant von S abweichen. Alternativ kann auch ein stets ausreichender konstanter Höchstradius vorgegeben werden. Dann werden solange konzentrische Kreise K2,...Kn um den grob bestimmten Pupillenmittelpunkt abgetastet, wobei der Radius ri jedes Kreises Ki größer als der Radius r(i-1) des vorhergehenden Kreises K(i-1) ist, bis die Helligkeitswerte längs eines Kreises Kn zwischen Punkten PAUS und PEIN auf dem Umfang des Kreises Kn signifikant größer als der Referenwert S sind. In diesem Fall wird der grob bestimmte Pupillenmittelpunkt PMG längs der Mittelsenkrechten auf die Sekante durch die Punkte PAUS und PEIN verschoben, beispielsweise um die Differenz der Radien rn und r(n-1). Falls die Punkte PAUS und PEIN (näherungsweise) identisch sind, liegt die Pupille vollständig innerhalb des Kreises Kn. Dann wird der Radius rn solange verringert, bis zumindest auf einem Teilbereich des Umfangs des Kreises Kn die Helligkeitswerte nicht mehr signifikant vom Referenzwert S abweichen, und das Verfahren wird fortgesetzt.

Das oben beschriebene Verfahren kann mehrmals wiederholt werden, wobei die Differenz der Radien aufeinanderfolgender Kreise gegenüber dem vorhergehenden Verfahrensdurchlauf jeweils geeignet reduziert und als erster Kreis K1 ein Kreis mit dem Radius des Kreises K(n-1) gewählt wird, der noch vollständig innerhalb der Pupille lag, um so den Pupillenmittelpunkt PMG iterativ in den exakten Pupillenmittelpunkt PM zu verschieben. Unterschreitet dabei die Radiendifferenz einen vorbestimmten Wert, bricht die erfindungsgemäße Vorrichtung das Verfahren ab und betrachtet den gefundenen Pupillenmittelpunkt PMG als exakt genug bestimmt.

Bei dem oben angegeben Verfahren können statt Kreisen Ellipsen abgetastet werden, um die Verzerrung der runden Pupille bei einer Verdrehung des Auges zu berücksichtigen. Analog zu obigen Verfahren kann auch mit einem ersten Kreis begonnen werden, der vollständig außerhalb der Pupille liegt und die Radien der nachfolgenden Kreise stetig verkleinert werden.

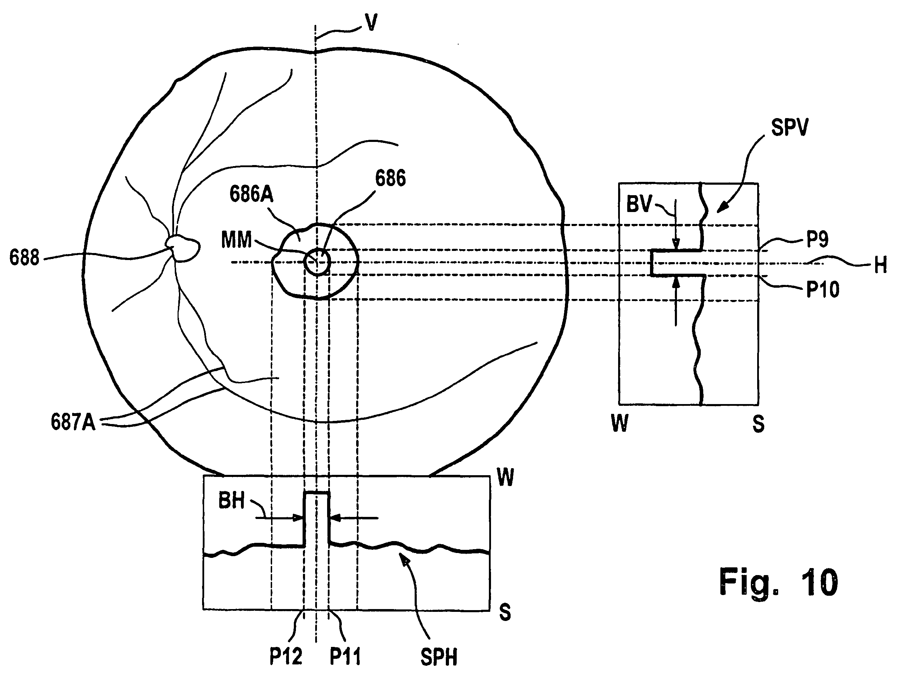

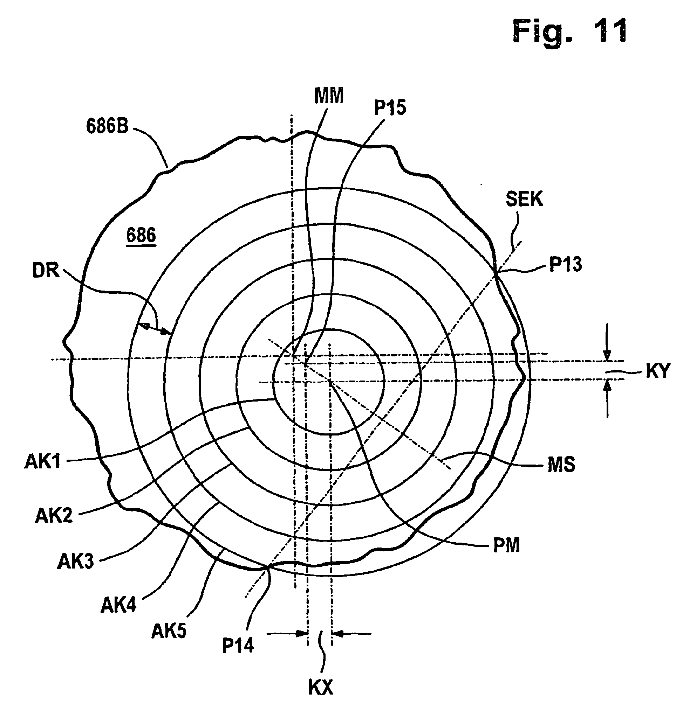

In analoger Weise zu den obigen Verfahren kann auch die Makula auf der Netzhaut bestimmt werden. Dies wird im folgenden anhand der Fig. 10 bzw. 11 beschrieben. Dabei kann als Ausgangspunkt für die Makulamitte zunächst ein nach einem der obigen Verfahren bestimmter Pupillenmittelpunkt angenommen werden.

*4.2.3 Grobbestimmung des Makulamittelpunktes*

Fig. 10 zeigt ein Bild der Netzhaut mit der Makula 686A, der darin gelegenen Fovea centralis 686 mit dem Makulamittelpunkt MM, dem Blinden Fleck 688 sowie mehreren Blutgefäßen 687A. Vorgenannte Merkmale unterscheiden sich voneinander und vom Rest der Netzhaut durch die Helligkeitswerte, die an ihnen reflektierte Lichtstrahlen aufweisen. So reflektiert beispielsweise die Fovea centralis 686 deutlich stärker als die restliche Netzhaut, die Helligkeitswerte von an der Fovea centralis reflektierten Lichtstrahlen liegt signifikant näher am Helligkeitswert W einer weißen Reflektionsfläche als die von Lichtstrahlen, die in ihrer Umgebung reflektiert werden und damit einen näher bei dem Helligkeitswert S einer schwarzen Reflektionsfläche liegenden Helligkeitswert aufweisen.

In einer Ausführung der vorliegenden Erfindung wird die Retina längs einer horizontalen Linie H und einer vertikalen Linie V abgetastet, die durch einen vorläufigen Makulamittelpunkt gehen, welcher beispielsweise aus dem zuvor ermittelten Pupillenmittelpunkt gewonnen wird, indem beispielsweise der Pupillenmittelpunkt selbst als vorläufiger Makulamittelpunkt festgelegt wird oder der vorläufige Makulamittelpunkt in einer vorbestimmten Position zum Pupillenmittelpunkt festgelegt wird, die beispielsweise empirisch aus vorangegangenen Untersuchungen der Relativpositionen der Pupillen- und Makulamittelpunkten bestimmt sein kann. Dann kann anhand der Lage der Punkte P9, P10 bzw. P11, P12, bei denen bei vertikaler bzw. horizontaler Abtastung im Helligkeitsverlauf SPV bzw. SPH ein signifikanter Sprung auftritt, und/oder anhand des Abstandes BV bzw. BH zwischen diesen Punkten analog zu den oben beschriebenen Verfahren zur Bestimmung des Pupillenmittelpunktes der Mittelpunkt der Fovea centralis 686 bzw. der Makulamittelpunkt 686A bestimmt werden.

Zusätzlich kann beispielsweise die Entfernung der Punktes P9, P10, P11 bzw. P12 zum Detektor ermittelt und damit näherungsweise die dreidimensionale Lage des Makulamittelpunktes relativ zur erfindungsgemäßen Vorrichtung bzw. zum Detektor und/oder relativ zum Pupillenmittelpunkt bestimmt werden. Aus der dreidimensionalen Lage von Pupillen- und Makulamittelpunkt kann dann beispielsweise die dreidimensionale Lage der Blickrichtung, beispielsweise relativ zum Detektor, ermittelt werden.

*4.2.4 Feinbestimmung des Makulamittelpunktes*

Anhand von Fig. 11 wird ein Verfahren zur Feinbestimmung der Makulamitte analog zum oben angegebene Verfahren zur Feinbestimmung der Pupillenmitte näher beschrieben. Ausgehend vom zuvor gefundenen Pupillenmittelpunkt PM, der als Ausgangspunkt dient, wird die Netzhaut im Bereich der Makula 686 in konzentrischen Kreisen AK1, AK2,...Akn abgetastet, wobei der Radius eines Kreises Aki stets größer als der Radius des vorhergehenden Kreises Ak (i-1) ist. Sobald die längs eines Kreises (in Fig. 6A Kreis AK5) erfaßten Helligkeitswerte in einem Bereich zwischen einem Punkt P13 und einem Punkt P14 auf dem Umfang des Kreises einen deutlich anderen Wert aufweisen, wird die vorläufige Makulamitte PM längs der Mittelsenkrechten auf die durch P13, P14 bestimmten Sekante in die neue vorläufige Makulamitte P15 verschoben. Das Verfahren wird mit einer reduzierten Differenz zwischen den Radien aufeinanderfolgender Kreise wiederholt, beginnend mit dem Kreis, der noch vollständig innerhalb der Makula lag, d.h., bei dem die Helligkeitswerte längs des gesamten Umfangs des Kreises näherungsweise einen für die Makula charakteristischen, beispielsweise emprisch ermittelten, Wert und keine signifikanten Sprünge aufweisen. Das Verfahren wird solange wiederholt, bis die Differenz zwischen den Radien aufeinanderfolgender Kreise einen vorbestimmten Grenzwert unterschreitet oder ein Kreis näherungsweise längs der Grenze der Makula verläuft, was dadurch festgestellt wird, daß die Helligkeitswerte längs dieses Kreises eine Vielzahl von Signalsprüngen zwischen dem für die Makula charakteristischen Wert und einem deutlich darüberliegenden Wert aufweisen. Der dabei zuletzt gefundene Makulamittelpunkt wird abgespeichert.

*zu 4.2.5 Mehrstufige und analoge Verfahren*

Obenstehend wurde beschrieben, wie anhand der reflektierten Lichtstrahlen der Pupillen- und/oder der Makulamittelpunkt grob bzw. genau bestimmt werden kann. Gleichermaßen können auch andere signifikante Merkmale des Auges, beispielsweise Blutgefäße der Netzhaut, Narben auf der Kornea oder dergleichen bestimmt werden. Hierzu kann beispielsweise ein abgetastetes Reflexbild mittels einer Bild- bzw. Mustererkennung analysiert und signifikante Muster (beispielsweise Blutgefäße, Narben oder dergleichen) lokalisiert werden.

*zu 4.3.2 Bestimmung der Orientierung des Netzhautreflexbildes (Retina-Karte)*

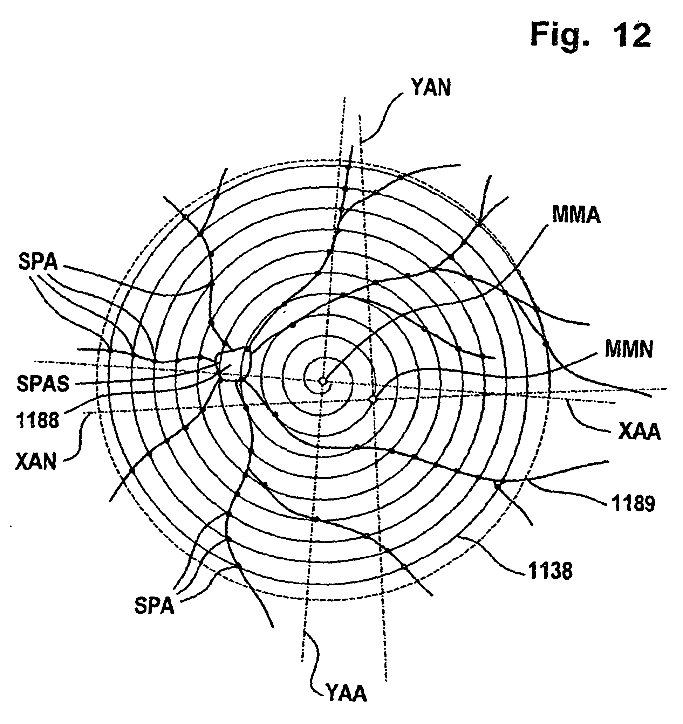

Fig. 12 zeigt eine zweidimensionale Abbildung der Netzhaut eines Auges mit blindem Fleck 1188, dem Makulamittelpunkt MMN und diversen Blutgefäßen. Die Netzhaut wird anhand eines geeigneten Bewegungsmusters abgetastet, beispielsweise wie in Fig. 12 dargestellt längs einer Spirale 1138.. Dabei kann das Abtastmuster, wie oben beschrieben, an dem alten Makulamittelpunkt MMA als Referenzpunkt orientiert sein, der in einem früheren Schritt ermittelt und gespeichert wurde. Während im Beispiel zur Erläuterung Spiralen verwendet sind, kann gleichermaßen auch entlang konzentrischer Kreise, entlang eines Rechteckrasters oder dergleichen abgetastet werden.

Der Makulamittelpunkt als Referenzpunkt kann anhand eines der oben beschriebenen Verfahren oder anhand des vorliegenden Verfahrens, das zu einem früheren Zeitpunkt durchgeführt wurde, bestimmt sein. Beginnend am alten Makulamittelpunkt MMA erfaßt der Detektor der erfindungsgemäßen Vorrichtung die Helligkeitswerte der Lichtstrahlen, die längs der Spirale reflektiert werden. Dabei weisen Punkte SPA der Kurve, die auf einem Blutgefäß liegen, andere Helligkeitswerte auf als Punkte auf dem Rest der Netzhaut, gleiches gilt für Punkte SPAS, die auf dem blinden Fleck 1188 liegen oder für Punkte MMN, die auf der Makula liegen. Die so gewonnenen Helligkeitswerte werden als Retina-Karte abgespeichert.

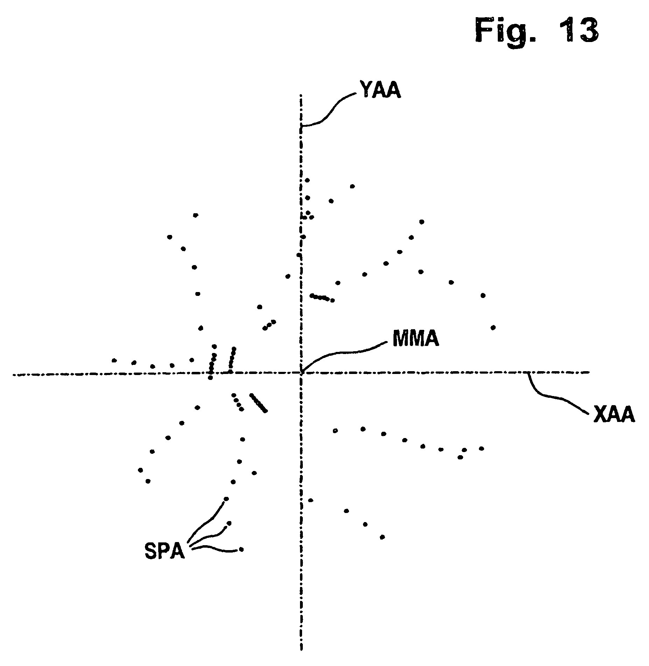

Fig. 13 zeigt eine so ermittelte Retina-Karte, bei der die Helligkeitswerte beispielsweise in binärer Form gespeichert sind, d.h. jeder Punkt, dessen Helligkeitswert einen bestimmten charakteristischen Wert übersteigt, wird mit 1, ansonsten mit 0 belegt. Eine Retina-Karte kann zweckmäßigerweise in Form einer Matrix abgespeichert sein.

Durch Vergleich der so ermittelten neuen Retina-Karte mit einer zu einem früheren Zeitpunkt ermittelten alten Retina-Karte kann eine entsprechende Software die Verschiebung und Verdrehung der neuen gegenüber der alten Retina-Karte und damit die Orientierungsänderung des Auges ermitteln. Dazu wird vorzugsweise zunächst anhand eines der oben näher beschriebenen Verfahrens die neue, i.e. aktuelle Makulamitte MMN bestimmt, deren Lage relativ zur alten Makulamitte MMA die Verschiebung der neuen Retina-Karte gegenüber der alten ergibt, d.h. der zugehörige Verschiebevektor W ist der Vektor von MMA zu MMN. Anschließend werden zwei vorzugsweise orthogonale Achsen XAN bzw. YAN in der neuen Retina-Karte bestimmt, die sich in der neuen Makulamitte MMN schneiden und anhand bestimmter charakteristischer Merkmale der abgetasteten Punkte der Retina orientiert sind. Beispielsweise kann die Achse XAN durch Punkte gehen, die aufgrund ihrer Anordnung oder ihrer Helligkeitswerte als Teil des blinden Flecks erkannt werden. Anschließend wird die alte Retina-Karte um den Vektor W verschoben, so daß die alte Makulamitte MMA auf der neuen Makulamitte MMN liegt und anschließend um diesen Punkt MMA=MMN so verdreht, daß die alten Achsen XAA bzw. YAA auf den neuen Achsen XAN bzw. YAN liegen. Der hierbei auftretende Drehwinkel beschreibt zusammen mit dem Verschiebevektor W die Lage- und Orientierungsänderung des Auges relativ zur Vorrichtung bezüglich der Lage bei Aufnahme der alten Retina-Karte.

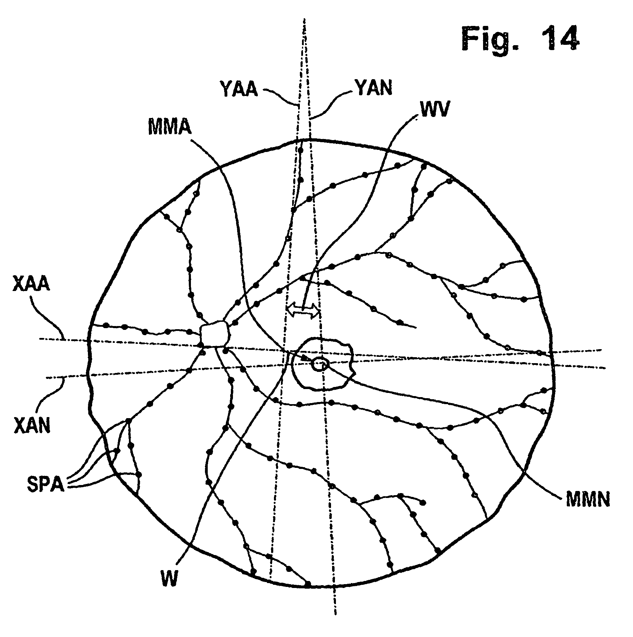

Gleichermaßen kann, wie in Fig. 14 dargestellt, eine entsprechende Mustererkennungs- und verarbeitungssoftware auch direkt ein aktuelles, d.h., neu abgetastetes Nethzhautrefelxbild bzw. eine aktuelle Retina-Karte mit einer gespeicherten alten Retina-Karte oder einem Netzhautreflexbild zur Deckung bringen, die hierbei durchgeführte Drehung und/oder Verschiebung beschreibt wiederum die Lage- und Orientierungsänderung des Auges gegenüber der Lage, die der alten Retina-Karte bzw. dem Netzhautreflexbild entspricht. Dabei sei darauf hingewiesen, daß ein aktuelles Netzhautreflexbild vorteilhafterweise mit deutlich weniger Bildpunkten abgetastet bzw. gespeichert sein kann. Diese reichen in der Regel trotzdem bereits dazu aus, das aktuelle Bild und das Referenzbild zur Deckung zu bringen, wobei vorteilhafterweise weniger Punkte abgetastet bzw. verarbeitet werden müssen.

Zusätzlich kann beispielsweise die Entfernung eines Punktes auf der Netzhaut zum Detektor ermittelt und damit näherungsweise die dreidimensionale Lage der Netzhaut und damit des Auges relativ zur erfindungsgemäßen Vorrichtung bzw. zum Detektor bestimmt werden.

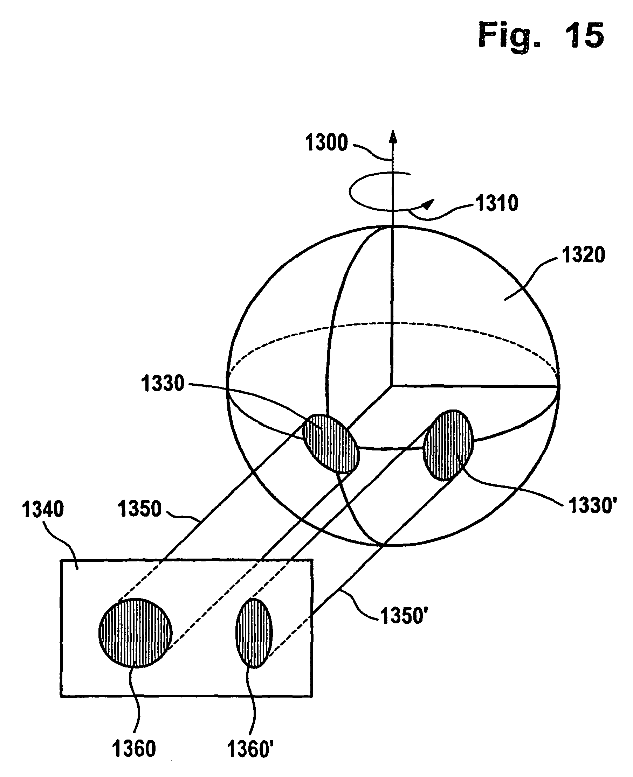

Gleichermaßen kann auch, wie oben dargestellt, die Verzerrung des Reflexbildes, beispielsweise des Reflexbildes der Makula oder der Netzhaut zur Bestimmung der Orientierung des Auges verwendet werden, wie dies anhand eines stark vereinfachten Modells in Fig. 15 gezeigt ist. Fig. 15 zeigt in perspektivischer Ansicht ein Auge 1320, das um den Winkel 1310 um die Achse 1300 verdreht wird.

Im unverdrehten Zustand ermittelt eine erfindungsgemäße Vorrichtung anhand an der Iris reflektierter Lichtstrahlen 1350 ein Bild 1360 der Pupille. Im verdrehten Zustand liefern die an der Iris reflektierten Lichtstrahlen 1350' im Detektor 1340 das verschobene und verzerrte Bild 1360' der Pupille 1330' des verdrehten Auges. Aus der Verschiebung und Verzerrung des Bildes 1360' gegenüber dem Bild 1360 läßt sich entsprechend die Verdrehung 1310 des Auges 1320 ermitteln. Bei Kenntnis der tatsächlichen physikalischen Form, beispielsweise der Kreisform der Pupille, läßt sich darüberhinaus aus dem verzerrten Bild auch die Orientierung relativ zum Detektor bestimmen. Die tatsächliche physikalische Form kann beispielsweise empirisch ermittelt sein.

Zusätzlich kann beispielsweise die Entfernung eines Punktes auf der Iris zum Detektor ermittelt und damit näherungsweise die dreidimensionale Lage der Iris bzw. der Pupille und damit des Auges relativ zur erfindungsgemäßen Vorrichtung bzw. zum Detektor bestimmt werden.

*zu 4.3.3 Orientierung bezüglich der Umgebung und/oder der Vorrichtung*

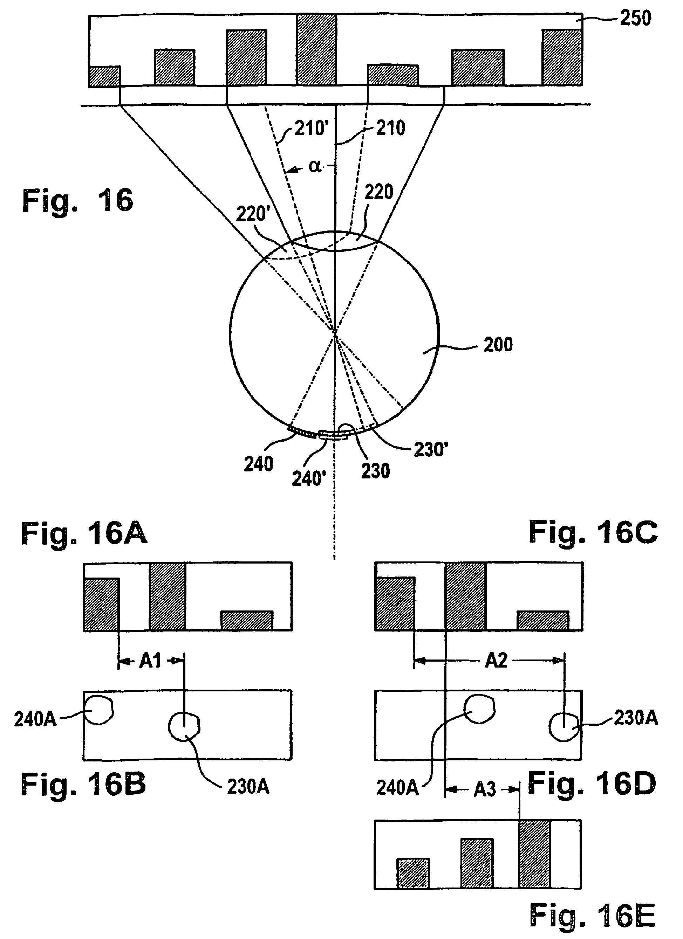

Im folgenden werden anhand der Fig. 16, 16A-16E verschiedene erfindungsgemäße Möglichkeiten zur Bestimmung der Orientierung des Auges relativ zur Umgebung bzw. der Vorrichtung erläutert. Dabei beschränkt sich die Darstellung und Beschreibung zur besseren Übersichtlichkeit auf den vereinfachten ebenen Fall, gleichermaßen können die Verfahren analog auch im allgemeinen räumlichen Fall angewendet sein. Zusätzlich kann stets die Entfernung eines oder mehrere abgetasteter Punkte des Auges beispielsweise relativ zum Detektor bestimmt werden, indem beispielsweise ein frequenzmoduliertes Lichtsignal auf diesen Punkt eingestrahlt und der reflektierte Lichtstrahl erfaßt wird. Aus der Frequenz des erfaßten Lichtstrahls kann der Zeitpunkt semer Emittierung und damit die seine Laufzeit ermittelt werden, woraus sich wiederum eine Entfernung zum Detektor bzw. zum Projektionssystem bestimmen läßt. Damit läßt sich auch die dreidimensionale Lage des Auges relativ zur erfindungsgemäßen Vorrichtung bestimmen.

Fig. 16 zeigt schematisch ein Auge 200 in einer Referenzstellung und in einer gegenüber dieser bezüglich der Umgebung um den Winkel $\alpha$ verdrehten Orientierung (gestrichelt dargestellt und mit ' bezeichnet), gekennzeichnet jeweils durch die Sehachse 210 bzw. 210', die durch die Mittelpunkte der Pupille 220 bzw. 220' und der Makula 230 bzw. 230' geht. Zusätzlich ist der Blinde Fleck 240 bzw. 240' auf der Retina des Auges 200 gekennzeichnet. Die Umgebung 250 ist hier durch ein inkrementales Schwarz-Weiß-Muster angedeutet. Sie kann in einer Ausführung der vorliegenden Erfindung von einer Kamera (nicht dargestellt) aufgenommen werden, die starr mit der erfindungsgemäßen Vorrichtung verbunden und in einer bevorzugten Ausführung näherungsweise konfokal zum Auge 200 angeordnet ist.

In der Referenzstellung kann eine erfindungsgemäße Vorrichtung beispielsweise das in Fig. 16A gezeigte Reflexbild der Umgebung 250 abtasten (Umgebungsreflexbild, passive Abtastung). Gleichermaßen kann eine erfindungsgemäße Vorrichtung zusätzlich oder alternativ einen Teil der Retina des Auges 200 aktiv abtasten, das entsprechende Bild zeigt schematisch Fig. 16B mit der Makula 230A und dem blinden Fleck 240A (Netzhautreflexbild, aktive Abtastung). Ist dabei das zur aktiven Abtastung eingestrahlte Licht geeignet moduliert, können Umgebungsreflexbild und Netzhautreflexbild voneinander getrennt erstellt werden. Beispielsweise wird zur aktiven Abtastung während einer bestimmten Zeitperiode T1 Infrarotlicht zusätzlich eingestrahlt, während einer darauffolgenden Zeitperiode T2 wird kein Licht aktiv eingestrahlt. Subtraktion der während der Periode T2 aufgenommenen Bilds der Netzhaut von dem der während der Periode T1 aufgenommenen Bilds der Netzhaut liefert näherungsweise das Netzhautreflexbild, während das während der Periode T2 aufgenommenen Bild das Umgebungsreflexbild darstellt.

Während das Umgebungsreflexbild Strukturen (Kanten etc.) zeigt, die relativ zur Umgebung fest sind (Umgebungsbild), sind die Strukturen im Netzhautreflexbild fest bezüglich des Auges (Augenbild).

Durch eine geeignete Bilderkennnungssoftware können die beide Bilder in Fig. 16A und Fig. 16B miteinander verglichen und beispielsweise der Abstand A1 zwischen einer signifikaten Kante im Reflexbild der Umgebung und dem Makulamittelpunkt bestimmt werden. Analog können auch ein von der Kamera aufgenommene Bild der Umgebung (Umgebungsbild) und das Bild der Netzhaut miteinander verglichen und ebenfalls ein Abstand zwischen einer signifikanten Kante und der Makulamitte bestimmt werden.

Orientierung des Auges gegenüber der Umgebung

Anhand der relativen Lage signifikanter Merkmale im Umgebungsbild 16A und dem Augenbild 16B, angedeutet beispielsweise durch den Abstand A1 zwischen einer signifikanten Kante und der Makulamitte, kann eine Orientierung des Auges bezüglich der Umgebung bestimmt werden. Beispielsweise kann bestimmt werden, daß die Blickrichtung des Auges auf die rechte Kante des höchsten schwarzen Balkens in der Umgebung zeigt.

Orientierungsänderung des Auges gegenüber der Umgebung

Ändert sich die Orientierung des Auges 200 gegenüber der Vorrichtung so, daß die Sehachse 210 um den Winkel $\alpha$ in die neue Sehachse 210' übergeht und bleibt dabei die Vorrichtung relativ zur Umgebung dabei konstant, zeigt eine Abtastung des Umgebungsreflexbildes das in Fig. 16C dargestellt Bild, daß im gezeigten Beispiel bis auf die Verzerrung aufgrund der zweidimensionalen Abbildung der dreidimensionalen Struktur identisch mit dem von Fig. 16A ist. Eine Abtastung der Netzhaut liefert das in Fig. 16D gezeigte Netzhautreflexbild mit der Makula 230A und dem Blinden Fleck 240A. Ein Vergleich dieses Bildes der Netzhaut mit dem Reflexbild der Umgebung (Fig. 16C) bzw. dem von der Kamera aufgenommenen Bild der Umgebung (Fig. 16) liefert wiederum für den Abstand zwischen einer signifikaten Kante im Bild der Umgebung und dem Makulamittelpunkt einen Wert A2. Aus der Änderung dieses Abstandes A1 zu A2 kann die Verdrehung $\alpha$ des Auges gegenüber der Umgebung bestimmt werden.

Orientierungsänderung der Vorrichtung gegenüber der Umgebung

Ändert sich die Orientierung des Auges gegenüber der Vorrichtung nicht, jedoch die Orientierung der Vorrichtung gegenüber der Umgebung so, daß wiederum die Sehachse 210 um den Winkel $\alpha$ in die Sehachsen 201' gedreht ist, so liefert eine Erfassung des an der Netzhaut reflektierten Bildes der Umgebung und/oder eine von der Kamera aufgenommenes Bild der Umgebung das in Fig. 16E gezeigte Umgebungsreflexbild. Aus einem Vergleich signifikanter Kanten, einer Bildkorrelation oder dergleichen zwischen diesem Bild und dem zuerst aufgenommenen Umgebungsreflexbild aus Fig. 16A kann dann analog eine Verdrehung der Vorrichtung gegenüber der Umgebung bestimmt werden.

Eine Abtastung der Netzhaut liefert wiederum das in Fig. 16B gezeigte Netzhautreflexbild mit der Makula 230A und dem Blinden Fleck 240A, woraus geschlossen werden kann, daß sich die Stellung des Auges relativ zur Vorrichtung nicht geändert hat.

Die Änderung der Orientierung des Auges gegenüber der Umgebung setzt sich, wie oben beschrieben, aus der Orientierungsänderung des Auges gegenüber der Vorrichtung und der Orientierungsänderung der Vorrichtung gegenüber der Umgebung zusammen. Deren Bestimmung wurde jeweils einzeln dargestellt, bei einer gleichzeitigen Änderung der Orientierung von Vorrichtung und Auge überlagern sich beide Effekte und können durch einen entsprechende Verknüpfung beider Verfahren ermittelt werden.

Insgesamt sind in oben beschriebenem Beispiel verschiedene Möglichkeiten zur Bestimmung der Orientierung des Auges vorhanden, die im folgenden aufgelistet werden. Dabei können bei einer erfindungsgemäßen Vorrichtung bzw. einem erfindungsgemäßen Verfahren mehrere dieser Möglichkeiten kombiniert sein, um mehrere relative Orientierungen gleichzeitig zu ermitteln oder Ergebnisse zu überprüfen und gegebenenfalls zu korrigieren. Wie eingangs erwähnt, sind die Varianten anhand einer vereinfachten ebenen Darstellung erläutert, im allgemeinen räumlichen Fall sind entsprechende Verfahren analog anzuwenden. Zusätzlich kann die dreidimensionale Lage des Auges beispielsweise anhand einer Entfernungsmessung zwischen einem Teil des Auges und dem Detektor bestimmt werden.

Kamerabild - Netzhautbild

Vergleicht man das Bild, das von einer fest mit der Vorrichtung verbundenen Kamera von der Umgebung aufgenommen wird (Fig. 16), mit dem Bild der Netzhaut, das sich beispielsweise aus einer aktiven Abtastung ergibt (Fig. 16B, 16 D), so können signifikante Merkmale im Umgebungsbild (Kanten etc.) und im Netzhautbild (Makula etc.) einander zugeordnet werden. Anhand dieser relativen Lage (bsp. Abstand A1) kann eine Orientierung des Auges relativ zur Umgebung bestimmt werden. Aus der Änderung der relativen Lage dieser Merkmale zueinander (Abstand A1, A2 etc.) kann die Änderung der Orientierung des Auges bezüglich der Umgebung bestimmt werden. Gleichermaßen können auch die beide Bilder (beispielsweise durch ein Korrelationsverfahren oder durch Drehung und Verschiebung) zur Deckung gebracht werden. Aus den hierbei auftretenden Werten (beispielsweise Korrelationsfaktoren oder Drehwinkel und Verschiebung) läßt sich wiederum die Orientierungsänderung des Auges gegenüber der Umgebung bestimmen.

Kamerabild/Umgebungsreflexbildstruktur

Aus der Änderung des Bildes der Umgebung, das von einer fest mit einer Vorrichtung verbundenen Kamera aufgenommen oder das von der Netzhaut reflektiert und von einer erfindungsgemäßen Vorrichtung erfaßt wird, läßt sich die Änderung der Orientierung der Vorrichtung gegenüber der Umgebung bestimmten. Hierzu werden beispielsweise zwei Umgebungsreflexbilder (Fig. 16C, 16E) miteinander verglichen, die in einem zeitlichen Abstand voneinander aufgenommen wurden. Hat sich die Orientierung der Vorrichtung gegenüber der Umgebung in der Zwischenzeit um den Winkel $\alpha$ verändert, so weisen signifikante Muster (beispielsweise der durchgehende vertikale Balken) einen Abstand A3 voneinander auf, aus dem sich die Orientierungsänderung $\alpha$ ergibt. Gleichermaßen können auch die beide Bilder (beispielsweise durch ein Korrelationsverfahren oder durch Drehung und Verschiebung) zur Deckung gebracht werden. Aus den hierbei auftretenden Werten (beispielsweise Korrelationsfaktoren oder Drehwinkel und Verschiebung) läßt sich wiederum die Orientierungsänderung des Auges gegenüber der Umgebung bestimmen.

Orientierung Auge - Vorrichtung

Erfindungsgemäß kann eine Markierung an einem Brillenglas vor dem Auge vorhanden sein oder in anderer geeigneter Form in einer festen Position relativ zur Vorrichtung vor dem Auge angeordnet sein.

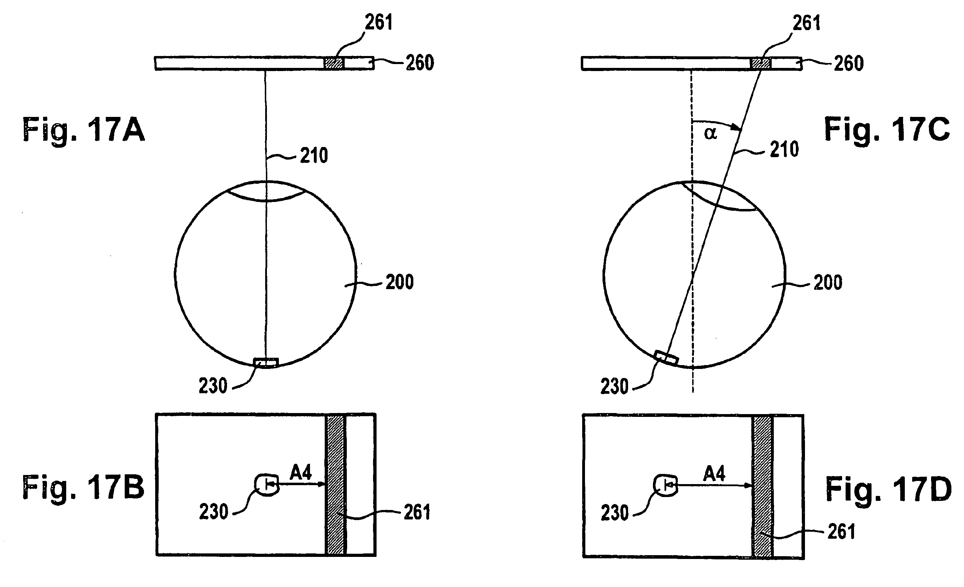

Fig. 17A zeigt hierzu exemplarisch in einer vereinfachten ebenen Darstellung schematisch ein Auge 200 mit der Sehachse 210 und der Makula 230. Vor dem Auge ist ein Brillenglas 260 angeordnet, das im Sichtfeld des Auges eine Markierung 261 aufweist. Fig. 17B zeigt ein Bild der Umgebung, das von der Netzhaut reflektiert wird und in dem die Markierung 261 erkennbar ist (Umgebungsreflexbild), und überlagert ein Bild der Netzhautreflexbild mit der Makula 230.

Anhand der relativen Lage des Netzhautreflexbilds (Augenbild) zur Markierung im Umgebungsreflexbild (Vorrichtungsbild), angedeutet durch den Abstand A4 zwischen Makulamittelpunkt und einer signifikanten Kante der Markierung, kann eine Orientierung des Auges relativ zur Vorrichtung bestimmt sein. Als Vorrichtungsbild wird ein bezüglich der Vorrichtung festes Bild, beispielsweise das Bild der Markierung 261, verwendet.

Gleichermaßen kann auch bereits durch die relative Lage des Augenbildes, angedeutet beispielsweise durch die Makulamitte innerhalb des Bildes Fig. 17B, eine Orientierung des Auges bezüglich eines vorrichtungsfesten Koordinatensystems, gegeben beispielsweise durch die linke bzw. untere Bildkante in Fig. 17B, bestimmt sein, ohne daß eine Markierung notwendig ist.

Fig. 17 C zeigt ebenfalls das Auge 200, dessen Orientierung, gekennzeichnet durch die Sehachse 210, sich gegenüber dem Brillenglas 260 um den Winkel α verändert hat. Fig. 17 D zeigt wiederum überlagert ein von der Netzhaut reflektiertes Bild der Umgebung mit der Markierung 261 und ein Bild der Netzhaut mit der Makula 230. Das Bild der Netzhaut kann dabei beispielsweise mittels aktiver Abtastung erfaßt sein.

Aus dem Vergleich der beiden Bilder 17B und 17C zeigt sich, daß sich der Abstand A4 der Bilder der Markierung 261 und der Makula 230 entsprechend der Orientierungsänderung α des Auges relativ zur Vorrichtung ändert. Aus der Änderung dieses Abstandes kann also die Orientierungsänderung bestimmt werden. Wie oben erwähnt kann statt der Markierung 261 beispielsweise auch die geometrische Form des Brillenrandes selber als Referenz gewählt sein.

Gleichermaßen kann auch ohne Markierung bereits aus der Änderung der relativen Lage der Makula (Augenbild) innerhalb des vorrichtungsfesten Koordinatensystems (wiederum gegeben durch linke bzw. untere Kante in Fig. 17D), die Orientierungsänderung des Auges relativ zur Vorrichtung bestimmt werden.

Obenstehend wurden verschiedene erfindungsgemäße Möglichkeiten erläutert, wie anhand von Lichtstrahlen, die von einem Teil eines Auges abgestrahlt werden, charakteristische Merkmale des Auges bestimmt werden können und wie diese dazu verwendet werden können, die Orientierung des Auges relativ zur Umgebung und/oder relativ zur Vorrichtung zu bestimmen.

Nachdem vorstehend einige Ausführungen der verschiedenen Merkmale einer erfindungsgemäßen Vorrichtung bzw. eines erfindungsgemäßen Verfahrens detailliert beschrieben wurden, die bei einer Ausführung der vorliegenden Erfindung geeignet miteinander kombiniert sein können, werden im folgenden exemplarisch einige Anwendungsbeispiele der vorliegenden Erfindung näher beschrieben.

Wie eingangs dargestellt bietet die Kenntnis der momentanen Orientierung eines Auges eine Vielzahl von Möglichkeiten:

*5.1 Medizin*

In der Medizin kann eine Vorrichtung zur Behandlung des Auges durch eine Vorrichtung, die von einer erfindungsgemäßen Vorrichtung die momentane Orientierung bzw. Orientierungsänderung eines Auges empfängt und diese Signale geeignet weiterverarbeitet (beispielsweise innerhalb einer Positionsregelung, bei der Orientierungsänderungen als Abweichung verwendet sind), willkürliche und vor allem unwillkürliche Augenbewegungen (beispielsweise sog. Sakadenbewegungen bzw. Mikrotremor) kompensieren, so daß beispielsweise ein Laserstrahl zur Behandlung des Auges oder ein Lichtsignal zur Untersuchung des Auges relativ zum Auge ruhend bzw. auf einer relativ zum Auge festgelegten Trajektorie geführt werden.

*5.2 Psychologie, Neurologie*

In der Neurologie oder der Psychologie können bestimmte Augenbewegungen, i.e. Orientierungsänderungen, als Hilfsmittel zur Diagnose verwendet werden, beispielsweise zur Vorhersage eines epileptischen Anfalls, eines Blackouts, zur Diagnose von Schizophrenie oder dergleichen.

Wesentlich weitergehende Anwendungen erschließen sich, falls insbesondere die Blickrichtung des Auges bestimmt wird: in der Psychologie können beispielsweise Erkennungsmuster beim Betrachten bestimmter Bilder erfaßt und analysiert werden.

*5.3 Blickrichtung*

Die Kenntnis der Blickrichtung ermöglicht es, zu bestimmen, wohin der Anwender gerade blickt. Dies kann verwendet werden, um einen vom Anwender fixierten (evtl. nur virtuellen) Gegenstand zu identifizieren. Dazu kann beispielsweise ein Umgebungsreflexbild der Netzhaut abgetastet werden. Wird beispielsweise durch aktive Abtastung die Lage des Pupillen- oder der Makulamittelpunkts innerhalb dieses Umgebunsgreflexbildes bestimmt, so kann dieser Punkt als der Punkt bestimmt werden, auf den der Anwender gerade blickt. Eine Bilderkennung kann daraus beispielsweise den Gegenstand identifiziern, den der Anwender gerade anblickt.

Dabei kann der Anwender beispielsweise durch Lidschlag, Tastendruck oder ähnliches signalisieren, daß er gerade den ausgewählten Gegenstand fixiert.

Gleichermaßen kann auch nach einer gewissen Zeitspanne, in der keine bzw. nur kleine Augenbewegungen auftreten, bestimmt werden, daß der Anwender einen Gegenstand fixiert und dieser Gegenstand aus der ermittelten Blickrichtung bestimmt werden.

Gleichermaßen kann der Anwender auch angewiesen werden, einen bestimmten (unter Umständen auch relativ zum Anwender bewegten) Punkt bzw. Gegenstand zu fixieren. Während der Anwender fixiert, kann gleichzeitig eine Orientierung seines Auges durchgeführt werden. Beispielsweise kann jeweils die Lage des Pupillenmittelpunktes bestimmt werden. Aus der Korrelation des fixierten Gegenstandes und der jeweils ermittelten Orientierung kann dann beispielsweise eine Korrelation zwischen der Orientierung des Auges und einer Sehachse bestimmt werden, die beispielsweise durch die Verbindungsgerade vom fixierten Punkt zum Pupillen- oder Makulamittelpunkt bestimmt sein kann. Anhand dieser Korrelation kann dann aus einer ermittelten Orientierung des Auges (beispielsweise der Lage des Pupillenmittelpunktes) die Orientierung der Sehachse relativ zur Vorrichtung ermittelt werden.

*5.4 Fokus des Betrachters*

Eine geeignet Vorrichtung, die das Signal empfängt, welchen Bildausschnitt der Anwender gerade fixiert und diesen Bildausschnitt gegebenenfalls mittels Bilderkennung identifiziert, kann diese Information weiterverarbeiten und eine gewünschte Aktion auslösen:

ein Schütze kann so ein gewünschtes Ziel auswählen, dessen Koordinaten beispielsweise als Zieldaten einer Raketenwaffe dienen;

ein Anwender kann (beispielsweise im Hausnalt oder in einem Produktionsbetrieb) ein Gerät auswählen (beispielsweise einen Lichtschalter, ein Haushaltsgerät oder eine Werkzeugmaschine), das aktiviert, deaktiviert oder in sonstiger Weise gesteuert werden soll. Eine Steuervorrichtung empfängt das Signal, wohin der Betrachter blickt, identifiziert das Gerät bzw. den Schalter, den er gerade anblickt, und steuert das ausgewählte Gerät entsprechend eines vorbestimmten Steuerprogramms. Beispielsweise blickt der Anwender den Systemschalter des Fernsehers an, blinzelt mehrmals (um unwillkürliche Lidschläge auszuschließen) und die Vorrichtung schaltet den Fernseher ein. Dann blickt der Betrachter auf ein bestimmtes Programmfeld und die Vorrichtung schaltet den Fernseher auf das zugeordnete Programm um, das wiederum durch mehrmaliges Blinzeln signalisiert wurde etc.

Die vom Anwender zu fixierenden Muster brauchen dabei nicht notwendigerweise real existieren, sie können auch rein virtuell vorhanden sein. Beispielsweise zeigt ein Computerbildschirm verschiedene Felder an, die jeweils einem auszuwählenden Menüpunkt entsprechen. Oder solche Felder werden durch die Vorrichtung direkt auf die Netzhaut des Anwenders projiziert.

*5.5 Projektion*

Bei der Projektion von Informationen auf die Netzhaut des Auges, wie sie beispielsweise in den PCT-Arnneldungen WO 0231580, WO 0231577, WO 0231578, WO 0231579 bzw. WO 02097511 des Anmelders offenbart sind, ist es notwending bzw. vorteilhaft, die in das Auge eingespielten (Bild)Informationen mit der Blickrichtung des Auges zu korrelieren:

wird die Information zusätzlich zum Umgebungsbild einprojiziert, das der Anwender beispielsweise mittels einer halbdurchlässig verspiegelten Brille wahrnimmt (s. den Abschnitt "opt. Vorrichtung"), kann es vorteilhaft sein, die Information mit dem von der Netzhaut wahrgenommenen Umgebungsbild so zu korrelieren, daß die eingespielte Information relativ zur Umgebung als ruhend erscheint. Beispielsweise kann hierzu das von der Netzhaut reflektierte Bild der Umgebung im Detektor erfaßt, geeignet aufbereitet und unter Ausnutzug der ermittelten Orientierung des Auges deckungsgleich mit dem tatsächlich wahrgenommenen Umgebungsbild wieder in das Auge zurückprojiziert werden.

Gleichermaßen kann beispielsweise auch zusätzlich von einer Kamera ein Bild der Umgebung aufgenommen, geeignet aufbereitet und unter Ausnutzung der ermottelten Orienteirung des Auges derart in das Auge projiziert werden, daß es deckungsgleich mit dem tatsächlich wahrgenommenen Bild erscheint. Damit ist es möglich, beispielsweise mit einer Kamera ein Infrarotbild der Umgebung aufzunehmen und das daraus erhaltene Umgebungsbild als zusätzliche Information in das Auge einzuspielen, um die Sicht bei Nebel, Nacht oder anderen erschwerten Sichtbedingungen zu verbessern.

Gleichermaßen kann die Kamera eine Femglas-Optik zur Vergrößerung entfernter Bilder aufweisen. Dann kann beispielsweise in der Mitte des vom Auge wahrgenommenen Umgebungsbild ein vergrößerter Bildausschnitt in das Auge hineinprojiziert werden, was gegenüber einem konventionellen Fernglas den Vorteil aufweisen kann, daß Randbereiche des Umgebungsbildes nicht vergrößert werden und damit die Orientierung des Anwenders zur Umgebung nicht bzw. nicht so stark beeinträchtigt wird. Alternativ kann auch ein komplettes vergrößertes Bild der Umgebung in das Auge hineinprojiziert werden, daß das natürlich wahhrgenommene Umgebungsbild überdeckt, wobei kleine Relativbewegungen der Vorrichtung, die beispielsweise aus dem natürlichen Muskelzittem oder den Erschütterungen während einer Autofahrt resultierten, so kompensiert werden, daß das "Fernglas"-Bild dem Anwender als ruhend erscheint. Eine solche Fernglas-Vorrichtung vermeidet die störend wahrgenommenen Mikrobewegungen bei einem konventionellen Fernglas.

Gleichermaßen ist es anhand einer Ermittlung der Orientierung des Auges möglich, eine Information so in das Auge hineinzuprojizieren, daß sie sich relativ zur Umgebung bewegt bzw. relativ zu einem sich bewegenden Objekt als ruhend erscheint: Beispielsweise könnte der Abstand zu einem vorausfahrenden Fahrzeug gemessen und in das Auge des Fahrers so hineinprojiziert werden, daß die Information relativ zu dem vorausfahrenden Fahrzeug fixiert erscheint. Der Fahrer erhält somit notwendige Informationen, ohne den Blick von dem vorausfahrenden Fahrzeug abwenden zu müssen. Umgekehrt kann beispielsweise ein zu lesender Text so in das Auge hineinprojiziert werden, daß er sich scheinbar vor dem Fokus des Betrachters (i.e. der Bereich des wahrgenommenen Bildes, auf den das Interesse gerichtet ist) entsprechend eines vorbestimmten Bewegungsmusters (beispielsweise gleichförmig von rechts nach links, diskret wort- bzw. buchstabengruppenweise etc.) bewegt. Gleichermaßen kann ein zu lesender Text auch so in das Auge hineinprojiziert werden, daß er relativ zur Umgebung als ruhend erscheint, indem beispielsweise die ermittelten Orientierungsänderungen bei der Projektion des Textes berücksichtigt werden.

Wird die Information hingegen in das Auge hineinprojiziert, ohne daß gleichzeitig auf natürlichem Wege die Umgebung wahrgenommen wird, wie dies beispielsweise bei virtual-reality-Brillen der Fall ist, bei denen der Anwender lediglich die in das Auge hineinprojizierten Bilder wahrnimmt, so ermöglicht die Bestimmung der Blickrichtung des Auges es, die hineinprojizierten Informationen, etwa die Sicht auf eine virtuelle Landschaft, der Blickrichtung des Betrachters anzupassen. Blickt der Betrachter also nach links, indem er sein Auge relativ zur Brille verdreht, erkennt eine erfindungsgemäße Vorrichtung diese Orientierungsänderung und verändert entsprechend den scheinbaren Blickwinkel auf die virtuelle Landschaft.

**Patentansprüche**

1. Vorrichtung (620) zur Bestimmung der Orientierung eines Auges (680), mit

   - einem Detektorsystem (651) zum Erfassen eines von einem Teil des Auges ausstrahlenden Lichtstrahls (630) gemäß einer Scanbewegung, bei der ein Ausgangspunkt des erfaßten Lichtstrahls ein Bewegungsmuster (1138) auf einer Struktur (681) des Auges beschreibt, wenn der Strahlengang des Lichtstrahls aufgrund der Scanbewegung zeitlich verändert wird,

   **gekennzeichnet durch**

   - eine Bestimmungseinrichtung, die das Bewegungsmuster zur Bestimmung der **Position der Makulamitte (MM) oder der Fovea (686)** des Auges heranzieht; **und**
   - eine Verlegeeinrichtung, die das Zentrum des Bewegungsmusters In die **Fovea** nachführt.

2. Vorrichtung nach Anspruch 1, bei der keine aktive Beleuchtung des Auges erfolgt, und das Detektorsystem eine pixelweise Abtastung des vom Auge reflektierten Umgebungslichtes und/oder des vom Auge emittierten Lichts durchführt.

3. Vorrichtung nach Anspruch 1, mit einem Projektionssystem (653) zum Projizieren von Licht ins Auge.

4. Vorrichtung nach **Anspruch 3,** wobei das Projektionssystem das Auge flächenhaft mit Infrarotlicht beleuchtet und das Detektorsystem eine pixelweise Abtastung des vom Auge zurückreflektierten Infrarotlichtes durchführt.

5. Vorrichtung nach Anspruch 3, wobei das Projektionssystem das Licht als Lichtstrahl gemäß einer Projektionsbewegung ins Auge projiziert, bei der ein Endpunkt des projizierten Lichtstrahls (630) ein Bewegungsmuster (1138) auf einer Struktur (681) des Auges beschreibt, wenn der Strahlengang des Lichtstrahls aufgrund der Projektionsbewegung zeitlich verändert wird.

6. Vorrichtung nach Anspruch 5, wobei die Bestimmungseinrichtung das Bewegungsmuster der Projektionsbewegung zur Bestimmung der **Position der Makulamitte oder der Fovea des Auges heranzieht.**

7. Vorrichtung nach Anspruch 5 oder 6, wobei die Verlegeeinrichtung das Zentrum des Bewegungsmusters der Projektionsbewegung in die **Makulamitte oder die Fovea** nachführt.

8. Vorrichtung (620) zur Bestimmung der Orientierung eines Auges (680), mit

   - einem Projektionssystem (653) zum Projizieren eines Lichtstrahls (630) gemäß einer Projektionsbewegung ins Auge, bei der ein Endpunkt des projizierten Lichtstrahls ein Bewegungsmuster (1138) auf einer Struktur (681) des Auges beschreibt, wenn der Strahlengang des Lichtstrahls aufgrund der Projektionsbewegung zeitlich verändert wird; und
   - einem Detektorsystem (651) zum Erfassen von Licht, das vom Auge abgestrahlt oder reflektiert worden ist, **gekennzeichnet durch**
   - eine Bestimmungseinrichtung, die das Bewegungsmuster zur Bestimmung der **Position der Makulamitte (MM) oder der Fovea (686)** des Auges heranzieht; und
   - eine Verlegeeinrichtung, die das Zentrum des Bewegungsmusters in die **Makulamitte oder die Fovea** nachführt.

9. Vorrichtung nach einem der Ansprüche 4-8, mit

   - einem Teilerspiegel (656), wobei
   - das Detektorsystem und das Projektionssystem derart konfiguriert sind, daß das Detektorsystem Licht erfaßt, das vom Projektionssystem über den Teilerspiegel über eine gemeinsame Lichtleitanordnung in das Auge projiziert und vom Auge über den Teilerspiegel zurückreflektiert worden ist, und
   - der Teilerspiegel derart konfiguriert ist, daß das Verhältnis der zum Detektor reflektierten Strahlen zu den vom Auge auf den Teilerspiegel fallenden Strahlen 80%-95% beträgt.

10. Vorrichtung nach Anspruch 9, wobei der Teilerspiegel derart konfiguriert ist, daß der Teilerspiegel 95% des einfallenden Lichtes reflektiert und 5% passieren läßt.

11. Vorrichtung nach einem der Ansprüche 5-10, bei der das Projektionssystem ein Infrarotlichtstrahl auf das Auge projiziert wird, dessen Durchmesser unter 100 $\mu$m an dem Luft-Kornea-Übergang ist, und das Detektorsystem einen okularen, Insbesondere einen retinalen, Reflex des Infrarotlichtstrahls erfaßt.

12. Vorrichtung nach einem der Ansprüche 4-11, wobei

   - das Detektorsystem und das Projektionssystem derart konfiguriert sind, daß das Detektorsystem Licht erfaßt, das vom Projektionssystem in das Auge projiziert und vom Auge zurückreflektiert worden ist;
   - die Vorrichtung eine Erhöhung der Lichtstärke des vom Projektionssystem projizierten Lichtes veranlaßt, wenn das Detektorsystem das vom Auge zurückrefiektiertes, projiziertes Licht nicht ausreichend deutlich erfassen kann; und
   - die Vorrichtung eine entsprechende Reduzierung der Lichtstärke des vom Projektionssystem projizierten Lichtes veranlaßt, wenn das Detektorsystem eine unnötig hohe Lichtstärke des vom Auge zurückreflektierten, projizierten Licht registriert.

13. Vorrichtung nach einem der vorhergehenden Ansprüche 5-12, bei der das Projektionssystem Licht pixelweise mit einer vorgegebenen Pixelfrequenz auf das Auge projiziert und das Projektionssystem das projizierte Licht mit einer Frequenz moduliert, die höher als die Pixelfrequenz ist.

14. Vorrichtung nach einem der Ansprüche 1-13, wobei die Vorrichtung zur Erfassung von Referenzbildern, die eine bestimmte Anzahl von Bildpunkten umfassen, sowie zur Erfassung von okularen Bildern, die weniger als die bestimmte Anzahl von Bildpunkten umfassen, konfiguriert ist, wobei die okularen Bilder vom Detektorsystem erfaßt werden.

15. Vorrichtung nach Anspruch 14, wobei die Vorrichtung zur Bestimmung der Orientierung des Auges die Korrelation eines der okularen Bilder zu einem der Referenzbilder ermittelt.

16. Vorrichtung nach Anspruch 14 oder 15, wobei die okularen Bilder und/oder die Referenzbilder Bilder sind, in dem Strukturen (1188, 1189) des Auges erkennbar sind.

17. Vorrichtung nach einem der Ansprüche 14-16, wobei die okularen Bilder und/oder die Referenzbilder Bilder sind, in dem ein okulares Reflexbild der Umgebung erkennbar ist.

18. Vorrichtung nach einem der Ansprüche 14-17, mit

- einer Kamera, mittels der die Referenzbilder erfaßt werden, wobei
- die Referenzbilder Bilder der Umgebung sind, die keine okulare Reflektion erfahren haben.

19. Vorrichtung nach einem der vorhergehenden Ansprüche, wobei die Vorrichtung sowohl die Position der **Makulamitte oder die Fovea** des Auges als auch die Position der Pupillenmitte (PM) des Auges bestimmt.

20. Vorrichtung nach einem der vorhergehenden Ansprüche, wobei die Vorrichtung tragbar, insbesondere in Form einer Brille, ausgebildet ist.

21. Vorrichtung nach einem der vorhergehenden Ansprüche, mit

- einer Lichtleitanordnung (652, 654), mit der der Strahlengang des vom Detektorsystem erfaßten und/oder des vom **Projektionssystem** projizierten Lichtes entsprechend dem Bewegungsmuster des Scan- bzw. Projektionsbewegung steuerbar ist; und
- einer Justiereinrichtung, mit der eine neutrale Mittelstellung der Lichtleitanordnung unter Heranziehung der Veränderung der Koordinaten der **Makulamitte oder der Fovea** nachführbar ist.

22. Vorrichtung nacht einem der vorhergehenden Ansprüche, mit einer eine vorbestimmte geometrische Form aufweisenden, vor dem Auge positionierbaren Fläche (623L, 623R), über die Licht in das Auge projiziert und/oder aus dem Auge erfaßt werden kann, wobei die geometrischen. Form der Fläche dazu herangezogen wird, die Relativlage mindestens eines charakteristischen Bereichs einer Struktur des Auges bezüglich der Vorrichtung zu bestimmen.

**Claims**

1. An apparatus (620) for determining the orientation of an eye (680), comprising:

- a detector system (651) for capturing a light beam (630) emanating from a part of the eye in accordance with a scanning motion in which an origin of the captured light beam describes a pattern of motion (1138) on a structure (681) of the eye when the beam path of the light beam is changed over time due to the scanning motion,

**characterized by**

- a determining apparatus that employs the pattern of motion for determining the position of the fovea (686) or the center of the macula (MM) of the eye; and
- a repositioning apparatus that tracks the center of the pattern of motion to the fovea or the center of the macula.

2. The apparatus of claim 1, in which no active illumination of the eye is carried out and the detector system carries out a pixel-wise scanning of the light reflected from the eye from the environment and/or of the light emitted from the eye.

3. The apparatus of claim 1, comprising a projection system (653) for projecting light into the eye.

4. The apparatus of claim 2, wherein the projection system broadly illuminates the eye with infrared light and the detector system carries out a pixel-wise scanning of the infrared light reflected back from the eye.

5. The apparatus of claim 3, wherein the projection system projects the light as a light beam into the eye in accordance with a projection motion in which an end point of the projected light beam (630) describes a pattern of motion (1138) onto a structure (681) of the eye when the beam path of the light beam is changed over time due to the projection motion.

**6.** The apparatus of claim 5, wherein the determining apparatus employs the pattern of motion of the projection motion for determining the position of the fovea or the center of the macula of the eye.

**7.** The apparatus of claim 5 or 6, wherein the repositioning apparatus tracks the center of the pattern of motion of the projection motion to the fovea or the center of the macula.

**8.** An apparatus (620) for determining the orientation of an eye (680), comprising:

- a projection system (653) for projecting a light beam (630) into the eye in accordance with a projection motion in which an end point of the projected light beam describes a pattern of motion (1138) onto a structure (681) of the eye when the beam path of the light beam is changed over time due to the projection motion; and
- a detector system (651) for capturing light that has been emitted or reflected from the eye,

**characterized by**

- a determining apparatus that employs the pattern of motion for determining the position of the fovea (686) or the center of the macula (MM) of the eye; and
- a repositioning apparatus that tracks the center of the pattern of motion to the fovea or the center of the macula.

**9.** The apparatus of any one of claims 4-8, comprising:

- a beam splitter (656), wherein
- the detector system and the projection system are configured such that the detector system captures light that has been projected by the projection system into the eye via the beam splitter and a common light guidance configuration and has been reflected back from the eye via the beam splitter, and
- the beam splitter is configured such that the ratio of the beams reflected to the detector to the beams falling onto the beam splitter from the eye is 80%-95%.

**10.** The apparatus of claim 9, wherein the beam splitter is configured such that the beam splitter reflects 95% of the incident light and allows 5% to pass.

**11.** The apparatus of any one of claims 5-10, in which the projection system projects an infrared light beam onto the eye, the diameter of which is less than 100 μm at the air-cornea-interface, and the detector system captures an ocular, in particular a retinal, reflex of the infrared light beam.

**12.** The apparatus of any one of claims 4-11, wherein

- the detector system and the projection system are configured such that the detector system captures light that has been projected into the eye by the projection system and has been reflected back by the eye;
- the apparatus effects an increase of the light intensity of the light projected by the projection system when the detector system cannot sufficiently distinctly capture the projected light reflected back from the eye;
- the apparatus effects a corresponding reduction of the light intensity of the light projected by the projection system when the detector system registers an unnecessarily high light intensity of the projected light reflected back from the eye.

**13.** The apparatus of any one of claims 5-12, in which the projection system projects light pixel-wise onto the eye at a predetermined pixel frequency and the projection system modulates the projected light at a frequency that is higher than the pixel frequency.

**14.** The apparatus of any one of claims 1-13, wherein the apparatus is configured for capturing references images that encompass a given number of pixels as well as for capturing ocular images that encompass less than the given number of pixels, wherein the ocular images are captured by the detector system.

**15.** The apparatus of claim 14, wherein the apparatus for determining the orientation of the eye determines the correlation of one of the ocular images to one of the reference images.

**16.** The apparatus of claim 14 or 15, wherein the ocular images and/or the reference images are images in which structures (1188, 1189) of the eye are recognizable.

17. The apparatus of any one of claims 14-16, wherein the ocular images and/or the reference images are images in which an ocular reflex image of the surroundings is recognizable.

18. The apparatus of any one of claims 14-17, comprising:

   - a camera via which the reference images are captured, wherein
   - the reference images are images of the surroundings that have not undergone ocular reflection.

19. The apparatus of any one of the preceding claims, wherein the apparatus determines both the position of the fovea or the center of the macula as well as the position of center of the pupil (PM) of the eye.

20. The apparatus of any one of the preceding claims, wherein the apparatus is configured to be portable, in particular in the shape of spectacles.

21. The apparatus of any one of the preceding claims, comprising:

   - a light guiding apparatus (652, 654) via which the beam path of the light projected by the detector system and/or projected by the projection system is steerable in accordance with the pattern of motion of the scanning/ projection motion; and
   - an adjusting apparatus via which a neutral, central position of the light guiding apparatus can be repositioned on the basis of the change in coordinates of the fovea or the center of the macula.

22. The apparatus of any one of the preceding claims, comprising a surface (623L, 623R), positionable in front of the eye, having a predetermined geometric shape, via which surface light can be projected into the eye and/or captured from the eye, wherein the geometric shape of the surface can be employed in determining the relative position of a characteristic region of a structure of the eye relative to the apparatus.

**Revendications**

1. Dispositif (620) destiné à déterminer l'orientation d'un oeil (680), avec

   - un système de détection (651) prévu pour capter une partie du faisceau lumineux émis par l'oeil (630) selon un mouvement de balayage lors duquel le point de départ du faisceau lumineux intercepté décrit une trajectoire type (1138) sur une structure (681) de l'oeil, lorsque le trajet du faisceau lumineux est modifié dans le temps du fait du mouvement de balayage,

   **caractérisé par**

   - un moyen de détermination qui se sert de la trajectoire-type pour localiser la position de la fovéa (686) ou du centre de la macula (MM) de l'oeil ; et
   - un moyen de transfert qui induit le déplacement du centre de la trajectoire type sur la fovéa ou le centre de la macula.

2. Dispositif selon la revendication 1, sur lequel aucun éclairage actif de l'oeil n'a lieu et le système de détection effectue le balayage pixel par pixel de la lumière ambiante réfléchie par l'oeil et / ou de la lumière émise par l'oeil.

3. Dispositif selon la revendication 1, muni d'un système de projection (653) pour diffuser de la lumière dans l'oeil.

4. Dispositif selon la revendication 3, dans lequel le système de projection éclaire l'oeil en surface à l'aide d'une lumière infrarouge et le système de détection exécute le balayage de la lumière renvoyée par l'oeil pixel par pixel.

5. Dispositif selon la revendication 3, dans lequel le système de projection diffuse la lumière dans l'oeil sous la forme d'un faisceau lumineux selon un mouvement de projection où le point d'arrivée du faisceau lumineux diffusé (630) décrit une trajectoire type (1138) sur une structure (681) de l'oeil, lorsque le trajet du faisceau lumineux est modifié dans le temps du fait du mouvement de projection.

6. Dispositif selon la revendication 5, dans lequel le moyen de détermination exploite la trajectoire type du mouvement de projection pour localiser la position de la fovéa ou du centre de la macula de l'oeil.

7. Dispositif selon les revendications 5 ou 6, dans lequel le moyen de transfert induit le déplacement du centre de la trajectoire type du mouvement de projection sur la fovéa ou le centre de la macula.

8. Dispositif (620) destiné à déterminer l'orientation d'un oeil (680), avec

   - un système de projection (653) pour diffuser un faisceau lumineux (630) dans l'oeil selon un mouvement de projection où le point d'arrivée du faisceau lumineux diffusé (630) décrit une trajectoire type (1138) sur une structure (681) de l'oeil, lorsque le trajet du faisceau lumineux est modifié dans le temps du fait du mouvement de projection ; et
   - un système de détection (651) prévu pour capter la lumière rayonnée ou réfléchie par l'oeil,

   **caractérisé par**

   - un moyen de détermination qui se sert de la trajectoire-type pour localiser la position de la fovéa (686) ou du centre de la macula (MM) de l'oeil; et
   - un moyen de transfert qui induit le déplacement du centre de la trajectoire type sur la fovéa ou le centre de la macula.

9. Dispositif selon l'une des revendications de 4 à 6, avec

   - un miroir séparateur (656), dans lequel
   - le système de détection et le système de projection sont configurés de manière à ce que le système de détection capte la lumière qui a été diffusée dans l'oeil par le système de projection au moyen du miroir séparateur en profitant d'un montage commun de conduction de lumière et qui a été ensuite réfléchie par l'oeil à l'aide du miroir séparateur, et
   - le miroir séparateur est configuré de manière à ce que le rapport des rayons réfléchis en direction du détecteur avec ceux émanant de l'oeil et incidents à la surface du miroir séparateur soit compris entre 80% et 95%.

10. Dispositif selon la revendication 9, dans lequel le miroir séparateur est configuré de manière à réfléchir 95% de la lumière incidente et en laisse passer 5%.

11. Dispositif selon l'une des revendications de 5 à 10, dans lequel le système de projection diffuse un faisceau de lumière infrarouge dont le diamètre est inférieur à 100 $\mu$m au niveau de l'interface air-cornée et le système de détection décèle le reflet oculaire, notamment rétinien du faisceau de lumière infrarouge.

12. Dispositif selon l'une des revendications de 4 à 11, dans lequel

   - le système de détection et le système de projection sont configurés de manière à ce que le système de détection capte la lumière qui a été diffusée dans l'oeil par le système de projection et réfléchie par l'oeil ;
   - le dispositif induit l'élévation de la luminosité de la lumière diffusée par le système de projection si le système de détection ne peut pas déceler la lumière projetée et réfléchie par l'oeil avec suffisamment de netteté ; et
   - le dispositif induit la réduction appropriée de la luminosité de la lumière diffusée par le système de projection, si le système de détection enregistre la luminosité plus élevée que nécessaire de la lumière projetée et réfléchie par l'oeil.

13. Dispositif selon l'une des revendications précédentes de 5 à 12, dans lequel le système de projection diffuse de la lumière sur l'oeil pixel par pixel à une fréquence prédéterminée et le système de projection module la lumière projetée à une fréquence qui est supérieure à celle des pixels.

14. Dispositif selon l'une des revendications de 1 à 13, dans lequel ledit dispositif est configuré pour saisir des images de référence qui englobent un certain nombre de points d'image ainsi que pour capturer des images oculaires qui couvrent moins de points d'image que le nombre défini **caractérisé par** ailleurs, dans lequel les images oculaires sont enregistrées par le système de détection.

15. Dispositif selon la revendication 14, dans lequel le dispositif destiné à déterminer l'orientation de l'oeil évalue la corrélation de l'une des images oculaires avec l'une des images de référence.

**16.** Dispositif conformément aux revendications 14 ou 15, dans lequel les images oculaires et / ou les images de référence constituent des images sur lesquelles des structures (1188, 1189) de l'oeil sont reconnaissables.

**17.** Dispositif conformément aux revendications 14 ou 16, dans lequel les images oculaires et / ou les images de référence constituent des images sur lesquelles l'image du reflet oculaire de l'environnement est perceptible.

**18.** Dispositif selon l'une des revendications de 14 à 17, avec

  - un appareil photographique au moyen duquel les images de référence sont réalisées, où
  - les images de référence constituent des images de l'environnement qui n'ont fait l'objet d'aucune réflexion oculaire.

**19.** Dispositif selon l'une des revendications précédentes, dans lequel ledit dispositif détermine tant la position de la fovéa ou du centre de la macula que la position du centre de la pupille (PM) de l'oeil.

**20.** Dispositif selon l'une des revendications précédentes, dans lequel ledit dispositif est façonné sous une forme portable, notamment de lunettes.

**21.** Dispositif selon l'une des revendications précédentes, avec

  - un montage de conduction de lumière (652, 654) qui permet de diriger le trajet de la lumière captée par le système de détection et / ou diffusée par le système de projection selon la trajectoire type du mouvement de balayage et / ou de projection ; et
  - un asservisseur qui sert à restituer la position centrale neutre du montage de conduction de lumière en prenant en compte la modification des coordonnées de la fovéa ou du centre de la macula.

**22.** Dispositif selon l'une des revendications précédentes, avec une surface présentant une forme géométrique prédéfinie, positionnable devant l'oeil (623L, 623R), par l'intermédiaire de laquelle de la lumière peut être projetée dans l'oeil et / ou captée en provenance de l'oeil, dans lequel la forme géométrique de la surface peut être exploitée pour déterminer la position relative d'au moins une zone caractéristique d'une structure de l'oeil par rapport au dispositif.

# Fig. 1

## Fig. 2

290

20
27a
26
285
284

291

292

283
22
280
27c
27b

21

281

286

282
288

287

24

25

23

28

## Fig. 3a

$I_e$

100 %

0 %

T1   T2   T1

t

## Fig. 3b

$I_e$

100 %

0 %

$\lambda 1$   $\lambda 2$   $\lambda 1$

T1   T2   T1

t

**Fig. 4**

401

405a

402

403a    403b    406

α | α

404a

β

β    404c

405b

404b

404

**Fig. 5**

501

506

505

504    503    507

502

# Fig. 6

Fig. 7a

Fig. 7b

Fig. 8

EP 1 405 122 B1

Fig. 9

Fig. 10

# Fig. 11

**Fig. 12**

Fig. 13

Fig. 14

Fig. 15

**Fig. 16**

250

210'  210

α

220'  220

200

240  240'  230  230'

**Fig. 16A**

**Fig. 16C**

A1

240A  230A

A2

240A  230A

**Fig. 16B**

230A

**Fig. 16D**

A3

**Fig. 16E**

Fig. 17A

Fig. 17B

Fig. 17C

Fig. 17D

**Fig. 18**

**EP 1 405 122 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19631414 A1
- WO 02097511 A
- WO 9942315 A
- DE 19728890 A1
- WO 0231580 A
- WO 0231577 A
- WO 0231578 A
- WO 0231579 A
- DE 10127826